(19) Europäisches Patentamt
European Patent Office
Office européen des brevets

(11) EP 0 766 689 B1

(12) FASCICULE DE BREVET EUROPEEN

(45) Date de publication et mention de la délivrance du brevet:
**20.10.1999 Bulletin 1999/42**

(21) Numéro de dépôt: **95923392.5**

(22) Date de dépôt: **15.06.1995**

(51) Int Cl.$^{6}$: **C07H 15/12**, C07H 19/044, C07H 19/048, A61K 31/70, C07K 17/10

(86) Numéro de dépôt international:
**PCT/FR95/00790**

(87) Numéro de publication internationale:
**WO 96/00229 (04.01.1996 Gazette 1996/02)**

# (54) NOUVEAUX DERIVES D'OLIGOSIDES, LEUR PROCEDE DE PREPARATION ET LEURS APPLICATIONS

OLIGOSID-DERIVATE, VERFAHREN ZU IHRER HERSTELLUNG UND IHRE VERWENDUNG

NOVEL OLIGOSACCHARIDE DERIVATIVES, PREPARATION METHOD THEREFOR AND USES THEREOF

(84) Etats contractants désignés:
**AT BE CH DE DK ES FR GB GR IE IT LI LU MC NL PT SE**

(30) Priorité: **23.06.1994 FR 9407738**

(43) Date de publication de la demande:
**09.04.1997 Bulletin 1997/15**

(73) Titulaire: **I.D.M. IMMUNO-DESIGNED MOLECULES**
**75011 Paris (FR)**

(72) Inventeurs:
- **MONSIGNY, Michel**
  **F-45590 Saint-Cyr-en-Val (FR)**
- **ROCHE, Annie-Claude**
  **F-45640 Sandillon (FR)**
- **SDIQUI, Nadia**
  **F-45100 Orléans (FR)**
- **MAYER, Roger**
  **F-45100 Orléans (FR)**

(74) Mandataire:
**Grosset-Fournier, Chantal Catherine**
**Grosset-Fournier & Demachy s.a.r.l.,**
**20, rue de Maubeuge**
**75009 Paris (FR)**

(56) Documents cités:

| | |
|---|---|
| **WO-A-88/00592** | **WO-A-93/04701** |
| **WO-A-94/03184** | **FR-A- 2 277 823** |
| **US-A- 4 478 744** | **US-A- 5 028 594** |

- **NUCLEIC ACIDS RESEARCH, vol. 20, no. 17, 11 Septembre 1992 ARLINGTON, VIRGINIA US, pages 4621-4629, BONFILS E. ET AL 'Drug targeting: synthesis and endocytosis of oligonucleotide-neoglycoprotein conjugates'**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).



Printed by Jouve, 75001 PARIS (FR)

## Description

L'invention a pour objet de nouveaux dérivés d'oligosides, leur procédé de préparation et leurs applications.
Les oligosides naturels peuvent être préparés à l'état libre à partir de divers liquides physiologiques tels que le lait, ou extraits à partir des produits naturels ou transformés (miel, bière, etc). Les oligosides naturels peuvent également être obtenus par coupure d'une liaison glycosidique d'une des parties glucidiques de glycoconjugués (glycolipides, glycoprotéines, polyosides, protéoglycannes, etc), par hydrolyse à l'aide d'enzymes ou par catalyse chimique à partir desdits glycoconjugués.
Les oligosides naturels peuvent être utilisés comme substrats, comme inhibiteurs, comme signal de reconnaissance, etc. Dans la plupart des cas, il convient de fixer l'oligoside sur une molécule, une matrice ou une particule, qui peuvent être choisies parmi:

- une matrice comme support pour la chromatographie d'affinité ;
- une bille d'or, de latex, pour l'histologie et la cytologie ;
- une protéine pour la visualisation, la purification, etc, notamment 1) des récepteurs spécifiques des osides, récepteurs que l'on appelle lectines, adhésines, agglutinines etc, ou encore 2) des protéines à activité enzymatique ou non, qui ont une affinité pour les osides, notamment des glycosyltransférases, telles que la sialyltransférase, des sulfotransférases, des phosphotransférases, des exoglycosidases ou des endoglycosidases;
- un lipide pour la caractérisation des récepteurs précédents;
- des oligonucléotides pour augmenter sélectivement leur capture par des cellules cibles;
- une protéine ou des polymères pour le ciblage de médicaments, d'oligonucléotides ou de gènes, ou pour réaliser des inhibiteurs intramoléculaires.

La synthèse de dérivés d'oligosides susceptibles d'être liés de façon covalente à une protéine, une matrice, un oligonucléotide ou de façon générale à une molécule organique ou une particule, tout en préservant l'intégrité de la structure et des fonctions de chacun de ses oses constitutifs - ce qui est nécessaire pour préserver la capacité fonctionnelle de l'oligoside - peut être obtenue essentiellement par deux voies: la synthèse totale *de novo* et la transformation intermédiaire en glycosylamine. Une troisième voie conduit à un dérivé également utile mais en détruisant l'ose terminal réducteur, c'est la voie d'amination en milieu réducteur.
S'agissant de la synthèse totale, cette voie exige une protection sélective des hydroxyles non engagés dans une liaison glycosidique, des étapes de condensation, des étapes de déprotection sélective, et une étape de déprotection finale. Même si au cours des dernières décennies les rendements de certaines de ces étapes ont pu être améliorés, cette voie est une voie longue et le rendement global reste modeste, et ce d'autant plus que l'oligoside à synthétiser est plus complexe.
Les rendements sont compris pour chaque étape entre 20 et 95% selon les étapes considérées.
Par exemple la synthèse d'un dérivé *para*-nitrophénylé d'un pentasaccharide tel que le déterminant Lewis x:

$$Gal\beta3(Fuc\alpha4)GlcNAc\beta3Gal\beta4Glc\beta\text{-}O\text{-}p\text{-}C_6H_4\text{-}NO_2$$

exige au total plusieurs dizaines d'étapes. Chaque étape a un rendement compris entre 50 et 95%, plus généralement 80%. Au total le rendement du produit attendu est de quelques %.
Le nombre élevé d'étapes vient de ce que les fonctions alcool de chaque ose doivent être protégées de façon différente selon que l'hydroxyle considéré sera ou non impliqué dans une réaction de condensation.
Un ose tel que GlcNAc qui sera substitué 2 fois recevra momentanément 3 substituants différents pour permettre une substitution sélective sur l'hydroxyle 3 par le galactose, sur l'hydroxyle 4 par le fucose, l'hydroxyle 6 restant protégé jusqu'à déprotection finale.
Pour chaque étape de condensation, le rendement est affecté par le fait que le produit formé est en général un mélange de deux formes anomériques $\alpha$ ou $\beta$.
En outre, il faut signaler que les produits intermédiaires doivent être purifiés, soit par cristallisation, soit par chromatographie, ce qui est une cause importante de la durée de la synthèse totale. Enfin, il faut signaler que les rendements peuvent être très faibles pour certaines étapes par cause d'encombrement stérique, ce qui est particulièrement le cas au niveau des branchements: 2 oses substituant un même ose.
En définitive, cette synthèse globale est très coûteuse et très longue.
Quant à la formation d'une glycosylamine suivie d'acylation, cette voie repose sur une réaction décrite à la fin du siècle dernier: un oside possédant un ose réducteur, incubé en présence d'une concentration élevée d'ammoniac, d'un sel d'ammonium ou d'une amine aromatique, se transforme de façon réversible en glycosylamine.
Par exemple, le lactose donne une lactosylamine, avec l'ammoniac:

$$Gal\beta 4Glc + NH_3 \Rightarrow Gal\beta 4Glc\beta\text{-}NH_2 + H_2O$$

ou avec l'aniline:

$$Gal\beta 4Glc + NH_2\text{-}C_6H_5 \Rightarrow Gal\beta 4Glc\beta\text{-}NH\text{-}C_6H_5 + H_2O$$

Cette réaction est cependant réversible, c'est-à-dire que le produit isolé, dissous dans un tampon, s'hydrolyse pour redonner les produits de départ.

La glycosylamine peut cependant être stabilisée par acylation, par exemple par N-actétylation sélective:

$$Gal\beta 4Glc\beta\text{-}NH_2 + (CH_3\text{-}CO)_2\text{-}O \Rightarrow Gal\beta 4Glc\beta\text{-}NH\text{-}CO\text{-}CH_3 + CH_3\text{-}CO_2H$$

Sur ces bases, il a été récemment proposé de substituer l'amine des oligosylamines par un composé organique possédant un groupement fonctionnel réactif. Manger et al., 1992, Biochemistry 31, 10724 et 31, 10733.

Cette voie comprend la suite des étapes suivantes:

**1**) incubation de l'oside possédant un ose terminal réducteur en présence d'un sel d'ammonium très concentré.

Par exemple,

$$Gal\beta 4Glc + NH_4^+ \Leftrightarrow Gal\beta 4Glc\beta\text{-}NH_3^+ + H_2O$$

$$C_{12}H_{22}O_{11} + NH_4+ \Leftrightarrow C_{12}H_{24}O_{10}N + H_2O$$

**2**) Purification de la glycosylamine par chromatographie sur colonne pour éliminer le sel d'ammonium en excès.

**3**) Substitution de l'amine de la glycosylamine par réaction avec un dérivé activé de l'acide monochloroacétique, en milieu alcalin.

Par exemple,

$$Gal\beta 4Glc\beta NH_2 + (Cl\text{-}CH_2\text{-}CO)_2\text{-}O \Rightarrow Gal\beta 4Glc\text{-}NH\text{-}CO\text{-}CH_2\text{-}Cl$$

**4**) Transformation des résidus chloroacétyle en résidu glycyle.

Le chloroacétylglycosylamide est incubé en présence d'un sel d'ammonium très concentré, ce qui permet d'introduire une fonction amine. Par exemple:

$$Gal\beta 4Glc\beta\text{-}NH\text{-}CO\text{-}CH_2\text{-}Cl + NH_4^+ \Rightarrow Gal\beta 4Glc\beta\text{-}NH\text{-}CO\text{-}CH_2\text{-}NH_3^+,\ Cl^-H^+$$

**5**) Purification du glycyl-glycosylamide par chromatographie sur colonne pour éliminer les sels d'ammonium.

**6**) Condensation du glycyl-glycosylamide et d'un composé susceptible de réagir sélectivement sur un groupement aminé du résidu glycyl.

Par exemple:

$$Gal\beta 4Glc\beta\text{-}NH\text{-}CO\text{-}CH_2\text{-}NH_2 + R\text{-}CO\text{-}G \Rightarrow Gal\beta 4Glc\beta\text{-}NH\text{-}CO\text{-}CH_2\text{-}NH\text{-}CO\text{-}R + HG$$

dans lequel G est un activateur de la fonction carboxylique.

Cette voie en 6 étapes implique deux étapes de purification intermédiaire et l'utilisation d'un produit toxique: le dérivé activé de l'acide chloroacétique.

Le rendement de chaque étape est variable et varie entre 50 et 95%; le rendement global est inférieur à

environ 60%.

Une autre voie a également été suggérée, mais elle entraîne la transformation de l'ose réducteur en polyol; cette voie a été proposée en 1974 par Gray (Arch. Biochem. Biophys., 163, 426-428).

L'oligoside est condensé avec un composé comportant une fonction amine (ou plusieurs) en présence de cyanoborohydrure de sodium en milieu alcalin; l'imine formée entre l'ose réducteur et l'amine est réduite par le cyanoborohydrure de sodium en une amine secondaire.

Par exemple:

$$Gal\beta 4Glc + NH_2\text{-}R \Rightarrow$$

$$Gal\beta\text{-}O\text{-}CH(CHOH\text{-}CH_2OH)\text{-}CHOH\text{-}CHOH\text{-}CH = N\text{-}R$$

$$+ NaBH_3CN \Rightarrow Gal\beta 4\text{-}O\text{-}CH(CHOH\text{-}CH_2OH)\text{-}CHOH\text{-}CHOH\text{-}CH_2\text{-}NH\text{-}R$$

Le rendement de cette réaction varie selon la taille des partenaires et est habituellement compris entre 5 et 70%.

Il y a destruction de l'ose réducteur, ce qui n'est pas souhaitable.

Le brevet français n° 2 277 823 concerne des N-osides de l'acide L-pyrrolidone-2-carboxylique-5, des dérivés de ceux-ci et leur procédé de préparation, consistant à condenser l'acide L-pyrrolidone-2carboxylique-5 et/ou l'acide L-glutamique et/ou un sel de ces acides avec un ose ou un oside, à propriétés réductrices ou non.

D'après les exemples, les sucres réducteurs sont tous des monosaccharides (cétoses ou aldoses), tandis que les sucres non réducteurs sont par exemple le saccharose; compte tenu des conditions réactionnelles, le saccharose s'hydrolyse en glucose et en fructose.

Il faut également noter que dans le procédé du susdit brevet français n° 2 277 823, la condensation se fait de préférence en milieu aqueux, et à une température de 50°C à 150°C, de préférence aux environs de 105°C.

Ces conditions opératoires nécessitent de travailler avec des solutions très concentrées d'ose et d'acide qui sont inapplicables à la préparation d'oligoside; la solubilité des oligosides décroît rapidement lorsque le nombre d'oses croît.

Par ailleurs, la réaction de condensation entre l'ose et l'acide étant une déshydratation thermique, elle est inapplicable à la préparation d'oligosides en raison de la fragilité des liaisons osidiques, qui sont facilement hydrolysées à chaud (voir par exemple, le cas du saccharose).

La condensation thermique présente en outre l'inconvénient de conduire à l'obtention de produits colorés, témoins de réactions de dégradation et nécessitant une étape ultérieure de purification.

Ce procédé ne permet donc d'obtenir que des dérivés de monosaccharides et n'est pas adapté à la production de dérivés d'oligosides.

Le PCT WO93/04701 décrit des complexes moléculaires pour cibler des oligonucléotides. Un oligonucléotide simple brin ou pluri brins est complexé à un conjugué entre un agent de liaison spécifique d'une cellule tel qu'une asialoglycoprotéine et un agent susceptible de se lier à un poly ou oligonucléotide, l'agent de liaison étant par exemple un polycation tel que la polylysine. Le procédé décrit ne permet pas de préparer un dérivé covalent entre un oligonucléotide et un oligoside. En effet, il est proposé d'utiliser des fragments d'arabinogalactane ou du lactose pour former des conjugés avec un transporteur (protéine ou polylysine) par lactosamination réductive ; dans ce cas, l'ose réducteur est réduit en un polyol-aminé, c'est-à-dire qu'il est ouvert et cesse d'être un ose.

Le brevet US 5028 594 décrit des agents cytotoxiques comprenant un ligand spécifique d'une cellule, un groupe de liaison et un composé toxique, dans lesquels le ligand peut être une molécule de saccharide ou de polysaccharide, par exemple le mannose et le groupe de liaison peut être un acide aminé ou un oligopeptide. Le procédé de préparation est tel que lorsque le saccharide ou polysaccharide est attaché de façon covalente au groupe de liaison, les molécules de saccharide sont liés de façon covalente au groupe ε-amino du groupe de liaison. Ce procédé ne permet pas de transformer un oligoside naturel en un dérivé fonctionnalisé en conservant l'intégralité dudit oligoside. Il n'y a aucune indication d'un nouveau procédé de condensation entre une partie glucidique et une partie peptidique ou protéique.

L'un des aspects de l'invention est de proposer de nouveaux dérivés d'oligosides dans lesquels les oligosides sont fixés sur au moins une molécule, une matrice ou une particule.

Un autre aspect de l'invention est de proposer des dérivés de glycosylamine, stables en milieu aqueux, susceptibles d'être fixés sur au moins une molécule, une matrice ou une particule.

L'un des buts de la présente invention est de fournir un procédé de préparation d'oligosides fixés sur au moins une molécule, une matrice ou une particule, simple à mettre en oeuvre, présentant un nombre réduit d'étapes et permettant d'obtenir un rendement pouvant atteindre pratiquement 100%.

Un autre aspect de l'invention est de pouvoir fixer des oligosides de façon covalente sur au moins une molécule, une matrice ou une particule, tout en préservant la capacité fonctionnelle des oligosides.

L'invention a pour objet des nouveaux composés comprenant un ou plusieurs oligosides, chacun desdits oligosides étant fixé de façon covalente sur une ou plusieurs molécules, matrices ou particules, en particulier une, deux ou trois, grâce à une molécule intermédiaire possédant un atome d'azote porté par un carbone en $\alpha$ d'un groupe C = O et un ou plusieurs groupes fonctionnels, en particulier un, deux ou trois, la liaison covalente entre ladite molécule intermédiaire et l'oligoside ayant lieu par l'intermédiaire du susdit atome d'azote, et la liaison covalente entre ladite molécule intermédiaire et la susdite molécule, la susdite matrice, la susdite particule, ou les susdites molécules, les susdites matrices, les susdites particules ayant lieu par l'intermédiaire du ou des susdits groupes fonctionnels de ladite molécule intermédiaire et des groupes fonctionnels appropriés sur la (les) molécule(s), la (les) matrice(s) ou la (les) particule(s).

Le terme oligosides correspond à la présence d'au moins deux, et avantageusement à la présence de plus de deux oses, et avantageusement d'au moins 4.

Les oligosides entrant dans la constitution des composés de l'invention sont tels qu'avant la liaison avec la molécule intermédiaire, ils présentent un ose terminal réducteur. De ce fait, la liaison covalente entre la susdite molécule intermédiaire et l'oligoside a lieu par l'intermédiaire de l'atome d'azote de la molécule intermédiaire et du carbone anomérique de l'ose terminal réducteur de l'oligoside, c'est-à-dire du carbone en position 1 de l'ose terminal réducteur de la série des aldoses, ou du carbone en position 2 de l'ose terminal réducteur de la série des cétoses.

L'invention a pour objet des composés de formule générale (I)

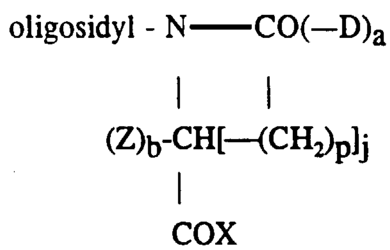

(I)

dans laquelle:

- * a = 0 ou 1,
- * j = 0 ou 1,
- * b = 0 ou 1,
- * p = 2 à 4, notamment 2,
- * sous réserve que
  - ** a = b = 0, lorsque j = 1, ce qui entraîne la présence d'une molécule cyclique,
  - ** ou a = b = 1, lorsque j = 0 ce qui implique l'absence de groupe $(CH_2)_p$,
- * D représente un résidu d'un acide organique de formule $DCO_2H$, notamment H ou une chaîne alkyle de 1 à 10 atomes de carbone, en particulier $CH_3$,
- * Z représente
  - ** B, B étant H, un alkyle de 1 à 10 atomes de carbone, ou une chaîne latérale d'un acide $\alpha$ aminé, naturel ou synthétique tel que $CH(CH_3)_2$, $CH_2OH$, $CH_3$, et de préférence H, ou
  - ** B'-P', B' étant une chaîne alkylidène de 1 à 10 atomes de carbone, ou une chaîne latérale d'un acide $\alpha$ aminé, naturel ou synthétique, lesdites chaînes contenant un groupe dérivé d'un groupement fonctionnel susceptible d'être activé, tel que carboxylique, SH, OH ou amine, libre ou protégé, de préférence protégé, P' ayant les significations indiquées ci-après,
- * X représente:
  - . le groupe

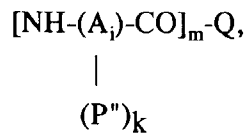

. ou le groupe

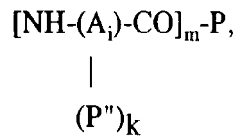

. ou le groupe

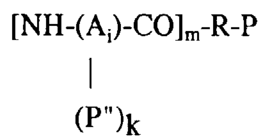

* m étant un nombre entier de 0 à 10, de préférence de 0 à 5 et avantageusement 1 ou 2, k = 0 ou 1
* Q représente OH, $OCH_3$, $OCH_2$-$C_6H_5$, O-$C_6H_5$, O-$C_6F_5$, O-p-$C_6H_4$-$NO_2$,

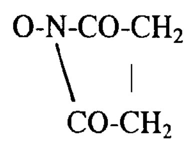

* R représentant un groupe possédant une fonction alcool, phénol, thiol ou amine,
* P étant tel que défini ci-après,
* $A_i$ représente un radical organique tel qu'une chaîne alkylidène de 1 à 10 atomes de carbone, notamment $(CH_2)_n$-W-$(CH_2)_{n'}$, n + n' représentant un nombre entier de 0 à 10, W représentant CHY, Y étant H, un alkyle de 1 à 6 atomes de carbone linéaire ou ramifié, un résidu d'amino $\alpha$ acide, naturel ou synthétique, ou W représentant un composé aromatique, notamment phényle,
* P, P' et P" sont identiques ou différents et représentent :

  - une matrice comme support pour la chromatographie d'affinité;
  - une bille d'or, de latex, pour l'histologie et la cytologie;
  - une protéine pour la visualisation, la purification, etc, notamment 1) des récepteurs spécifiques des osides, récepteurs que l'on appelle lectines, adhésines, agglutinines etc, ou encore 2) des protéines à activité enzymatique ou non, qui ont une affinité pour les osides, notamment des glycosyltransférases, telles que la sialyltransférase, des sulfotransférases, des phosphotransférases, des exoglycosidases ou des endoglycosidases;
  - un lipide pour la caractérisation des récepteurs précédents;
  - des oligonucléotides pour augmenter sélectivement leur capture par des cellules cibles;
  - une protéine ou des polymères pour le ciblage de médicaments, d'oligonucléotides ou de gènes;

  P, P' et P" possédant au moins une fonction permettant une réaction de condensation par réaction avec un oligopeptide, par exemple

  . une fonction amine (-$NH_2$) permettant la formation d'amide avec un ester actif, d'amidine avec un imidate, de thiourée avec un isothiocyanate,
  . une fonction thiol (-SH) permettant la formation d'un pont disulfure avec un oligopeptide contenant un thiol, d'un thioether avec un oligopeptide contenant un groupement maléimide, ou halogéno-alkyle, ou un halogénoalkanoyle,

. un phénol ($-C_6H_4OH$) permettant la formation d'un azoïque avec un oligopeptide contenant un diazoïque,

sous réserve que Z représente B'-P', et/ou X comporte P et/ou P" dans sa formule.

Dans la formule I et dans celles qui suivent, le groupe oligosidyle provient d'un oligoside dont l'ose terminal est réducteur.

On aura reconnu que dans la définition des composés de formule générale (I) indiquée ci-dessus, la molécule intermédiaire susmentionnée comprend dans sa structure l'entité chimique suivante:

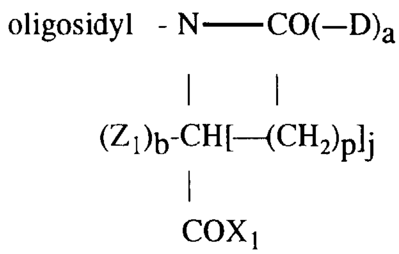

dans laquelle D, a, b, j et p sont tels que définis ci-dessus, $Z_1$ et $X_1$ comportant un ou plusieurs groupes fonctionnels susceptibles de former au moins une liaison avec une (des) molécule(s), particule(s), ou une (des) matrice(s), dont il est question ci-dessus, et $Z_1$ et $X_1$ étant plus précisément définis dans ce qui suit (cf. produits de formule Ia définis ci-après).

Dans les définitions ci-dessus, les acides aminés impliqués ont une configuration L ou D, et de préférence L.

Les composés de l'invention sont donc constitués d'un ou plusieurs oligosides, (identiques ou dont certains sont éventuellement différents) chacun de ces oligosides étant attaché:

- à une molécule, une matrice ou une particule, par l'intermédiaire soit d'un groupe fonctionnel de X, soit d'un groupe fonctionnel de Z,
- ou à deux molécules, matrices ou particules, par l'intermédiaire de groupes fonctionnels respectifs de X et de Z, ou par l'intermédiaire de deux groupes fonctionnels de X, ou à trois molécules, matrices ou particules, par l'intermédiaire de groupes fonctionnels de X et de Z.

Il faut noter que la formule générale (I) ne représente que les possibilités suivantes:

- un seul oligoside fixé à une molécule, une matrice ou une particule, P,
- un seul oligoside fixé à une molécule, une matrice ou une particule, P',
- un seul oligoside fixé à une molécule, une matrice ou une particule, P",
- un seul oligoside fixé à deux molécules, matrices ou particules, respectifs P et P', ou P' et P" ou P et P",
- un seul oligoside fixé à trois molécules, matrices ou particules, P, P' et P".

Une telle représentation a pour but de ne pas compliquer la compréhension de la formule générale (I). Mais on peut envisager la possibilité que plusieurs oligosides (identiques ou dont certains sont éventuellement différents) soient fixés soit à une molécule, une matrice ou une particule, P, soit à une molécule, une matrice ou une particule, P', soit à une molécule, une matrice ou une particule, P", ou que plusieurs oligosides (identiques ou dont certains sont éventuellement différents) soient fixés à deux molécules, matrices ou particules, P et P', ou P et P", ou P' et P", ou que plusieurs oligosides (identiques ou dont certains sont éventuellement différents) soient fixés à trois molécules, matrices ou particules, P, P' et P".

Comme exemple de B' on peut citer:

$-CH_2-S-$

ou

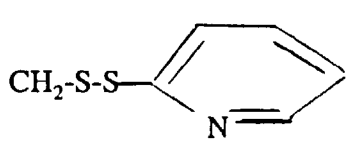

ou $-(CH_2)_3$-NH-
ou $-(CH_2)_4$-NH-

Comme exemple de P, ou P' ou P", on peut citer la polylysine, notamment la polylysine gluconoylée.

Selon un mode de réalisation avantageux de l'invention, un ou plusieurs oligosides sont liés à une même molécule, matrice ou particule, P, constituant des composés pouvant être représentés par la formule générale (II)

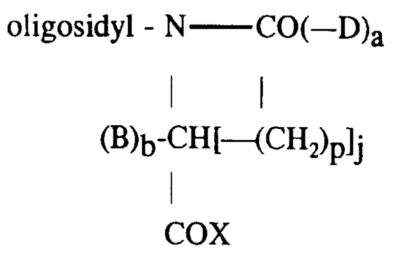

(II)

dans laquelle:

* a = 0 ou 1,
* j = 0 ou 1,
* b = 0 ou 1,
* p = 2 à 4, notamment 2,
* sous réserve que
  ** a = b = 0, lorsque j = 1, ce qui entraîne la présence d'une molécule cyclique,
  ** ou a = b = 1, lorsque j = 0 ce qui implique l'absence de groupe $(CH_2)_p$,
* D représente un résidu d'un acide organique de formule $DCO_2H$, notamment H ou une chaîne alkyle de 1 à 10 atomes de carbone, en particulier $CH_3$,
* B représente H, un alkyle de 1 à 10 atomes de carbone, ou une chaîne latérale d'un acide $\alpha$ aminé tel que $CH(CH_3)_2$, $CH_2OH$, $CH_3$, et de préférence H,
* X représente :
  . soit le groupe $[NH-(A_i)-CO]_m$-P,
  . soit le groupe $[NH-(A_i)-CO]_m$-R-P

  m étant un nombre entier de 0 à 10, de préférence de 0 à 5 et avantageusement 1 ou 2, R et P étant tels que définis ci-après,
* $A_i$ représente un radical organique tel qu'une chaîne alkylidène de 1 à 10 atomes de carbone, notamment $(CH_2)_n$-W-$(CH_2)_{n'}$, n + n' représentant un nombre entier de 0 à 10, W représentant CHY, Y étant H, un alkyle de 1 à 6 atomes de carbone linéaire ou ramifié, un résidu d'amino $\alpha$ acide, naturel ou synthétique, ou W représentant un composé aromatique, notamment phényle,
* R représentant un groupe possédant une fonction alcool, phénol, thiol ou amine,
* P représente :
  - une matrice comme support pour la chromatographie d'affinité;
  - une bille d'or, de latex, pour l'histologie et la cytologie;
  - une protéine pour la visualisation, la purification, etc,
  - des récepteurs spécifiques des osides, récepteurs que l'on appelle lectines, adhésines, agglutinines etc;
  - un lipide pour la caractérisation des récepteurs précédents;
  - des oligonucléotides pour augmenter sélectivement leur capture par des cellules cibles;
  - une protéine ou des polymères pour le ciblage de médicaments, d'oligonucléotides ou de gènes.

Une classe avantageuse de composés selon l'invention répond à la formule générale (III)

**oligosidyl - N-CO-CH$_2$** **(III)**

**| |**

**CH—CH$_2$**

**|**

**COX**

dans laquelle X représente [NH-(A$_i$)-CO]$_m$-P ou [NH-(A$_i$)-CO]$_m$-R-P, A$_i$, m, P et R ayant les significations indiquées ci-dessus.

Une classe avantageuse de composés selon l'invention répond à la formule (III):

**oligosidyl - N-CO-CH$_2$** **(III)**

**| |**

**CH—CH$_2$**

**|**

**COX**

dans laquelle X représente:

**[NH-(A$_i$)-CO]$_m$-P,**

**|**

**P"**

ou

**[NH-(A$_i$)-CO]$_m$-R-P,**

**|**

**P"**

A$_i$, m, P, R et P" ayant les significations indiquées ci-dessus.

Une autre classe avantageuse de composés selon l'invention a comme formule générale (IV)

**oligosidyl - N-CO-D** **(IV)**

**|**

**B-CH-COX**

dans laquelle D et B ont les significations indiquées ci-dessus, et X représente [NH-(A$_i$)-CO]$_m$-P ou [NH-(A$_i$)-CO]$_m$-R-P, A$_i$, m, R et P ayant les significations indiquées ci-dessus.

Une autre classe avantageuse de composés selon l'invention répond à la formule (IV):

oligosidyl - N-CO-D (IV)
|
B-CH-COX

dans laquelle D et B ont les significations indiquées ci-dessus, et X représente:

$[NH-(A_i)-CO]_m-P,$
|
P"

ou

$[NH-(A_i)-CO]_m-R-P,$
|
P"

$A_i$, m, R, P et P" ayant les significations indiquées ci-dessus.

Une autre classe avantageuse de composés selon l'invention a comme formule générale (V)

oligosidyl —— N-CO—$CH_2$ (V)
| |
$CH-CH_2-CH_2$
|
COX

dans laquelle X représente $[NH-(A_i)-CO]_m-P$ ou $[NH-(A_i)-CO]_m-R-P$, P, $A_i$, m et R ayant les significations indiquées ci-dessus.

L'invention a également pour objet de nouveaux produits, susceptibles notamment de servir de produits intermédiaires pour la préparation des composés de l'invention, lesdits produits comprenant un oligoside lié à une molécule possédant un atome d'azote, porté par un carbone en $\alpha$ d'un groupe C = O, et au moins un groupe fonctionnel, notamment un, deux ou trois groupes fonctionnels, la liaison covalente entre l'oligoside et la molécule s'effectuant par l'intermédiaire du susdit atome d'azote.

Les produits de l'invention répondent avantageusement à la formule générale (Ia)

oligosidyl - N——$CO(—D)_a$
| |
$(Z_1)_b-CH[—(CH_2)_p]_j$ (Ia)
|
$COX_1$

dans laquelle:

- * a = 0 ou 1,
- * j = 0 ou 1,
- * b = 0 ou 1,
- * p = 2 à 4, notamment 2,
- * sous réserve que
  - ** a = b = 0, lorsque j = 1, ce qui entraîne la présence d'une molécule cyclique,
  - ** ou a = b = 1, lorsque j = 0 ce qui implique l'absence de groupe $(CH_2)_p$,
- * D représente un résidu d'un acide organique de formule $DCO_2H$, notamment H ou une chaîne alkyle de 1 à 10 atomes de carbone, en particulier $CH_3$,
- * $Z_1$ représente
  - * B, B étant choisi parmi: H, une chaîne alkyle de 1 à 10 atomes de carbone, ou une chaîne latérale d'un acide α aminé tel que $CH(CH_3)_2$, $CH_2OH$, $CH_3$, et de préférence H, ou
  - * B', B' étant choisi parmi: une chaîne alkylidène de 1 à 10 atomes de carbone, ou une chaîne latérale d'un acide α aminé, lesdites chaînes contenant un groupe fonctionnel tel que carboxylique, SH, OH ou amine, libre ou protégé,
- * $X_1$ représente
  - . le groupe $[NH-(A_i)-CO]_m-R$,
  - . ou le groupe $[NH-(A_i)-CO]_m-Q$
- * R représente un composé possédant une fonction alcool, phénol, thiol ou amine,
- * Q représente OH, $OCH_3$, $OCH_2-C_6H_5$, $O-C_6H_5$, $O-C_6F_5$, $O-pC_6H_4-NO_2$,

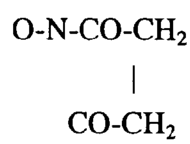

- * m étant un nombre entier de 0 à 10, de préférence de 0 à 5 et avantageusement 1 ou 2,
- * $A_i$ représente un radical organique tel qu'une chaîne alkylidène de 1 à 10 atomes de carbone, notamment $(CH_2)_n-W-(CH_2)_{n'}$, n + n' représentant un nombre entier de 0 à 10, W représentant CHY, Y étant H, un alkyle de 1 à 6 atomes de carbone linéaire ou ramifié, un résidu d'amino α acide, naturel ou synthétique, ou W représentant un composé aromatique, notamment phényle.

Ces groupes sont des dérivés de glycosylamine acylée.

Les dérivés de glycosylamine acylés sont stables, en milieu aqueux dans une large gamme de pH de part et d'autre de la neutralité. Ceci signifie qu'il se produit une hydrolyse inférieure à 1% à pH 5-8, pendant 24 h, quelle que soit la température entre 0 et 95°C.

Les sudits produits de formule (Ia) peuvent être utilisés tels quels.

Les susdits produits de formule (Ia) peuvent également être utilisés pour préparer les composés de l'invention de formule I.

Une classe avantageuse de produits selon l'invention, répond à la formule générale (IIa)

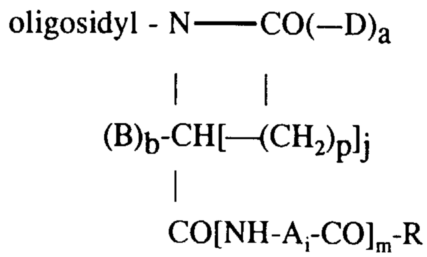

(IIa)

dans laquelle:

* a = 0 ou 1,
* j = 0 ou 1,
* b = 0 ou 1,
* p = 2 à 4, notamment 2,
* sous réserve que
  ** a = b = 0, lorsque j = 1, ce qui entraîne la présence d'une molécule cyclique,
  ** ou a = b = 1, lorsque j = 0 ce qui implique l'absence de groupe $(CH_2)_p$,
* D représente un résidu d'un acide organique de formule $DCO_2H$, notamment H ou une chaîne alkyle de 1 à 10 atomes de carbone, en particulier $CH_3$,
* B représente H, un alkyle de 1 à 10 atomes de carbone, ou une chaîne latérale d'un acide α aminé tel que CH $(CH_3)_2$, $CH_2OH$, $CH_3$, et de préférence H,
* m étant un nombre entier de 0 à 10, de préférence de 0 à 5 et avantageusement 1 ou 2,
* $A_i$ représente un radical organique tel qu'une chaîne alkylidène de 1 à 10 atomes de carbone, notamment $(CH_2)_n$-W-$(CH_2)_{n'}$, n + n' représentant un nombre entier de 0 à 10, W représentant CHY, Y étant H, un alkyle de 1 à 6 atomes de carbone linéaire ou ramifié, un résidu d'amino α acide, naturel ou synthétique, ou W représentant un composé aromatique, notamment phényle,
* R représente un composé possédant une fonction alcool, phénol, thiol ou amine.

Les susdits produits de formule (IIa) peuvent être utilisés pour préparer les composés de formule (II).

Une autre classe avantageuse de produits selon l'invention répond à la formule générale (IIIa)

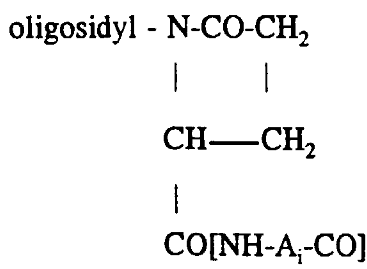

**(IIIa)**

dans laquelle $A_i$, m et R ont les significations indiquées ci-dessus.

Les susdits produits de formule (IIIa) peuvent être utilisés pour préparer les composés de formule (III).

Une autre classe avantageuse de produits selon l'invention répond à la formule générale (IVa)

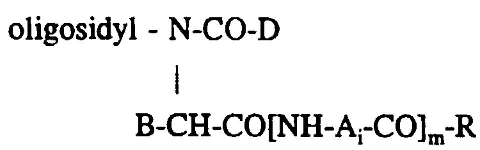

**(IVa)**

dans laquelle D, B, m, $A_i$ et R ont les significations indiquées ci-dessus.

Les susdits produits de formule (IVa) peuvent être utilisés pour préparer les composés de formule (IV).

Une autre classe avantageuse de produits selon l'invention répond à la formule générale (Va)

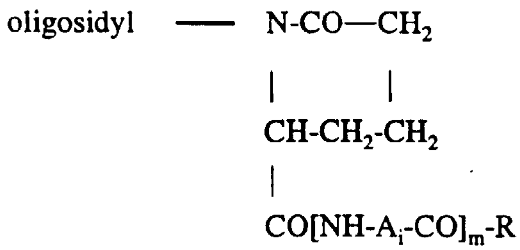

(Va)

dans laquelle $A_i$, m et R ont les significations indiquées ci-dessus.

Les susdits produits de formule (Va) peuvent être utilisés pour préparer les composés de formule (V).

Les produits de formule (VI)

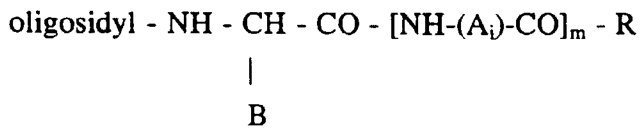

(VI)

dans lesquels B, $A_i$, m et R ont les significations indiquées ci-dessus, sont des produits intermédiaires obtenus au cours de la préparation des produits définis ci-dessus et sont nouveaux.

Les produits de formule (VII)

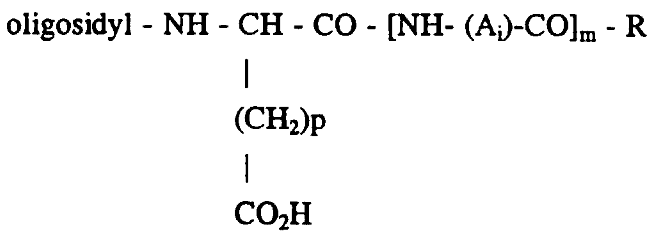

(VII)

dans lesquels p, $A_i$, R, et m ont les significations indiquées ci-dessus, sont des produits intermédiaires obtenus au cours de la préparation des produits définis ci-dessus et sont nouveaux.

Dans tous les composés ou produits de l'invention, R peut représenter les radicaux suivants:

OH, $O-CH_2-C_6H_5$, $O-C_6H_5$, $O-C_6F_5$, $O-pC_6H_4-NO_2$,

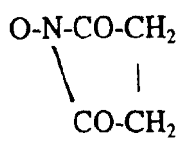

$-NH-pC_6H_4-N=C=S$ et ses précurseurs:

$-NH-pC_6H_4-NO_2$

$$-NH-pC_6H_4-NH_2$$

$$-NH-CH_2-(CH_2)_m-C(=NH_2^+)OCH_3$$

$$-NH-CH_2-(CH_2)_m-CN$$

$$-NH-CH_2-(CH_2)_m-CH_2-NH-CO-CH_2-(CH_2)_m-C(=NH_2^+)OCH_3$$

$$-NH-CH_2-(CH_2)_m-CH_2-NH-CO-CH_2-(CH_2)_m-CN$$

ces composés permettant une addition sur un composé aminé.

R peut également représenter:

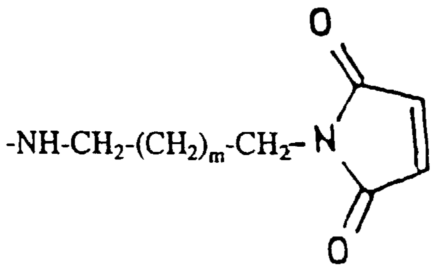

dérivé de maléimide

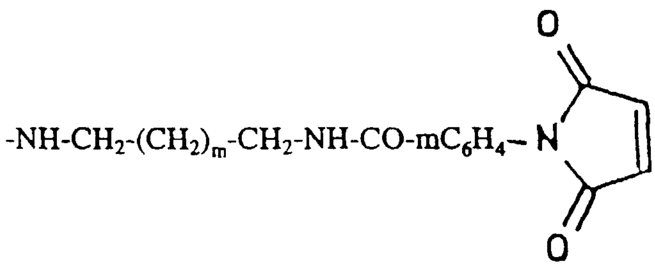

dérivé de *m*-maleimidyl benzoyl

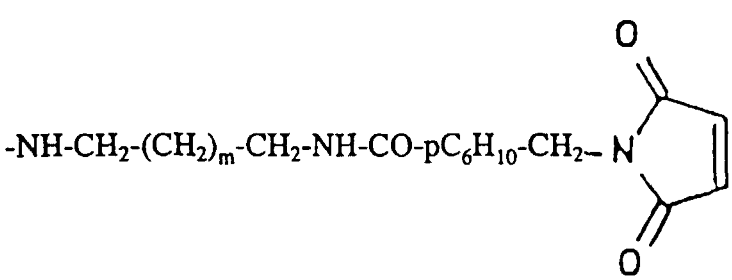

dérivé de N-méthylmaléimidyl p-cyclohexylcarboxy-

$-NH-CH_2-(CH_2)_m-CH_2-NH-CO-CH_2-T$ T = Br, I, Cl

dérivé de halogénoacétyl

$-NH-CH_2-CH_2-NH-CO-CH_2-(CH_2)_m-S-S-Pyr$

dérivé de dithiopyridine
N.B.: -Pyr: -2-pyridine

$-NH-CH_2-(CH_2)_m-CH_2-S-S-Pyr$

dérivé de dithiopyridine
ces composés permettant une addition sur un thiol.

R peut également représenter:

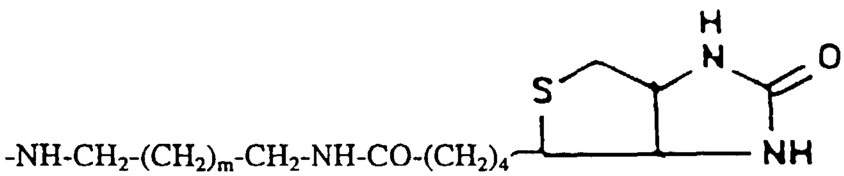

dérivé de biotine
ce composé comprenant un résidu biotine permettant la formation d'un complexe avec l'avidine ou la streptavidine.

R peut également représenter:

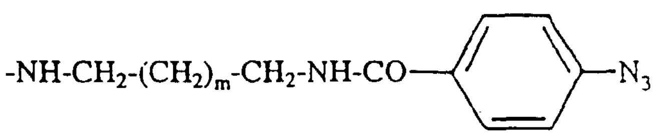

dérivé du 4-azidobenzoyl

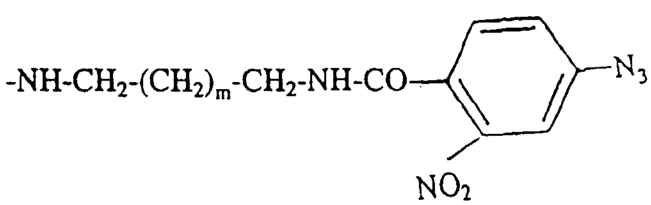

dérivé du 4-azido 2-nitrobenzoyl

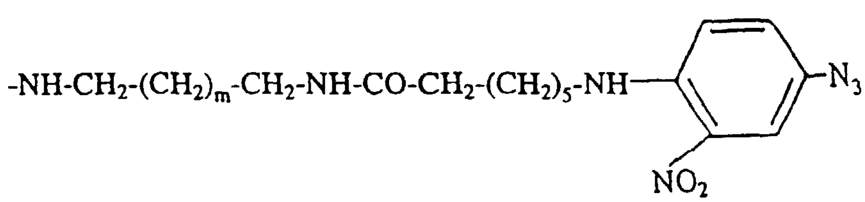

dérivé de 4-azido 2-nitroanilide

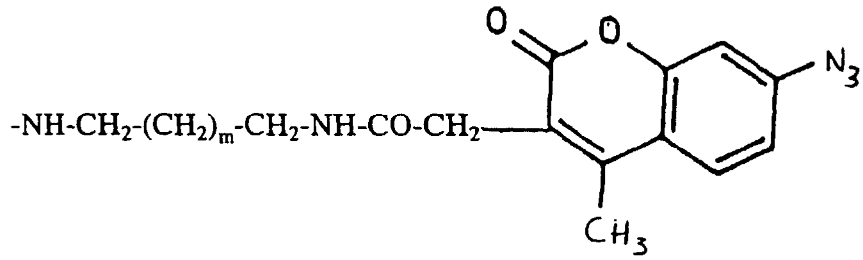

dérivé du 7-azido 4-méthylcoumarine 3-acétyl

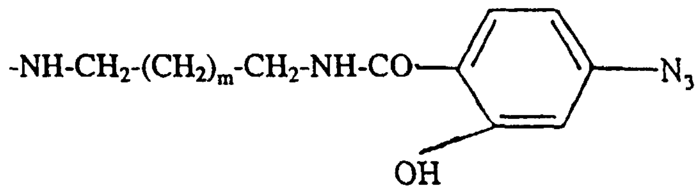

dérivé du 4-azidosalicyclique ou du 4-azido 2-hydroxybenzoyle

ces composés permettant une fixation covalente sur un récepteur, une enzyme, un anticorps, spécifiques de la partie glucidique par activation photonique.

R peut également représenter:

$$-NH-(CH_2)_m-pC_6H_4OH$$

$$-NH-CH_2-(CH_2)_m-CH_2-NH-CO-(CH_2)_m-pC_6H_4OH$$

ces composés permettant la fixation d'un ou deux atomes d'iode, particulièrement d'un atome d'iode radio-actif.

R peut également représenter:

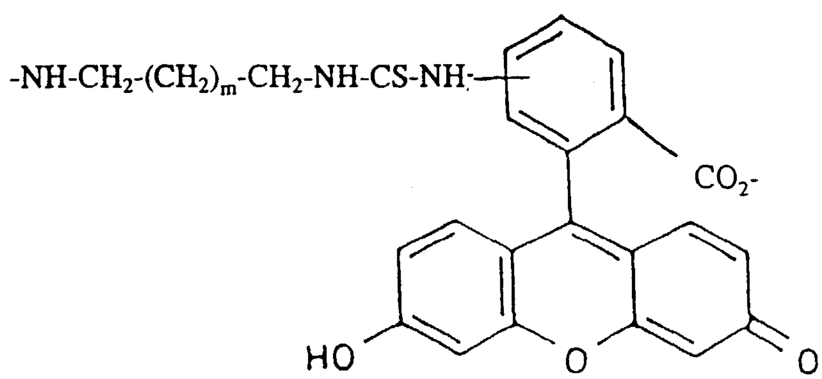

dérivé de fluorescéine

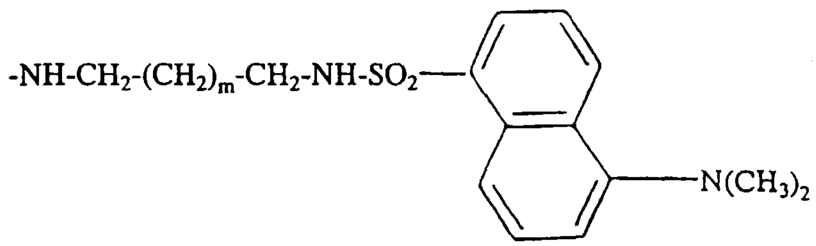

dérivé de dansyle

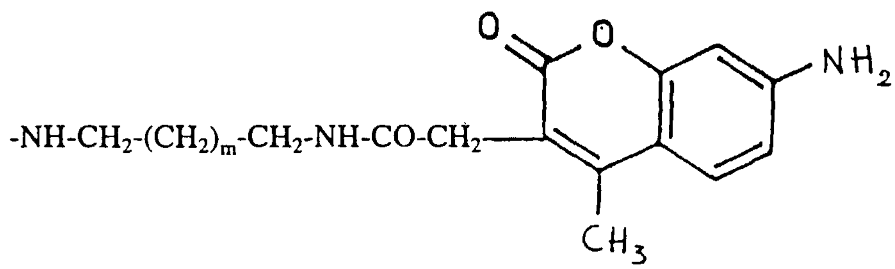

dérivé de 7-amino 4-méthylcoumarin 3-acétyl

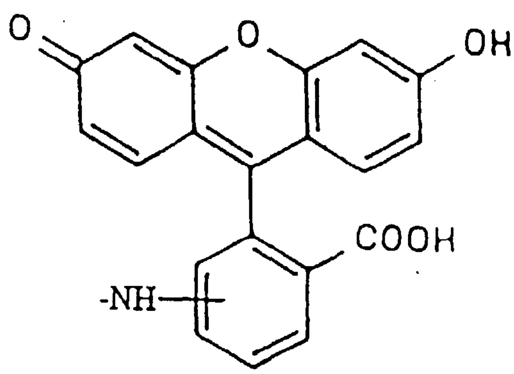

dérivé de l'amino-fluorescéine

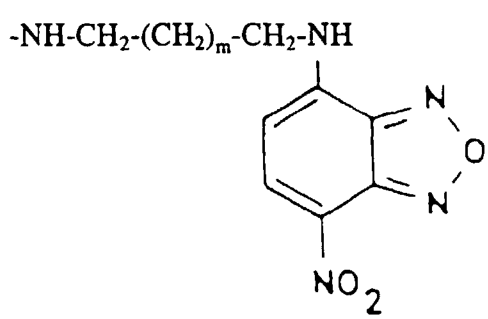

dérivé du nitrobenzoxadiazole
ces composés fluorescents permettant de visualer la position de l'oligosidylpeptide dans une cellule, un tissu,

un organe, sur un gel ou une bande d'électrophorèse, etc...

Dans les composés de l'invention, P peut représenter un oligopeptide, ou un polypeptide, notamment la polylysine gluconoylée, et P' ou P" peut représenter un oligonucléotide, ou R peut représenter la fluorescéine ou un de ses dérivés ou un autre dérivé fluorescent, et P' ou P" peut représenter un oligonucléotide. P peut également représenter un agent thérapeutique ou toute molécule d'intérêt.

Dans les composés et produits de l'invention, le résidu oligosidique comporte de 2 à 50 oses et notamment est choisi parmi

| | |
|---|---|
| lacto-N-tétraose | Galβ 3 GlcNAcβ 3 Gal β 4 Glc |
| néolacto-N-tétraose | Galβ 4 GlcNAcβ 3 Gal β 4 Glc |
| Groupe H | Fuc α 2 Galβ 3 Galβ 4 Glc |
| Lewis$^a$ | Galβ 3 GlcNAcβ 3 Gal β 4 Glc<br>Fuc α 4-↑ |
| Lewis$^x$ | Galβ 4 GlcNAcβ 3 Galβ 4 Glc<br>Fuc α 3-↑ |
| Lewis$^b$ | Fucα 2 Galβ 3 GlcNAc β 3 Galβ<br>4 Glc Fuc α 4-↑ |
| Lewis$^y$ | Fuc α 2 Galβ 4 GlcNAcβ 3 Galβ<br>4 Glc Fuc α 3-↑ |
| Disialolacto-N-tétraose | Neu 5Acα 3 Gal β 3 GlcNAc β 3 Gal β 4 Glc<br>Neu 5Acα 6-↑ |

Type complexe à 3 antennes

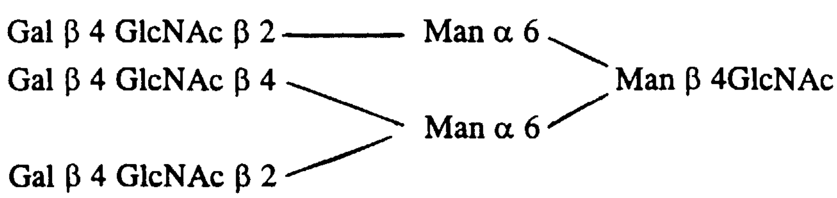


| | |
|---|---|
| Sialylactose 3 | Neu 5Ac α 3 Gal 4 Glc |
| Sialylactose 6 | Neu 5Ac α 6 Gal β 4 Glc |
| Disialylactose 3 | Neu 5Ac α 8 Neu 5Ac α 3 Gal β 4 Glc |

- des osides simples ou complexes reconnus par des lectines membranaires, et choisis parmi:

**a. Asialo-oligoside de type lactosamine triantenné: récepteur d'asialoglycoprotéine**

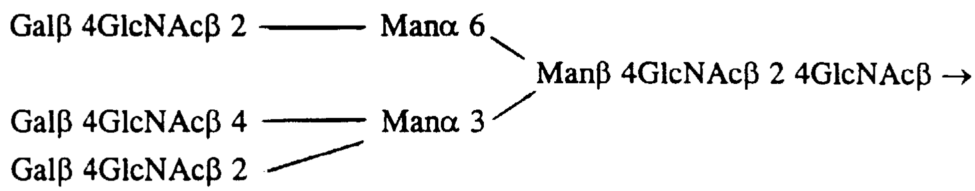

**b. Asialo oligoside de type lactosamine tétraantenné: récepteur d'asialoglycoprotéine**

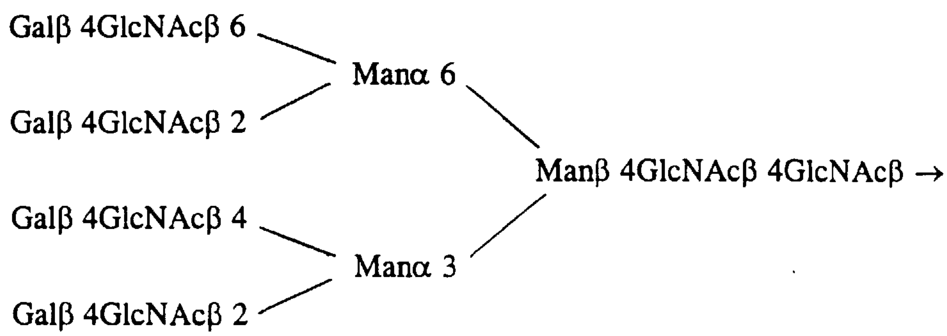

**c. Lewis $^{x}$: LECAM 2/3**

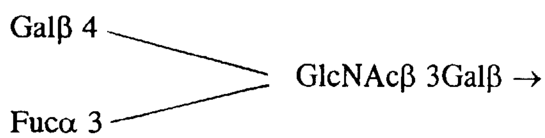

**d. Sialyl Lewis $^{x}$ : LECAM 3/2**

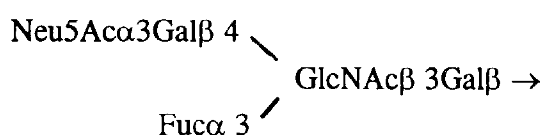

**e. Dérivé de Lewis $^{x}$ sulfaté (HNK1): LECAM 1**

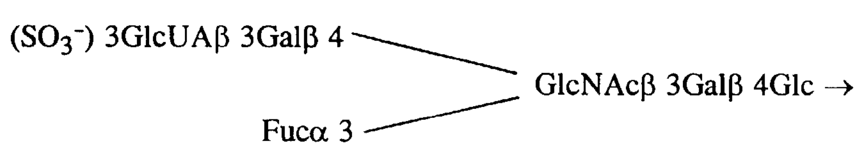

**f. Oligomannoside: récepteur du mannose**

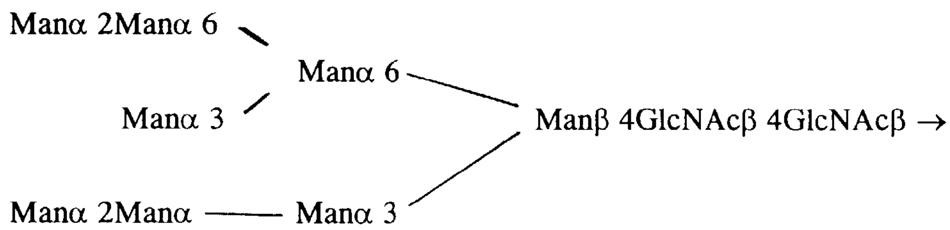

**g. Oligomannoside phosphorylé: récepteur de mannose 6 phosphate**

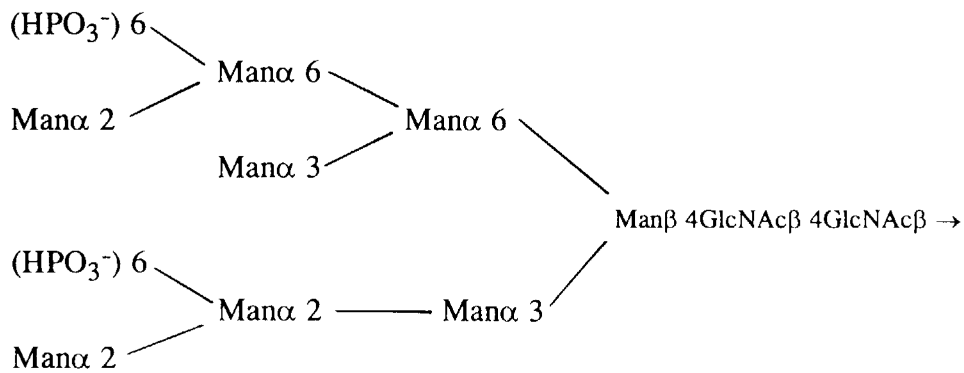

**h. Oligosaccharide de type lactosamine sulfaté: récepteur de GalNAc 4 sulfaté**

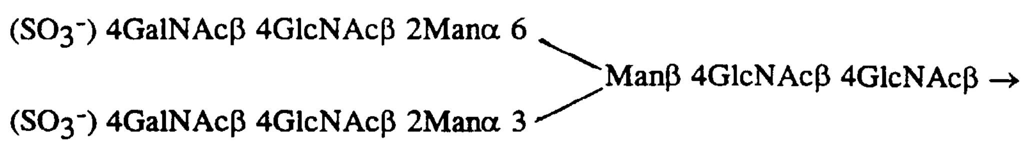

Pour préparer les composés de l'invention, il faut préalablement préparer les produits (dérivés de glycosylamine acylés), qui servent notamment d'intermédiaires à la préparation desdits composés de l'invention.

L'invention concerne également un procédé de préparation des produits (dérivés de glycosylamine acylés) définis ci-dessus, caractérisé en ce que:

- l'on condense un oligoside ayant un ose **terminal** réducteur libre, sur l'atome d'azote d'une molécule intermédiaire, cet atome d'azote appartenant à un groupe amine, lié à un atome de carbone placé en α d'un groupe C = O, la molécule intermédiaire possédant éventuellement une chaîne latérale contenant un groupe fonctionnel tel que OH, SH, $NH_2$ ou COOH, libre ou protégé, cette molécule intermédiaire étant choisie parmi les molécules intermédiaires suivantes: acide α aminé, naturel ou synthétique, dérivé d'acide α aminé, aminoacide en position N-terminale d'un peptide, ou d'un dérivé peptidique, éventuellement en présence d'un catalyseur tel que l'imidazole, dans un solvant approprié pour obtenir un dérivé de glycosylamine dans lequel l'ose terminal de l'oligoside conserve sa structure cyclique, et dans lequel l'hydroxyle semiacétalique est remplacé par l'amine α de l'une des susdites molécules de départ,
- lorsque la molécule intermédiaire ne possède pas de chaîne latérale contenant un groupe fonctionnel tel que défini ci-dessus, ou possède une chaîne latérale dont le groupe fonctionnel est éventuellement protégé, on acyle le dérivé de glycosylamine obtenu à l'issue de l'étape précédente par addition d'un acide organique activé par un activateur classique tel que le carbonyl diimidazole, le BOP (benzotriazolyl N-oxy-tris(diméthylamino) phosphonium hexafluorophosphate) ou HBTU (*O*-benzotriazol-1-yl-*N,N,N',N'*,tétraméthyluronium hexafluorophosphate) pour obtenir un dérivé de glycosylamine N-acylé, suivi éventuellement d'une déprotection du groupe fonctionnel de la susdite chaîne latérale, en vue d'une éventuelle substitution,

- lorsque la molécule intermédiaire possède une chaîne latérale contenant un groupe carboxylique, on active le susdit groupe carboxylique afin qu'il réagisse intramoléculairement avec la susdite amine a, entraînant une cyclisation à l'intérieur de la susdite molécule intermédiaire, pour obtenir un dérivé de glycosylamine N-acylé,
- lorsque la molécule intermédiaire possède une chaîne latérale contenant un groupe carboxylique, on peut ajouter l'agent d'acylation sous forme d'un ester actif.

Le susdit procédé de préparation de l'invention des dérivés de glycosylamine comprend donc deux étapes, une étape de condensation d'un oligoside sur une molécule intermédiaire pour obtenir un dérivé de glycosylamine, et une étape d'acylation du susdit dérivé de glycosylamine.

Dans l'étape d'acylation envisagée dans le procédé de l'invention, on utilise toujours un activateur, que la molécule intermédiaire possède ou non un chaîne latérale contenant un groupe fonctionnel.

Par ailleurs, il faut préciser que l'étape de condensation de l'oligoside sur la molécule intermédiaire, pour obtenir un dérivé de glycosylamine, ainsi que l'étape d'acylation du susdit dérivé de glycosylamine sont effectuées en présence de solvants organiques appropriés.

L'un des avantages d'utiliser un solvant organique est notamment de permettre de coupler aux oligosides des peptides et dérivés qui sont peu ou très peu solubles dans l'eau.

Ces cas peuvent être schématisés de la façon suivante:

**1**) la molécule intermédiaire ne possède pas de chaîne latérale contenant un groupe fonctionnel,

$$\text{Oligoside} + NH_2\text{-}CH(R_1)\text{-}CO\text{-}R_2 \Rightarrow \text{oligosidyl-}NH\text{-}CH(R_1)\text{-}CO\text{-}R_2$$

$$+ R_3\text{-}CO_2H + \text{activateur}$$

$$\downarrow$$

$$\text{Oligosyl-}N(CO\text{-}R_3)\text{-}CH(R_1)\text{-}CO\text{-}R_2$$

- . $R_1$ représentant un résidu d'une molécule organique ne comportant pas de groupement fonctionnel protégé, ou $R_1$ pouvant également représenter H;
- . $R_2$ représentant un résidu d'une molécule organique telle que $-CO-R_2$, soit un ester ou un amide:
- . $R_3$ représentant un résidu d'une molécule organique ne comportant pas, de préférence, de groupement fonctionnel libre.

L'ensemble $R_3$-$CO_2H$ + activateur, peut être remplacé par le produit activé ou par un anhydride.

Dans ce qui précède, on peut aussi inclure le cas où $R_3$ possède un groupement fonctionnel, et à cet égard, on peut se reporter au paragraphe 1 bis ci-après.

**1 bis**) la molécule intermédiaire ne possède pas de chaîne latérale contenant un groupe fonctionnel, mais l'agent d'acylation est bifonctionnel,

$$\text{oligoside} + NH_2\text{-}CH(R_1)\text{-}CO\text{-}R_2 \Rightarrow \text{oligosyl-}NH\text{-}CH(R_1)\text{-}CO\text{-}R_2$$

$$+ R_3\text{-}CO_2H + \text{activateur}$$

$$\downarrow$$

$$\begin{array}{l} \quad R_3\text{-}CO \\ \qquad | \\ \text{oligosyl-}N\text{-}CH(R_1)\text{-}CO\text{-}R_2 \end{array}$$

- $R_3$ représentant une molécule organique comportant un second groupement fonctionnel tel que, en particulier SH, libre ou protégé, ou $-CO_2H$.

Alternativement, l'ensemble $R_3$-$CO_2H$ + activateur peut être remplacé par le produit d'activation: $R_3$-CO-activé. Par exemple, $R_3$-CO-Cl, $(R_3\text{-}CO)_2O$ ou

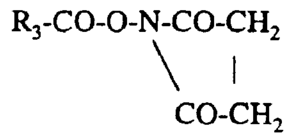

ou encore un anhydride cyclique:

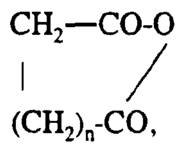

par exemple avec n entier égal à 1, 2, 3 ou 4;
ou encore un thioester:

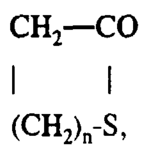

avec n entier égal à 1, 2, 3 ou 4, de préférence n = 2

Lorsque l'acylation de l'azote lié sur l'oligoside est acylé par un anhydride cyclique, le produit obtenu est du type:

$$\text{Oligosyl-N-(CO-CH}_2\text{-(CH}_2)_n\text{-CO}_2\text{H)-CH(R}_1\text{)-CO-R}_2$$

Le groupe carboxylique est utilisable pour une réaction de couplage sur une molécule organique ou une matrice, ou une particule possédant un groupement fonctionnel (hydroxyle ou amine par exemple).

Lorsque l'acylation de l'azote lié sur l'oligoside est acylé par un thio ester cyclique, le produit obtenu est du type:

$$\text{Oligosyl-N-(CO-CH}_2\text{-(CH}_2)_n\text{-SH)-CH(R}_1\text{)-CO-R}_2$$

Le groupe thiol est utilisable pour une réaction de couplage sur une molécule soluble ou non, capable d'être substituée par un thiol, par exemple un dithiopyridine ou un dérivé maléimide.

**2**) la molécule intermédiaire possède une chaîne latérale fonctionnelle et on n'effectue pas de cyclisation,

$$\text{Oligoside} + NH_2\text{-}CH(R_4)\text{-}CO\text{-}R_2 \Rightarrow \text{oligosyl-NH-}CH(R_4)\text{-}CO\text{-}R_2$$

$$+ R_3\text{-}CO_2H + \text{activateur}$$

$$\downarrow$$

$$\text{Oligosyl-N}(CO\text{-}R_3)\text{-}CH(R_4)\text{-}CO\text{-}R_2,$$

- $R_2$ et $R_3$ ayant les significations indiquées ci-dessus, et $R_4$ représentant un résidu d'une molécule organique possédant un groupement fonctionnel, en particulier un groupement carboxylique, $R_4$ représentant notamment $CH_2$-$CH_2$-$CO_2H$.

Dans ce cas, on utilise préférentiellement un produit d'activation de $R_3$-$CO_2H$ tel que défini plus haut.

Le groupement fonctionnel contenu dans $R_4$ est disponible pour une réaction de condensation ou de substitution sur une molécule soluble ou insoluble ou sur une matrice, ou une particule comportant un groupement susceptible de donner une liaison covalente avec le groupement fonctionnel de $R_4$, par exemple une amine lorsque $R_4$ comporte un groupement carboxylique.

**3**) La molécule intermédiaire possède une chaîne latérale contenant un groupe fonctionnel carboxylique, et on effectue une cyclisation, et on peut fixer la molécule sur 1 ou 2 molécule(s), matrice(s) ou particule(s),

$$\text{Oligoside} + NH_2\text{-}CH(R_5\text{-}CO_2H)\text{-}CO\text{-}R_2 \Rightarrow \text{oligosyl-NH-}CH(R_5\text{-}CO_2H)\text{-}CO\text{-}R_2$$

$$+ \text{activateur}$$

$$\downarrow$$

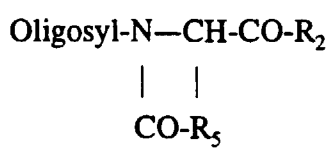

- $R_2$ ayant les significations indiquées ci-dessus,
- $R_5$-$CO_2H$ étant un résidu d'une molécule organique telle que -$(CH_2)_n$-$CO_2H$
- n étant un nombre entier de 1 à 10, de préférence 2 ou 3.

Le glycopeptide ainsi obtenu peut être utilisé en faisant réagir un groupement fonctionnel existant à l'état libre ou transformé en groupement actif sur $R_2$. Par exemple, si $R_2$ représente le groupe para-nitroanilide, le groupement $NO_2$ est réduit en $NH_2$, puis transformé en isothiocyanate -N = C = S, qui est un excellent réactif vis à vis des amines et des alcools.

Comme exemples de molécules intermédiaires, on peut citer:

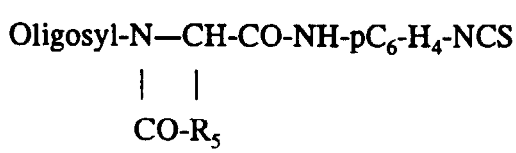

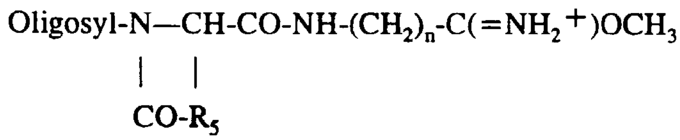

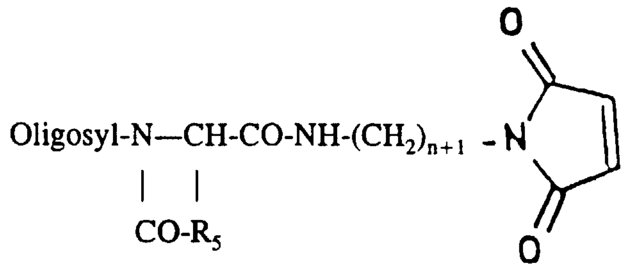

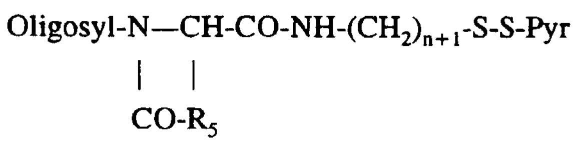

avec n = 1 à 10.

L'invention concerne également la préparation des composés de l'invention, caractérisée en ce que l'on fait réagir un ou plusieurs produits (dérivés de glycosylamine acylés) de l'invention, portant soit un groupe R tel que défini ci-dessus, activé ou activable, soit un groupe B' contenant un groupe fonctionnel activé ou activable, soit un groupe $A_i$ contenant un groupe fonctionnel pouvant réagir sur une molécule, matrice ou particule, respectivement P, P' ou P", contenant un groupe fonctionnel, pour obtenir un produit du type:

(glycopeptidyl-R)$_n$-P, (glycopeptidyl -B')$_n$-P' ou (glycopeptidyl-$A_i$)$_n$-P", n étant supérieur ou égal à 1.

L'invention concerne également la préparation de composés de l'invention, caractérisée en ce que l'on fait réagir un ou plusieurs produits (dérivés de glycosylamine acylés) de l'invention, portant d'une part un groupe R tel que défini ci-dessus, activé ou activable, d'autre part un groupe B' contenant un groupe activé ou activable, ou un groupe $A_i$ contenant un groupe fonctionnel sur des molécules, matrices ou particules P et P', ou des molécules, matrices ou particules P et P".

L'invention concerne également la préparation de composés de l'invention, caractérisée en ce que l'on fait réagir un ou plusieurs produits (dérivés de glycosylamine acylés) de l'invention, portant d'une part un groupe R tel que défini ci-dessus, activé ou activable, d'autre part un groupe B' contenant un groupe activé ou activable, et un groupe $A_i$ contenant un groupe fonctionnel respectivement sur des molécules, matrices ou particules P, P' et P".

Les molécules, matrices ou particules entrant dans la préparation des composés de l'invention peuvent avantageusement être une molécule naturelle ou synthétique, soluble ou non dans un solvant organique, aqueux ou hydroorganique, une nanoparticule, une vésicule lipidique, une matrice insoluble dans un solvant organique, aqueux ou hydroorganique, une protéine, un lipide, un acide nucléique, un oligonucléotide, une polylysine, un polymère insoluble dans un solvant organique, aqueux ou hydroorganique, une bille de latex ou une bille d'or, etc ...

De façon détaillée, s'agissant du procédé selon l'invention, l'oligoside, ayant un ose réducteur libre est placé dans un solvant approprié, le diméthylsulfoxyde, la N-méthylpyrrolidone ou le diméthylformamide, par exemple, en présence d'une quantité équivalente ou de deux quantités équivalentes d'une molécule de départ choisie parmi: un acide aminé naturel ou synthétique, un peptide, un dérivé d'acide aminé ou un dérivé de peptide.

Par solvant approprié, on désigne un solvant permettant, d'une part la solubilisation des composés à condenser, et d'autre part, la solubilisation des composés résultant de la condensation.

Le produit principal de la réaction est le produit de condensation du type "dérivé de glycosylamine": l'ose terminal réducteur conserve sa structure cyclique, son hydroxyl semiacétalique est remplacé par l'amine $\alpha$ de l'aminoacide, du dérivé d'aminoacide, ou de l'aminoacide en position N terminale d'un peptide ou d'un dérivé peptidique.

Dans un second temps, le dérivé de glycosylamine ainsi formé est acylé par addition d'un acide organique activé ou dans le cas d'acide a aminé portant une chaine latérale contenant un groupe fonctionnel telle qu'une chaine latérale carboxylique, comme dans le cas de l'acide glutamique ou de ses homologues, par addition d'un activateur

de groupement carboxylique.

Le produit ainsi formé est un dérivé de glycosylamine N-acylé.

Les dérivés glycosylamine acylés sont isolés par chromatographie de tamisage moléculaire ou par toute autre technique de purification classique connue de l'homme de l'art.

Les dérivés de glycosylamine acylés sont ensuite utilisés pour substituer un composé (protéine, lipide, acide nucléique, oligonucléotide, polylysine, polymère insolubles, billes de latex, bille d'or, etc).

On tire partie de la fraction aminoacide, ou du peptide ou d'un substituant du peptide pour réaliser la réaction de condensation, de façon à ce que l'oligoside conserve toutes ses propriétés et son accessibilité pour servir de substrats ou de signal de reconnaissance.

Selon le schéma général suivant :

$$\text{oligoside} + NH_2 - CHB - CO - R$$

$$\Rightarrow \text{oligosidyl} - NH - CHB - CO - R$$

$$\Rightarrow \text{oligosidyl} - \underset{\displaystyle CHB\text{-}CO\text{-}R}{\underset{|}{N}} - CO\text{-}D$$

$$\Rightarrow \text{oligosidyl} - \underset{\displaystyle CHB\text{-}CO\text{-}P}{\underset{|}{N}} - CO\text{-}D$$

Par exemple, la condensation du lactose avec le glycylparanitroanilide s'écrit

$$Gal\beta 4\ Glc + NH_2 - CH_2 - CO - NH - pC_6H_4 - NO_2$$

$$\Rightarrow Gal\beta 4\ Glc\beta - NH - CH_2 - CO - NH - pC_6H_4 - NO_2$$

L'addition d'acide acétique et d'un activateur d'acide organique conduit à:

$$\Rightarrow Gal\beta 4\ Glc\beta - \underset{\displaystyle CH_2 - CO - NH - pC_6H_4 - NO_2}{\underset{|}{N}} - CO - CH_3$$

Dans l'exemple choisi, le groupement nitro peut être réduit quantitativement en amine, puis l'amine est transformée quantitativement en isothiocyanate (selon Roche et al. 1983, G. Cell Biochem. 22, 131-140, Monsigny et al. 1984, Biol. Cell 21, 187-196).

Le composé ainsi activé peut réagir en milieu légèrement alcalin sur une amine portée par une protéine, un lipide, un polymère, (la polylysine par exemple), un support solide comportant des groupements amine, ou un oligonucléotide substitué par une amine, ou encore sur une amine portée par une molécule ou un corps appropriés, telle que $NH_2$ - P.

On peut écrire, à titre d'exemple, le schéma réactionnel suivant:

=> Galβ4 Glcβ - N - CO - $CH_3$
|
$CH_2$ - CO - NH - $pC_6H_4$ - $NO_2$

↓ $H_2$

=> Galβ4 Glcβ - N - CO - $CH_3$
|
$CH_2$ - CO - NH - $pC_6H_4$ - $NH_2$

↓ $CsCl_2$

=> Galβ4 Glcβ - N - CO $CH_3$
|
$CH_2$ - CO - NH - $pC_6H_4$ - N = C = S

↓ $PNH_2$

=> Galβ4 Glcβ - N - CO $CH_3$ S
| ||
$CH_2$ - CO - NH - C - NH - P

Dans le cas où l'amino acide ou le dérivé est un acide α aminé possédant une chaine latérale contenant un groupement carboxylique comme c'est le cas pour le glutamate, la réaction s'écrit, par exemple:

Galβ4 Glc + Glu NH - $pC_6H_4$ - $NO_2$

=> Galβ4 Glcβ - NH - CH - CO - NH - $pC_6H_4$ - $NO_2$
|
$CH_2$ - $CH_2$ - $CO_2$ - H

L'addition d'un activateur de groupement carboxylique conduit au produit attendu:

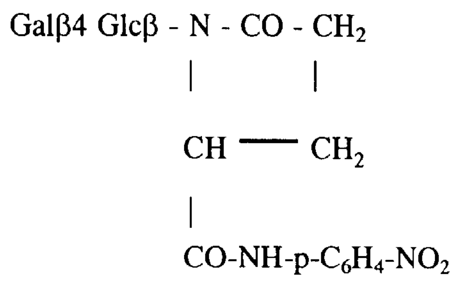

De façon analogue, ce composé pourra être activé et pourra réagir sur une aminé $NH_2$ - P, pour donner le produit final:

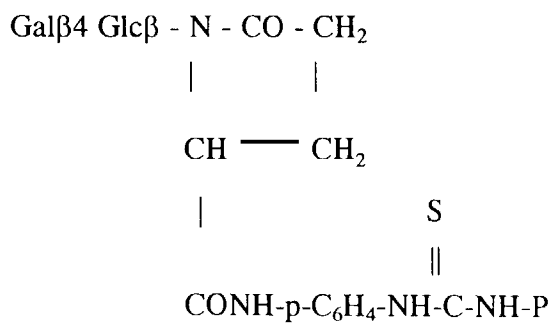

**EXEMPLES**

Exemple 1

Préparation d'un dérivé de glycosylamine acylé: le N-acétyl lactosyl β-glycyl-pNA.

Le glycyl amido paranitrophenyl (0,1 mmole) et le lactose (0,1 mmole) sont dissous dans 1 ml de diméthylsulfoxyde.
La solution est maintenue à 50°C pendant 48 h. On ajoute 0,1 mmole de gly-pNA (pNA = paranitroanilide) dans 0,5 ml de diméthylsulfoxyde aux temps 12 h, 24 h et 36 h. On refroidit à 25°C.
On ajoute ensuite 0,44 mmole du BOP, hexafluorophosphate, benzotriazolyl 1 yl-tris (diméthylamino) phosphonium, et 0,44 mmole d'acétate de diisopropyléthylamine et on agite 3 h à 25°C.
Le produit attendu est purifié par tamisage moléculaire dans une colonne d'Ultrogel GF05 (90 cm x 2,3) en utilisant comme solvant l'acide acétique 0,1, M contenant 3 % de *n*-butanol; avant l'injection, les produits de réaction sont dilués par addition de 7,5 ml de solvant de chromatographie.
Le produit attendu sort en tête, suivi des réactifs en excès et du diméthylsulfoxyde.
Le produit: le N-acétyl (lactosylβ)-gly-pNA est obtenu par lyophilisation de la solution éluée de la colonne.

Exemple 2

Préparation d'un dérivé de glycosylamine acylé intramoléculairement: le N-lactosylβ-*pyro*glu-pNA.

L'α glutamyl paranitroanilide (Glu-pNA) (0,1 mmole) et le lactose (0,1 mmole) sont dissous dans 1 ml de diméthylsulfoxyde. La solution est maintenue à 50°C pendant 48 h. On ajoute 0,1 mmole de glu-pNA dissous dans 0,5 ml de dimethylsulfoxyde aux temps 12 h, 24 h, et 36h. On refroidit ensuite à 25°C.
On ajoute alors 0,44 mmole de BOP et on agite 3 h à 25°C.
Le produit attendu est purifié dans les mêmes conditions que dans l'exemple précédent.

Exemple 3

Préparation d'un composé organique dont la chaîne latérale de l'amine contient un groupe fonctionnel et utilisation du groupe fonctionnel pour former un conjugué avec un oligonucléotide.

L'oligoside est incubé en présence de deux à quatre équivalents du dérivé S-(thio-2-pyridine)cystéinyl-*para*-nitroanilide

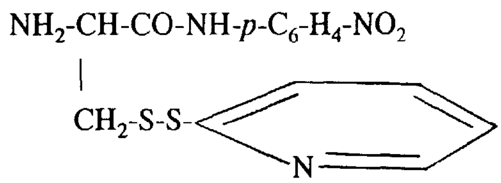

en solution dans la *N*-méthylpyrrolidone (ou dans du diméthylsulfoxyde ou du *N*-diméthylformamide) et en présence de quatre équivalents d'imidazole pendant 20 h à 50°C. La solution est refroidie à 25°C. On ajoute alors dix équivalents d'acide acétique, d'imidazole et de BOP. La réaction d'acylation s'effectue en une demi-heure.

Le glycopeptide est isolé par chromatographie de tamisage moléculaire (colonne d'Ultrogel GF05, par exemple), dans l'acide acétique 0,1M. La fraction contenant le glycopeptide purifié est congelée et lyophilisée.

Le glycopeptide dissous dans un tampon acétate de sodium 0,1M, pH 6 est réduit par addition d'un équivalent de TCEP (tris-carboxyéthylphosphine) à 25°C pendant 30 min (voir K. Arar et al.; 1993, Tetrahedron Letters 34, 8087-8090, J. A. Burns et al., 1991, J. Org. Chem., 56, 2648-2650). On ajoute alors un équivalent d'un oligonucléotide substitué sur son extrémité 5' par un substituant terminé par un groupement dithio-2-pyridine.

Le conjugué glycopeptide-oligonucléotide formé a la structure générale suivante:

oligosidyl-NH-CH-CO-X
| |
$CH_3$-CO $CH_2$-S-S-5'-Oligonucléotide

dans lequel X représente NH-p-$C_6$-$H_4$-$NO_2$, et 5' représente le bras réunissant le premier S du dithio-2-pyridine à l'hydroxyle primaire du premier nucléotide de l'oligonucléotide.

Exemple 4

Préparation du lactosyl-pyroglutamyl-*para*nitro-anilide (dans le diméthylsulfoxyde).

Le lactose Galβ4Glc (0,15 mmole) est dissous dans 1,25 ml de diméthylsulfoxyde ($CH_3$-SO-$CH_3$). On ajoute 0,30 mmole de Glu-pNA dans 1,25 ml de diméthylsulfoxyde contenant 0,6 mmole d'imidazole (pNA = para nitro-anilide). La solution est gardée à 50°C pendant 20 h, puis refroidie à 25°C. Plus de 95% du lactose est transformé en glycopeptide: lactosyl-glu-pNA.

On ajoute 0,33 mmole de BOP et 0,6 mmole d'imidazole. La solution est agitée pendant 30 min à 25°C. Plus de 95% du glycopeptide est cyclisé en lactosyl-pyroglutamyl-paranitro-anilide:

Galβ4Glcβ*p*Glu-*p*NA

*p*Glu est: le résidu pyroglutamyle

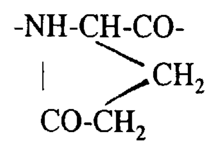

Exemple 5

Préparation du lactosyl-pyroglutamyl-*p*-nitroanilide (dans la N-méthylpyrrolidone).

Le lactose Galβ4Glc (0,15 mmole) est dissous dans 1,25 ml de N-méthylpyrrolidone de formule:

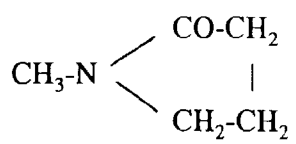

On ajoute 0,3 mmole de Glu-pNA dans 1,25 mmole de N-méthylpyrrolidone contenant 0,6 mmole d'imidazole. La solution est gardée à 50°C pendant 20 h puis refroidie à 25°C. Plus de 95% du lactose est transformé en glycopeptide: lactosyl-Glu-pNA. On ajoute 0,33 mmole de BOP et 0,6 mmole d'imidazole. La solution est agitée pendant 30 min à 25°C. Plus de 95% du glycopeptide est cyclisé en lactosyl-pyroglutamyl-p-nitro-anilide.

Les analyses sont effectuées par chromatographie sur colonne à haute pression sur un appareil Dionex, équipé d'un détecteur ampérométrique. Les rendements sont calculés par rapport à un témoin interne (le sorbitol) ajouté à la solution initiale de lactose.

Les temps de rétention des composés dans les conditions standard, exprimés en minutes, sont:

| | |
|---|---|
| Lactose | 9,9 ± 0,1 |
| Lactosyl-Glu-*p*NA | 21,4 ± 0,1 |
| Lactosyl-*p*-Glu-p-NA | 16,2 ± 0,1 |
| Imidazole | 4,3 ± 0,1 |
| Sorbitol | 2,7 ± 0,1 |

Exemple 6

Préparation d'un composé de formule:

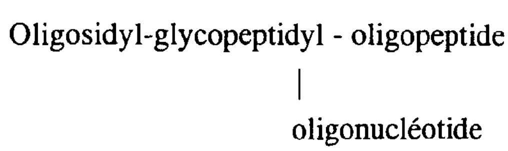

La préparation peut se faire selon le schéma réactionnel suivant.

Le produit de départ indiqué ci-après, peut être obtenu comme indiqué précédemment, à propos de la préparation des produits de l'invention.

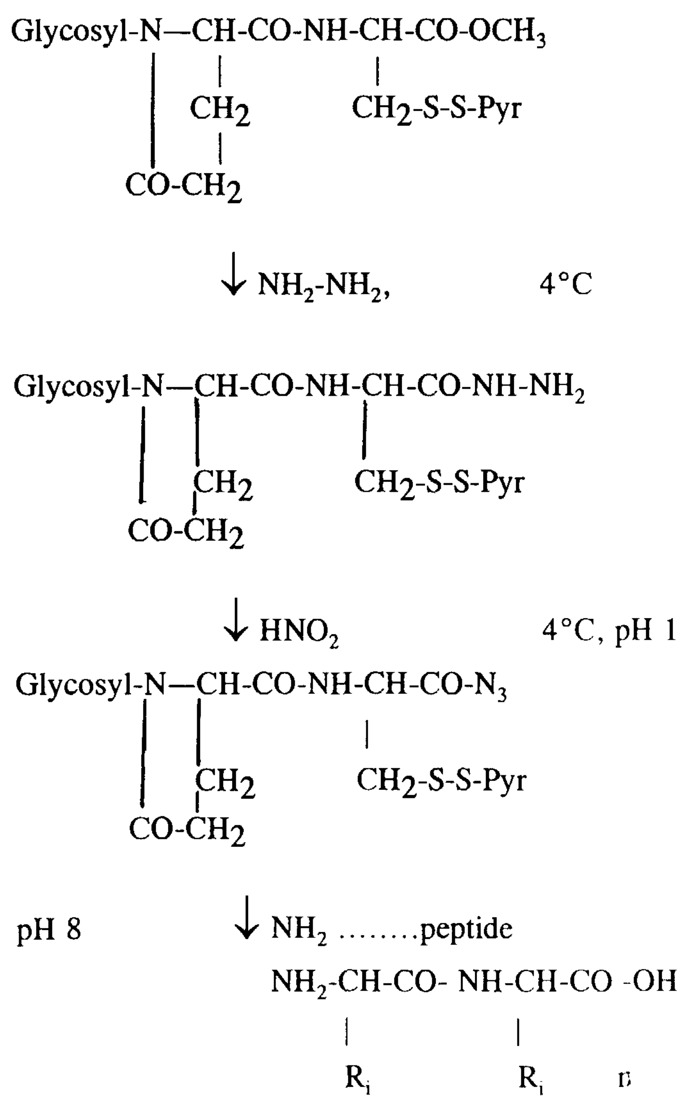


n représente un nombre entier égal ou supérieur à 0

$R_i$: chaîne latérale d'aminoacides

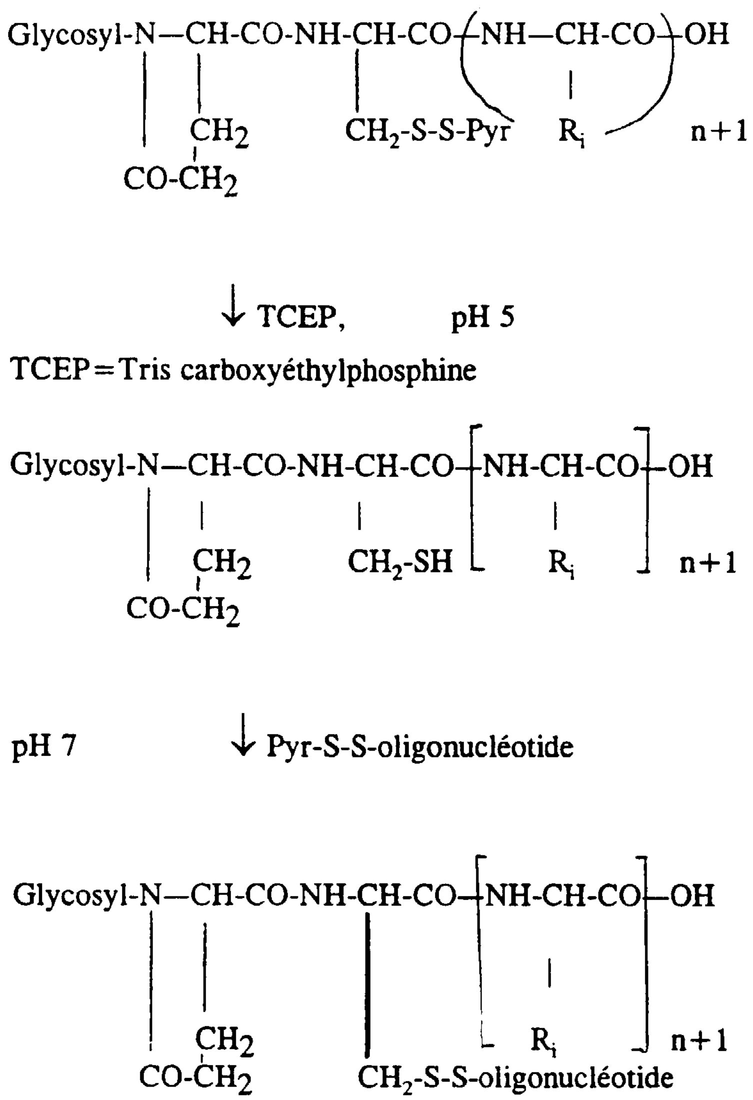


L'exemple choisi correspond à la préparation d'un dérivé d'oligonucléotide, tel que ce dérivé possède une activité biologique en relation avec la séquence de l'oligonucléotide choisi (l'oligonucléotide sens, anti-sens, antigène, leurre etc.., est spécifique d'un élément cellulaire ou viral de nature acide nucléique ou protéine), l'oligoside permet au dérivé d'être sélectivement reconnu par certaines cellules qui possèdent un récepteur membranaire (lectine) ayant une affinité pour l'oligoside choisi, le peptide permet au dérivé - une fois à l'intérieur des endosomes (vésicules intracellulaires), grâce au mécanisme d'endocytose dû à la lectine membranaire - de pénétrer dans les compartiments cytosolique, et ensuite nucléaire.

L'oligonucléotide est un oligomère comportant entre 10 et 40 nucléotides, de préférence 20 à 25. L'oligopeptide est un oligomère comportant entre 20 et 40 amino-acides, de préférence 20 à 25. Ce genre de dérivés correspond à une ligne de composés susceptibles d'être utilisés comme médicaments.

Exemple 7

Préparation d'un composé de formule:

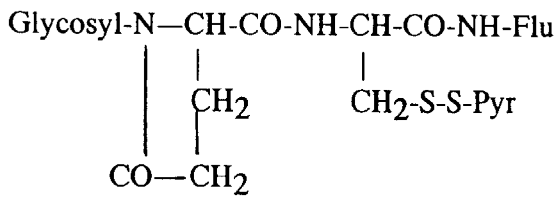

La préparation de ce composé peut se faire selon le schéma réactionnel suivant:

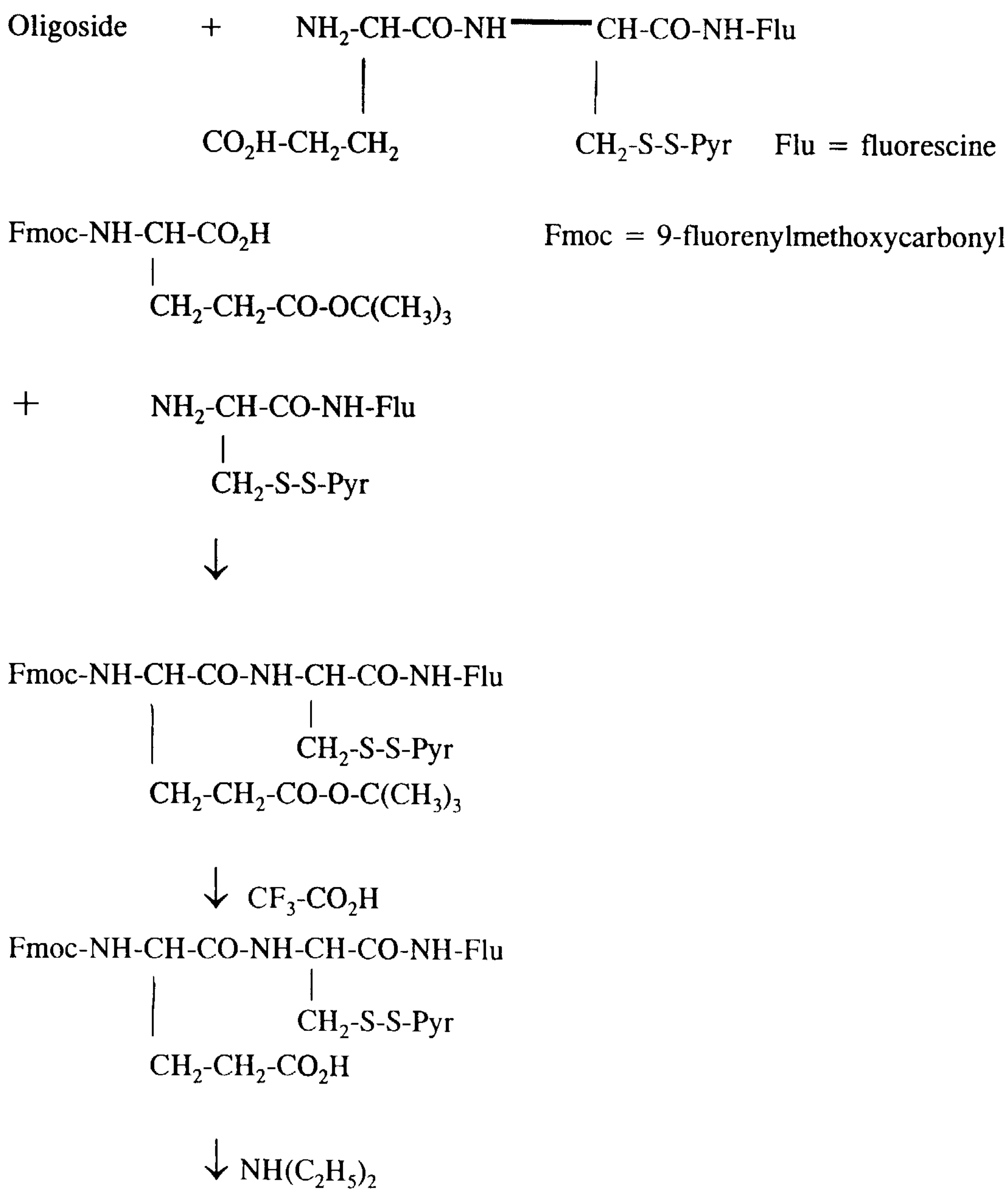
Flu = fluorescine
Fmoc = 9-fluorenylmethoxycarbonyl
$CF_3$-$CO_2H$
$NH(C_2H_5)_2$

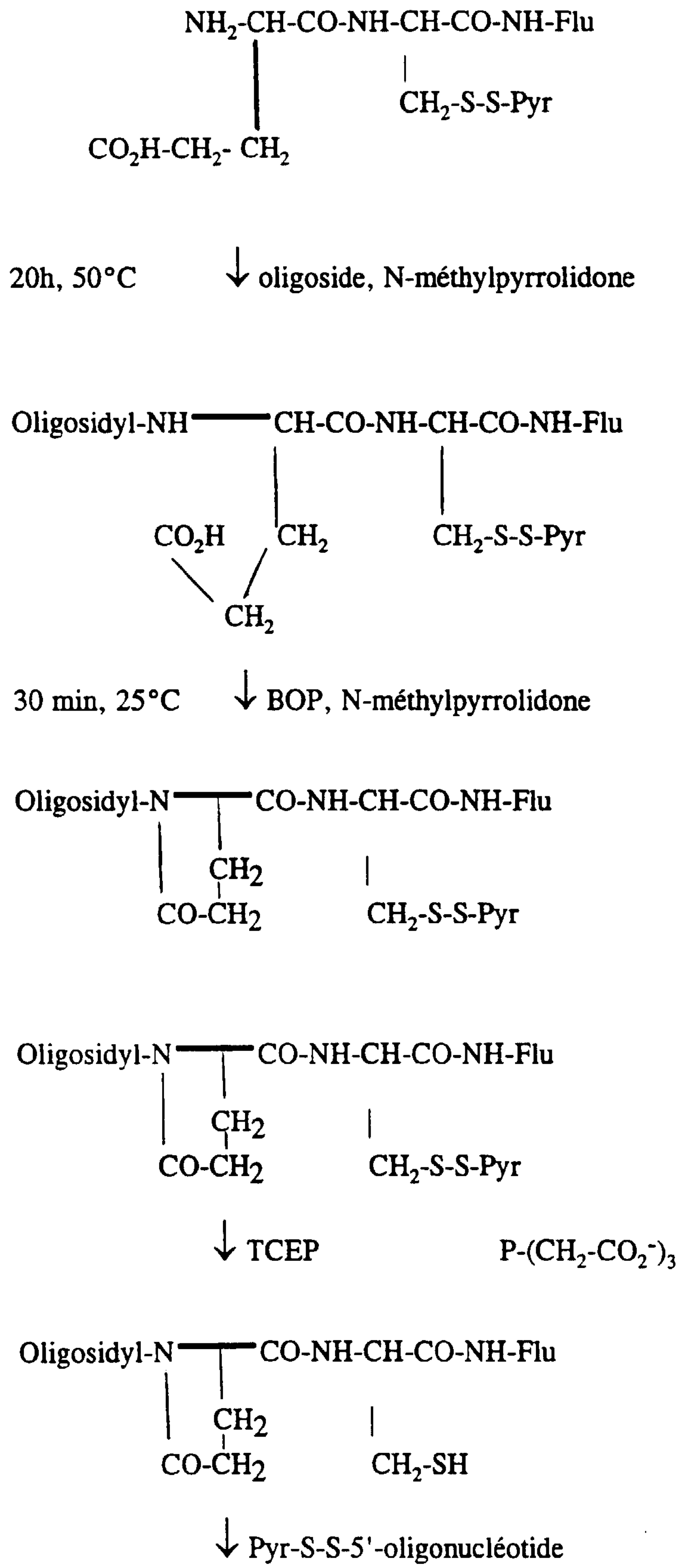
20h, 50°C
oligoside, N-méthylpyrrolidone
30 min, 25°C
BOP, N-méthylpyrrolidone
TCEP
P-(CH2-CO2-)3
Pyr-S-S-5'-oligonucléotide

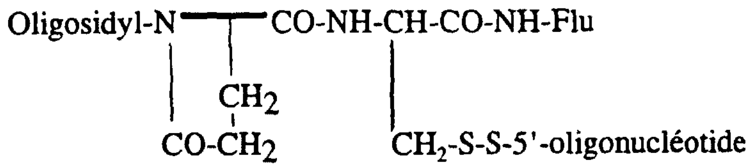

L'exemple choisi correspond à la préparation d'un dérivé glycopeptidique fluorescent d'utilisation générale.
Le résidu de fluorescéine permet une utilisation des glycopeptides à des fins de localisation, de visualisation, de façon générale, à des fins analytiques, en particulier en microscopie de fluorescence.
Le dérivé glycopeptidique fluorescent lié à un oligonucléotide peut également être utilisé comme agent antiviral ou anticancéreux, pour permettre à la fois une étude de l'activité biologique du dérivé et de son trafic intracellulaire, ainsi que de la pharmacocinétique, chez l'animal.
Dans cet exemple, le pont disulfure est présent dans le composé d'origine sur un résidu Ai de la formule générale.

Exemple 8

Préparation d'un composé de formule:

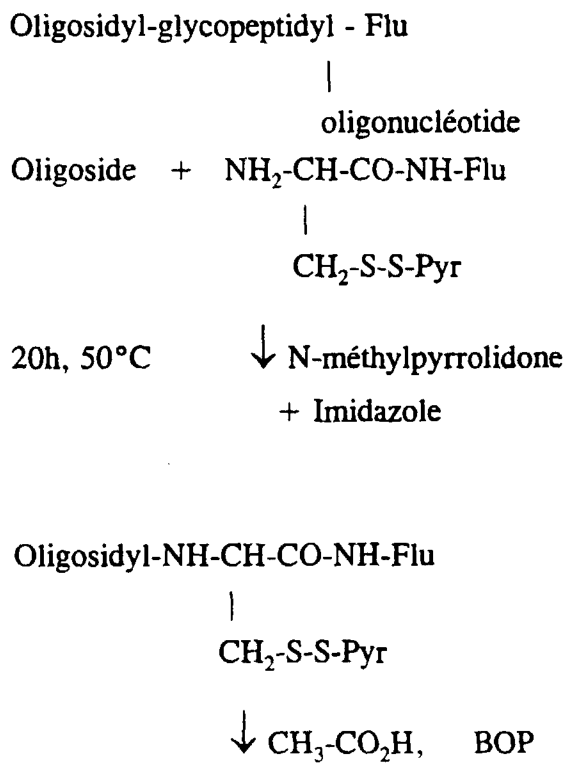

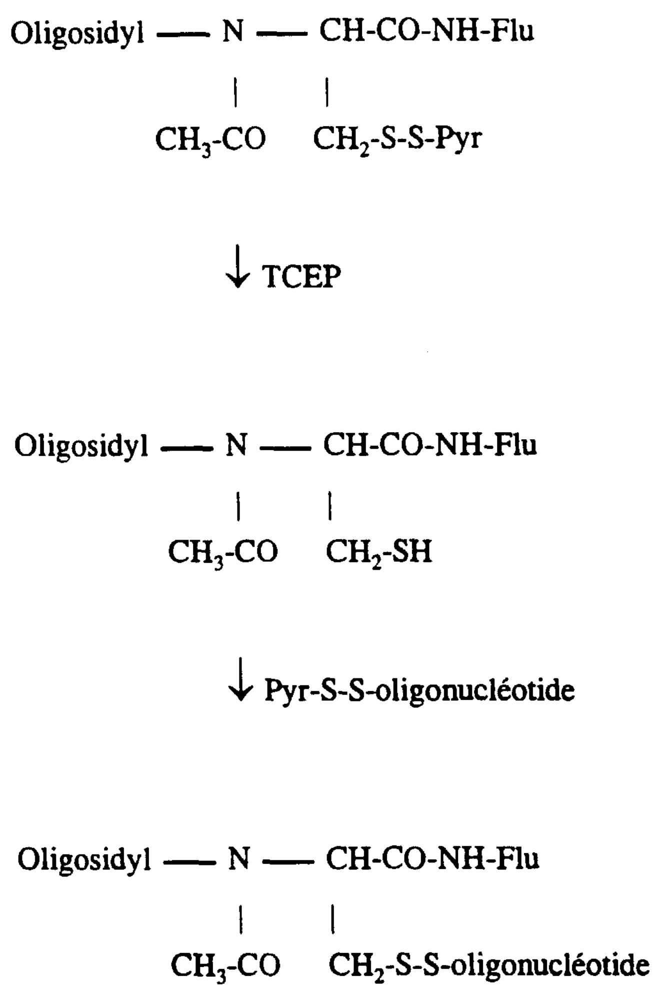


Ce qui a été dit à propos du dérivé glycopeptidique de l'exemple 7, s'applique à cet exemple. Il faut noter que dans l'exemple 8 ici considéré, le pont disulfure est présent dans le composé d'origine sur la chaîne Z de la formule générale.

<u>Exemple 9</u>

Préparation de dérivés glycosylés de la polylysine gluconoylée.

La polylysine gluconoylée est utilisée pour transférer des gènes dans les cellules animales. La substitution de la polylysine gluconoylée par un ou des glycopeptides permet de rendre le transfert de gènes sélectifs.

Les dérivés glycosylés de la polylysine gluconoylée pénètrent de préférence (100 à 1000 fois) dans des cellules qui expriment à leur surface une lectine (récepteur d'oligosides), qui reconnaît spécifiquement l'oligoside du glycopeptide lié à la polylysine gluconoylée.

**a**) Liaison d'un glycopeptide à la polylysine gluconoylée via un pont disulfure.

La polylysine gluconoylée (degré de polymérisation 190; contenant 60 résidus gluconoyle), est substituée par un dérivé de la dithiopyridine; le polymère (20 mg; 0,33 µmol) est dissous dans 0,5 ml de diméthylsulfoxyde.

On ajoute 1 µmol (312 µg) de N-succinimidyl 3-(2-pyridyldithio)propionate et 20 µmol (3,6 µl) de diisopropyléthylamine. La solution est agitée à 20°C pendant 15 h. Le polymère est précipité par addition de 10 volumes d'isopropanol; le précipité est récupéré après centrifugation (1 800 g, 15 min).

Après lavage par de l'isopropanol, le polymère est dissous dans un tampon phosphate de sodium 0,1 M de pH 7,2 (1 ml).

Le glycopeptide: oligosylpyroglutamyl amido éthyldithiopyridine:

Galβ4Glcβ-pyroglutamyl-NH-$(CH_2)_2$-S-S-pyridine (1 µmol) est traité par 1 µmole de TCEP (triscarboxyéthylphosphine: P $(CH_2\text{-}CH_2\text{-}CO_2^-)_3$) dans un tampon phosphate de sodium 0,1 M (1 ml), pendant 1 h à 20°C. Cette

solution est ajoutée à la solution de polylysine gluconoylée substituée par le pyridyldithiopropionate. Après 1 h à 20°C, le polymère est précipité par addition de 10 volumes d'isopropanol. Le précipité est récupéré après centrifugation (1 800 g, 15 min) et lavé dans l'isopropanol puis dissous dans l'eau et lyophilisé.

Le rendement de la réaction de couplage dans les conditions utilisées est égal ou supérieur à 90%.

Les réactions utilisées dans cette préparation sont dérivées de celles décrites dans Midoux, P., Mendes, C., Legrand, A., Rammond, J., Mayer, R., Monsigny, M. et Roche, A.C., 1993: Specific gene transfer mediated by lactosylated poly-1-lysine into hepatoma cells. Nucleic Acid Research, 21: 871-878, et dans Arar, K., Monsigny, M., et Mayer, R., 1993: Synthetis of oligonucleotide peptide conjugates containing a KDEL signal sequence. Tetrahedron Letters, 34: 8087-8090.

**b**) Liaison d'un glycopeptide à la polylysine gluconoylée via une liaison thiourée.

La polylysine gluconoylée (degré de polymérisation 190; contenant 60 résidus de gluconoyle), est substituée par un glycopeptide activé sous forme de phénylisothiocyanate.

Le glycopeptide oligosylpyroglutamyl *p*-nitroanilide est réduit en un dérivé p-aminoanilide qui est ensuite activé en un dérivé *p*-cyanato-anilide:

Galβ4Glcβ-pyroglutamyl-NH-p-$C_6H_4$-NCS selon un protocole adapté de celui décrit dans Roche, A.C., Barzilay, M., Midoux, P., Junqua, S., Sharon, N. et Monsigny, M. (1983): Sugar specific endocytosis of glycoproteins by Lewis lung carcinoma cells., J. Cell. Biochem., 22: 131-140.

Le dérivé cyanato-anilide (1 µmole) est dissous dans le diméthylsulfoxyde (1 ml) contenant 1 µmole de polylysine gluconoylée et 4 µmoles de diisopropyl éthylamine. La solution est agitée à 20°C pendant 24 h. Le polymère glycosylé est précipité par addition de 10 volumes d'isopropanol; le précipité est récupéré après centrifugation, lavé à l'isopropanol, et finalement dissous dans l'eau et lyophilisé.

Dans les conditions décrites, le rendement du couplage du glycopeptide sur la polylysine gluconoylée est supérieur à 95%

Exemple 10

Préparation d'un glycopeptide (l'oligosylpyroglutamyl-*p*-nitroanilide) dans le diméthylformamide comme solvant.

L'α glutamyl-*p*-nitroanilide (0,2 mmoles) et le lactose (0,1 mmole) sont dissous dans 1 ml de diméthylformamide, en présence de 0,2 mmoles d'imidazole. La solution est maintenue à 50°C pendant 8 h.

On refroidit à 25°C, et on ajoute 0,2 mmole de BOP et 0,2 mmole d'imidazole et on attend 30 min. Dans ces conditions, plus de 95% de l'oside de départ est transformé en dérivé glycopeptidique. La purification est identique à celle décrite en utilisant les autres solvants.

**Description des figures:**

La figure 1 représente le profil d'élution du β-lactosyl-pyroGlu-pNA obtenu par chromatographie d'échange d'anions à haute performance.

La figure 2 représente le profil d'élution du $Lewis^A/Lewis^X$-pyroGlu-pNA obtenu par chromatographie d'échange d'anions à haute performance.

Exemple 11

La pureté du produit préparé à l'exemple 2 (N-lactosylβ-pyroglu-pNA, que l'on désignera ci-après également par β-lactosyl-pyroGlu-pNA) a été vérifiée par chromatographie d'échange d'anions à haute performance et à détection ampérométrique (HPAE-PAD) sur un appareil de marque DIONEX.

S'agissant de cette technique, on procède comme suit:

Les osides sont ionisés en milieu alcalin (soude 0,1 M) sous forme de plurialcoolates. Leur séparation sur une résine cationique (ions ammoniums immobilisés) est très efficace. La détection des osides est avantageusement effectuée par une mesure ampérométrique en courant pulsé. L'appareil utilisé (Dionex) a été spécialement conçu pour réaliser la chromatographie en milieu alcalin et la détection ampérométrique en ligne.

Les temps de rétention ($t_r$) des différents produits ont été caractérisés (voir figure 1):

| Pic | $t_r$ (min) | Composé |
|---|---|---|
| 1 | 4,4 | Imidazole |
| 2 | 10,0 | Lactose |

(suite)

| Pic | $t_r$ (min) | Composé |
|---|---|---|
| 3 | 27,5 | β-lactosyl-Glu-pNA |
| 4 | 22,9 | β-lactosyl-pyroGlu-pNA |

Sur la figure 1:

- la première courbe (à partir du haut de la planche) correspond à l'injection de lactose seul,
- la deuxième courbe correspond à l'injection du mélange réactionnel après 12h,
- la troisième courbe correspond à l'injection du mélange réactionnel après 12 heures + 30 minutes de cyclisation.

Exemple 12

**Préparation d'un dérivé de glycosylamine acylé intramoléculairement: le $Lewis^A$/$Lewis^X$-pyroGlu-pNA.**

Le $Lewis^A$/$Lewis^X$ (0,06 mmole) est dissout dans 1 ml de diméthylformamide. On ajoute 0,12 mmole de Glu-pNA puis 0,24 mmole d'imidazole. La solution est gardée à 50°C pendant 15 h. La stabilisation du glycopeptide est obtenue en ajoutant, au milieu réactionnel ramené à 20°C, 0,13 mmole de BOP et 0.24 mmole d'imidazole. Après 30 min, le glycopeptide est cyclisé en $Lewis^A$/$Lewis^X$-pyroGlu-pNA. La réaction est suivie par HPAE-PAD.
**Synthèse de $Lewis^A$/$Lewis^X$-pyroGlu-pNA.** Profils d'élution Dionex. Les temps de rétention ($t_r$) des différents produits ont été caractérisés (voir figure 2):

| Pic | $t_r$ (min) | Composé |
|---|---|---|
| 1 | 4,5 | Imidazole |
| 2 | 8,9/9,4 | $Lewis^A$/$Lewis^X$ |
| 3 | 22,5/22,7 | $Lewis^A$/$Lewis^X$-Glu-pNA |
| 4 | 17,4/17,6 | $Lewis^A$/$Lewis^X$-pyroGlu-pNA |

Sur la figure 2:

- la première courbe (à partir du haut de la planche) correspond à l'injection du mélange réactionnel après 15 minutes,
- la deuxième courbe correspond à l'injection du mélange réactionnel après 15 heures, et
- la troisième courbe correspond à l'injection du mélange réactionnel après 15 heures + 30 minutes de cyclisation.

**Purification du glycopeptide.**

Après la synthèse décrite ci-dessus, on procède à la purification en deux étapes:

- par tamisage moléculaire dans une colonne de Trisacryl GF05 (100 cm x 2,3 cm) avec un débit de 10 ml/h, éluée par une solution aqueuse contenant 0.1 M d'acide acétique et 3% de n-butanol; cette première étape permet d'éliminer les oligosaccharides n'ayant pas réagi ainsi que les excès de BOP = hexafluorophosphate de benzotriazolyl-oxy-tris(diméthylamino)phosphonium, et de Glu pNA.
- par précipitation éthanolique (90%), au cours de laquelle l'échantillon est maintenu à 4°C pendant 24 h; cette deuxième étape permet d'éliminer l'excès d' imidazole.

La purification est suivie par HPAE-PAD.
Sur la figure 2, la quatrième courbe correspond à l'injection du produit $Lewis^A$/$Lewis^X$-pyroGlu-pNA purifié par tamisage moléculaire suivi d'une précipitation éthanolique ($t_r$: 17,4/17,6).

**Caractérisation du glycopeptide ($Lewis^A$/$Lewis^X$pyroGlu-pNA).**

Une analyse en $^1H$ RMN à 300 MHz a été réalisée.
Le $Lewis^A$/$Lewis^X$-pyroGlu-pNA est dissout dans $D_2O$ ($6.10^{-3}$ mole/l). $Lewis^A$ possède un galactose terminal lié en 3 et un fucose terminal lié en 4 sur la N-acétylglucosamine. $Lewis^X$ possède un galactose terminal lié en 4 et un

fucose terminal lié en 3 sur la N-acétylglucosamine. L'examen du spectre a permis d'identifier un certain nombre de protons caractéristiques:

- communs aux 2 glycopeptides: 8,33 et 7,79 (4H, 2d, H aromatique); 4,90 (2H, m, H5 αFuc); 4,67 (1H, d, $J_{1,2}$, 7,32 Hz, H1 βGlcNAc); 4,37 (1H, d, $J_{1,2}$ 7,42 Hz, H1 $βGal^{int}$); 4,16 (1H, s, H4 $βGal^{int}$); 2,83 (2H, m, $γCH_2$ pyroGlu); 2,33 (2H, m, β et $β'CH_2$ pyroGlu); 2,05 et 2,04 (6H, 2s, $CH_3$ Ac GlcNAc); 1,22 et 1,21 (6H, 2s, $CH_3$ Fuc);
- spécifiques de $Lewis^A$: 5,05 (1H, d, H1 αFuc); 4,53 (1H, d, H1 βGal);
- spécifiques de $Lewis^X$: 5,24 (1H, d, $J_{1,2}$, 7,2 Hz, H1 αFuc); 4,50 (1H, d, H1 βGal).

Exemple 13

On a préparé comme indiqué à l'exemple 12, le $Lewis^B$-pyroGlu-pNA et l'oligoH-pyroGlu-pNA.
On a récapitulé ci-après, l'analyse des glycopeptides obtenus, y compris celle du β-lactosyl-pyroGlu-pNA (N-lactosylβ-pyroGlu-pNA) et du $Lewis^A$/$Lewis^X$-pyroGlu-pNA.

**Analyse des glycopeptides (appareil Dionex).**

Séparation par chromatographie échangeuse d'anions. Colonne CarboPac PA1 (4 x 250 mm). Débit: 1 ml/min. Détection par ampérométrie pulsée. Travail à température ambiante.
On a recours pour bien séparer à un gradient acétate de sodium:

| Temps (min) | NaOH 100 mM | NaOH 100 mM $CH_3COONa$ 1M |
|---|---|---|
| 0 | 100 | 0 |
| Injection 0,1 | 100 | 0 |
| 5 | 100 | 0 |
| 15 | 80 | 20 |
| 30 | 0 | 100 |
| 35 | 0 | 100 |
| 40 | 100 | 0 |

Temps de rétention exprimés en minutes:

| | |
|---|---|
| Imidazole | 4,4 ± 0,1 |
| Lactose | 10,0 ± 0,1 |
| β-lactosyl-Glu-pNA | 27,5 ± 0,1 |
| β-lactosyl-pyroGlu-pNA | 22,9 ± 0,1 |
| | |
| Imidazole | 4,5 ± 0,1 |
| $Lewis^A$/$Lewis^X$ | 8,9-9,4 ± 0,1 |
| $Lewis^A$/$Lewis^X$-Glu-pNA | 22,5/22,7 ± 0,1 |
| $Lewis^A$/$Lewis^X$-pyroGlu-pNA | 17,4/17,6 ± 0,1 |
| | |
| Imidazole | 4,4 ± 0,1 |
| $Lewis^B$ | 7,3 ± 0,1 |
| $Lewis^B$-Glu-pNA | 19,7 ± 0,1 |
| $Lewis^B$-pyroGlu-pNA | 16,6 ± 0,1 |
| Imidazole | 4,5 ± 0,1 |
| OligoH | 8,2/8,7 ± 0,1 |
| OligoH-Glu-pNA | 23,8 ± 0,1 |
| OligoH-pyroGlu-pNA | 18,9 ± 0,1 |

**Revendications**

1. Composés comprenant un ou plusieurs oligosides, chacun desdits oligosides étant fixé de façon covalente sur

une ou plusieurs molécules, matrices ou particules, lesquelles représentent une matrice comme support pour la chromatographie d'affinité ; une bille d'or, de latex, pour l'histologie et la cytologie ; une protéine, un lipide, des oligonucléotides ou encore des polymères pour le ciblage de médicaments, d'oligonucléotides ou de gènes, en particulier une, deux ou trois, grâce à une molécule intermédiaire possédant un atome d'azote porté par un carbone en a d'un groupe C = O, l'atome d'azote étant N-acylé, et un ou plusieurs groupes fonctionnels, en particulier un, deux ou trois, la liaison covalente entre ladite molécule intermédiaire et l'oligoside ayant lieu par l'intermédiaire du susdit atome d'azote, et la liaison covalente entre ladite molécule intermédiaire et la susdite molécule, la susdite matrice, la susdite particule, ou les susdites molécules, les susdites matrices, les susdites particules ayant lieu par l'intermédiaire du ou des susdits groupes fonctionnels de ladite molécule intermédiaire et des groupes fonctionnels appropriés sur la (les) molécule(s), la (les) matrice(s) ou la (les) particule(s).

**2.** Composés de formule générale (I)

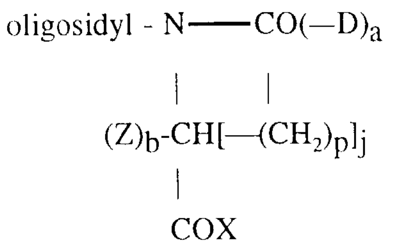

(I)

dans laquelle:

* a = 0 ou 1,
* j = 0 ou 1,
* b = 0 ou 1,
* p = 2 à 4, notamment 2,
* sous réserve que
  ** a = b = 0, lorsque j = 1, ce qui entraîne la présence d'une molécule cyclique,
  ** ou a = b = 1, lorsque j = 0 ce qui implique l'absence de groupe $(CH_2)_p$,
* D représente un résidu d'un acide organique de formule $DCO_2H$, notamment H ou une chaîne alkyle de 1 à 10 atomes de carbone, en particulier $CH_3$,
* Z représente
  ** B, B étant H, un alkyle de 1 à 10 atomes de carbone, ou une chaîne latérale d'un acide α aminé, naturel ou synthétique tel que $CH(CH_3)_2$, $CH_2OH$, $CH_3$, et de préférence H, ou
  ** B'-P', B' étant une chaîne alkylidène de 1 à 10 atomes de carbone, ou une chaîne latérale d'un acide α aminé, naturel ou synthétique, lesdites chaînes contenant un groupe dérivé d'un groupement fonctionnel susceptible d'être activé, tel que carboxylique, SH, OH ou amine, libre ou protégé, de préférence protégé, P' ayant les significations indiquées ci-après,
* X représente:
  . le groupe

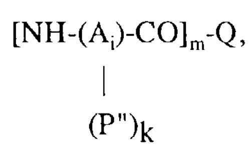

  . ou le groupe

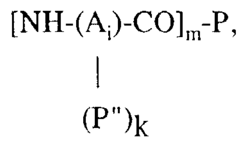

. ou le groupe

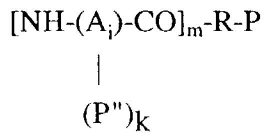

* m étant un nombre entier de 0 à 10, de préférence de 0 à 5 et avantageusement 1 ou 2, k = 0 ou 1
* Q représente OH, $OCH_3$, $OCH_2$-$C_6H_5$, O-$C_6H_5$, O-$C_6F_5$, O-p$C_6H_4$-$NO_2$,

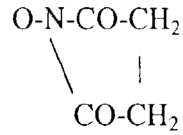

* R représentant un groupe possédant une fonction alcool, phénol, thiol ou amine,
* P étant tel que défini ci-après,
* $A_i$ représente un radical organique tel qu'une chaîne alkylidène de 1 à 10 atomes de carbone, notamment $(CH_2)_n$-W-$(CH_2)_{n'}$, n + n' représentant un nombre entier de 0 à 10, W représentant CHY, Y étant H, un alkyle de 1 à 6 atomes de carbone linéaire ou ramifié, un résidu d'amino $\alpha$ acide, naturel ou synthétique, ou W représentant un composé aromatique, notamment phényle,
* P, P' et P" sont identiques ou différents et représentent :

  - une matrice comme support pour la chromatographie d'affinité;
  - une bille d'or, de latex, pour l'histologie et la cytologie;
  - une protéine pour la visualisation, la purification, notamment 1) des récepteurs spécifiques des osides, récepteurs que l'on appelle lectines, adhésines, agglutinines etc, ou encore 2) des protéines à activité enzymatique ou non, qui ont une affinité pour les osides, notamment des glycosyltransférases, telles que la sialyltransférase, des sulfotransférases, des phosphotransférases, des exoglycosidases ou des endoglycosidases;
  - un lipide pour la caractérisation des récepteurs précédents;
  - des oligonucléotides pour augmenter sélectivement leur capture par des cellules cibles;
  - une protéine ou des polymères pour le ciblage de médicaments, d'oligonucléotides ou de gènes,

  P, P' et P" possédant au moins une fonction permettant une réaction de condensation par réaction avec un oligopeptide, par exemple

  . une fonction amine (-$NH_2$) permettant la formation d'amide avec un ester actif, d'amidine avec un imidate, de thiourée avec un isothiocyanate,
  . une fonction thiol (-SH) permettant la formation d'un pont disulfure avec un oligopeptide contenant un thiol, d'un thioether avec un oligopeptide contenant un groupement maléimide, ou halogéno-alkyle, ou un halogéno-alkanoyle,
  . un phénol (-$C_6H_4OH$) permettant la formation d'un azoïque avec un oligopeptide contenant un diazoïque,

sous réserve que Z représente B'-P', et/ou X comporte P et/ou P" dans sa formule.

**3.** Composés selon l'une des revendications 1 ou 2, de formule générale (II)

oligosidyl - N——CO(—D)$_a$

| |

(B)$_b$-CH[—(CH$_2$)$_p$]$_j$ (II)

|

COX

dans laquelle:

* a = 0 ou 1,
* j = 0 ou 1,
* b = 0 ou 1,
* p = 2 à 4, notamment 2,
* sous réserve que

  ** a = b = 0, lorsque j = 1, ce qui entraîne la présence d'une molécule cyclique,
  ** ou a = b = 1, lorsque j = 0 ce qui implique l'absence de groupe $(CH_2)_p$,

* D représente un résidu d'un acide organique de formule $DCO_2H$, notamment H ou une chaîne alkyle de 1 à 10 atomes de carbone, en particulier $CH_3$,
* B représente H, un alkyle de 1 à 10 atomes de carbone, ou une chaîne latérale d'un acide $\alpha$ aminé tel que $CH(CH_3)_2$, $CH_2OH$, $CH_3$, et de préférence H,
* X représente :

  . ou le groupe $[NH-(A_i)-CO]_m$-P,
  . ou le groupe $[NH-(A_i)-CO]_m$-R-P

  m étant un nombre entier de 0 à 10, de préférence de 0 à 5 et avantageusement 1 ou 2, R et P étant tels que définis ci-après,

  * $A_i$ représente un radical organique tel qu'une chaîne alkylidène de 1 à 10 atomes de carbone, notamment $(CH_2)_n$-W-$(CH_2)_{n'}$, n + n' représentant un nombre entier de 0 à 10, W représentant CHY, Y étant H, un alkyle de 1 à 6 atomes de carbone linéaire ou ramifié, un résidu d'amino $\alpha$ acide, naturel ou synthétique, ou W représentant un composé aromatique, notamment phényle,
  * R représentant un groupe possédant une fonction alcool, phénol, thiol ou amine,
  * P représente :

    - une matrice comme support pour la chromatographie d'affinité;
    - une bille d'or, de latex, pour l'histologie et la cytologie;
    - une protéine pour la visualisation, la purification, etc, notamment 1) des récepteurs spécifiques des osides, récepteurs que l'on appelle lectines, adhésines, agglutinines etc, ou encore 2) des protéines à activité enzymatique ou non, qui ont une affinité pour les osides, notamment des glycosyltransférases, telles que la sialyltransférase, des sulfotransférases, des phosphotransférases, des exoglycosidases ou des endoglycosidases;
    - un lipide pour la caractérisation des récepteurs précédents;
    - des oligonucléotides pour augmenter sélectivement leur capture par des cellules cibles;
    - une protéine ou des polymères pour le ciblage de médicaments, d'oligonucléotides ou de gènes.

**4.** Composés selon l'une des revendications 1 ou 2, de formule générale (III)

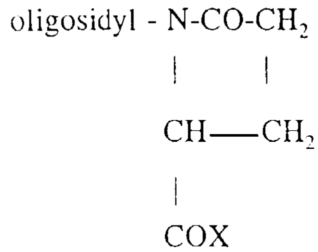

(III)

dans laquelle X représente $[NH-(A_i)-CO]_m-P$ ou $[NH-(A_i)-CO]_m-R-P$, $A_i$, m, P et R ayant les significations indiquées à la revendication 2.

**5.** Composés selon l'une des revendications 1 ou 2, de formule générale (IV)

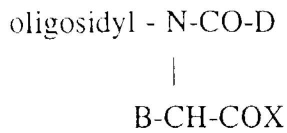

(IV)

dans laquelle D et B ont les significations indiquées à la revendication 2, et X représente $[NH-(A_i)-CO]_m-P$ ou $[NH-(A_i)-CO]_m-R-P$, $A_i$, m, R et P ayant les significations indiquées à la revendication 2.

**6.** Composés selon l'une des revendications 1 ou 2, de formule générale (V)

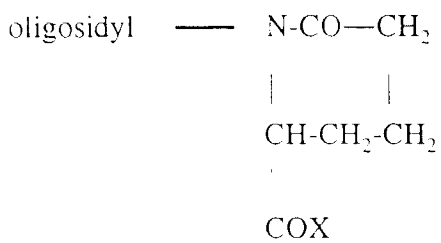

(V)

dans laquelle X représente $[NH-(A_i)-CO]_m-P$ ou $[NH-(A_i)-CO]_m-R-P$, P, $A_i$, m et R ayant les significations indiquées à la revendication 2.

**7.** Produits, susceptibles notamment de servir de produits intermédiaires pour la préparation d'un des composés selon l'une quelconque des revendications 1 à 6, lesdits produits comprenant un oligoside lié à une molécule possédant un atome d'azote, porté par un carbone en α d'un groupe C = O, et au moins un groupe fonctionnel, notamment un, deux ou trois groupes fonctionnels, la liaison covalente entre l'oligoside et la molécule s'effectuant par l'intermédiaire du susdit atome d'azote.

**8.** Produits de formule générale (Ia)

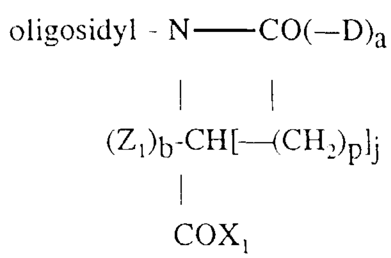

(Ia)

dans laquelle:

* a = 0 ou 1,
* j = 0 ou 1,
* h = 0 ou 1,
* p = 2 à 4, notamment 2,
* sous réserve que

  ** a = b = 0, lorsque j = 1, ce qui entraîne la présence d'une molécule cyclique,
  ** ou a = b = 1, lorsque j = 0 ce qui implique l'absence de groupe $(CH_2)_p$,

* D représente un résidu d'un acide organique de formule $DCO_2H$, notamment H ou une chaîne alkyle de 1 à 10 atomes de carbone, en particulier $CH_3$,
* $Z_1$ représente

  * B, B étant choisi parmi: H, une chaîne alkyle de 1 à 10 atomes de carbone, ou une chaîne latérale d'un acide $\alpha$ aminé tel que $CH(CH_3)_2$, $CH_2OH$, $CH_3$, et de préférence H, ou
  * B', B' étant choisi parmi: une chaîne alkylidène de 1 à 10 atomes de carbone, ou une chaîne latérale d'un acide $\alpha$ aminé, lesdites chaînes contenant un groupe fonctionnel tel que carboxylique, SH, OH ou amine, libre ou protégé,

* $X_1$ représente

  . le groupe $[NH-(A_i)-CO]_m-R$,
  . le groupe $[NH-(A_i)-CO)_m-Q$

* R représente un composé possédant une fonction alcool, phénol, thiol ou amine,
* Q représente OH, $OCH_3$, $OCH_2-C_6H_5$, $O-C_6H_5$, $O-C_6F_5$, $O-pC_6H_4-NO_2$,

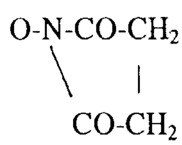

* m étant un nombre entier de 0 à 10, de préférence de 0 à 5 et avantageusement 1 ou 2,
* $A_i$ représente un radical organique tel qu'une chaîne alkylidène de 1 à 10 atomes de carbone, notamment $(CH_2)_n-W-(CH_2)_{n'}$, n + n' représentant un nombre entier de 0 à 10, W représentant CHY, Y étant H, un alkyle de 1 à 6 atomes de carbone linéaire ou ramifié, un résidu d'amino $\alpha$ acide, naturel ou synthétique, ou W représentant un composé aromatique, notamment phényle.

**9.** Produits selon l'une des revendications 7 ou 8, de formule générale (IIa)

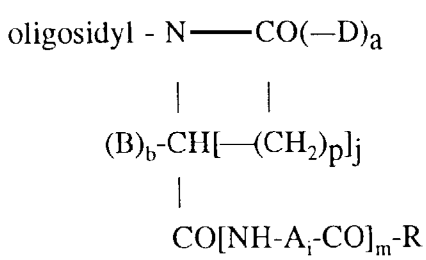

(IIa)

dans laquelle:

* a = 0 ou 1,
* j = 0 ou 1,

* b = 0 ou 1,
* p = 2 à 4, notamment 2,
* sous réserve que
    ** a = b = 0, lorsque j = 1, ce qui entraîne la présence d'une molécule cyclique,
    ** ou a = b = 1, lorsque j = 0 ce qui implique l'absence de groupe $(CH_2)_p$,
* D représente un résidu d'un acide organique de formule $DCO_2H$, notamment H ou une chaîne alkyle de 1 à 10 atomes de carbone, en particulier $CH_3$,
* B représente H, un alkyle de 1 à 10 atomes de carbone, ou une chaîne latérale d'un acide α aminé tel que $CH(CH_3)_2$, $CH_2OH$, $CH_3$, et de préférence H,
* m étant un nombre entier de 0 à 10, de préférence de 0 à 5 et avantageusement 1 ou 2,
* $A_i$ représente un radical organique tel qu'une chaîne alkylidène de 1 à 10 atomes de carbone, notamment $(CH_2)_n$-W-$(CH_2)_{n'}$, n + n' représentant un nombre entier de 0 à 10, W représentant CHY, Y étant H, un alkyle de 1 à 6 atomes de carbone linéaire ou ramifié, un résidu d'amino α acide, naturel ou synthétique, ou W représentant un composé aromatique, notamment phényle,
* R représente un composé possédant une fonction alcool, phénol, thiol ou amine.

**10.** Produits selon l'une des revendications 7 ou 8, de formule générale (IIIa)

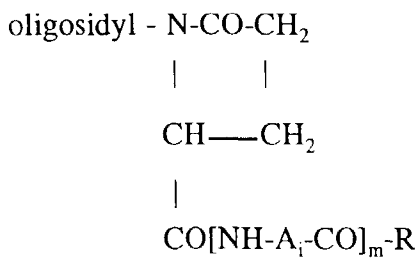

(IIIa)

dans laquelle $A_i$, m et R ont les significations indiquées à la revendication 8.

**11.** Produits selon l'une des revendications 7 ou 8, de formule générale (IVa)

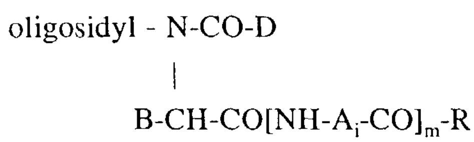

(IVa)

dans laquelle D, B, m, $A_i$ et R ont les significations indiquées à la revendication 8.

**12.** Produits selon l'une des revendications 7 ou 8, de formule générale (Va)

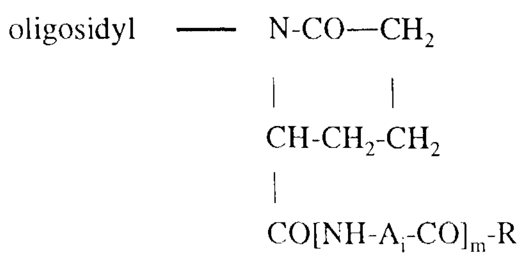

(Va)

dans laquelle $A_i$, m et R ont les significations indiquées à la revendication 8,

**13.** Produits de formule (VI)

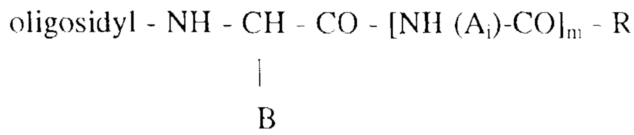

(VI)

dans lequel B, $A_i$, m et R ont les significations indiquées à la revendication 2.

**14.** Produits de formule (VII)

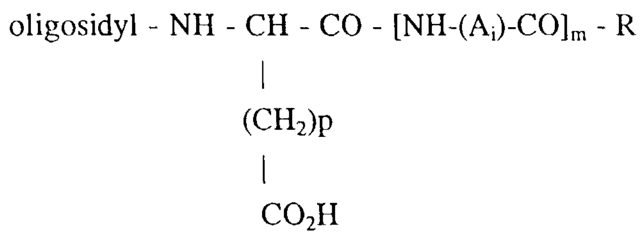

(VII)

dans lequel p, $A_i$, R, m ont les significations indiquées à la revendication 2.

**15.** Composés ou produits selon l'une des revendications 1 à 14, caractérisés en ce que Q représente les radicaux suivants:

$OH$, $O-CH_2-C_6H_5$, $O-C_6H_5$, $O-C_6F_5$, $O-pC_6H_4-NO_2$,

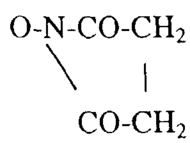

et caractérisés en ce que R représente l'un des radicaux suivants :

$-NH-pC_6H_4-N=C=S$ et ses précurseurs:

$$-NH-pC_6H_4-NO_2$$

$$-NH-pC_6H_4-NH_2$$

$$-NH-CH_2[-(CH_2)_m-C(=NH_2^+)OCH_3$$

$$-NH-CH_2-(CH_2)_m-CN$$

$$-NH-CH_2-(CH_2)_m-CH_2-NH-CO-CH_2-(CH_2)_m-C(=NH_2^+)OCH_3$$

$$-NH-CH_2-(CH_2)_m-CH_2-NH-CO-CH_2-(CH_2)_m-CN$$

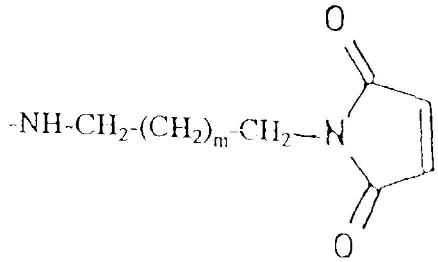

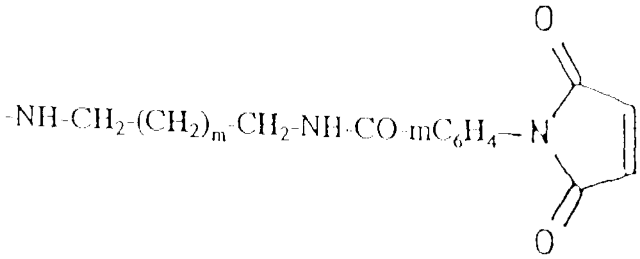

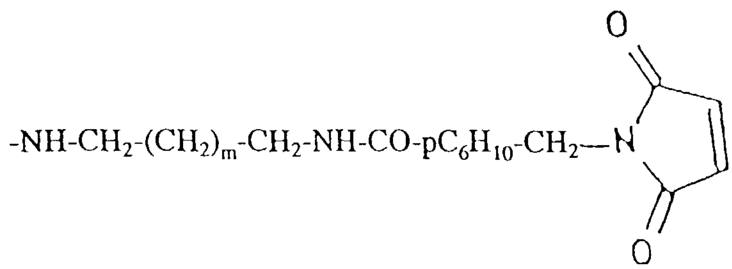

$-NH-CH_2-(CH_2)_m-CH_2-NH-CO-CH_2-T$     T = Br, I, Cl

$-NH-CH_2-CH_2-NH-CO-CH_2-(CH_2)_m-S-S-Pyr$

$-NH-CH_2-(CH_2)_m-CH_2-S-S-Pyr$

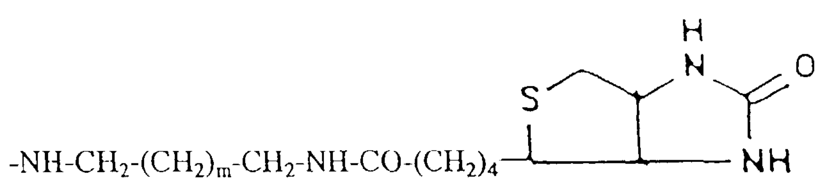

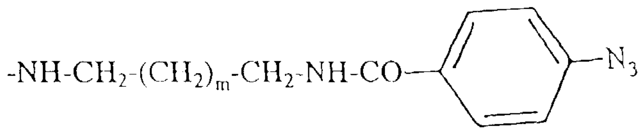

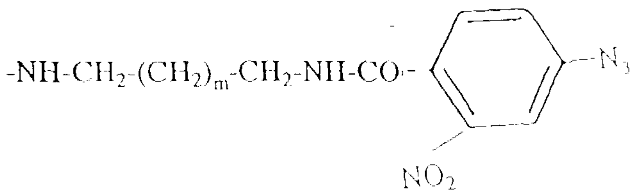

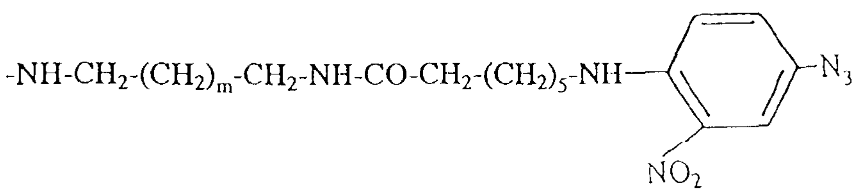

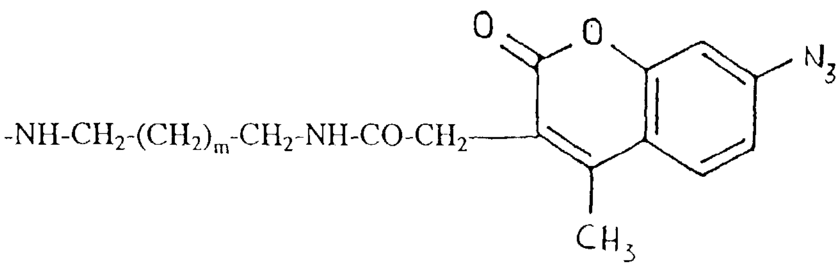

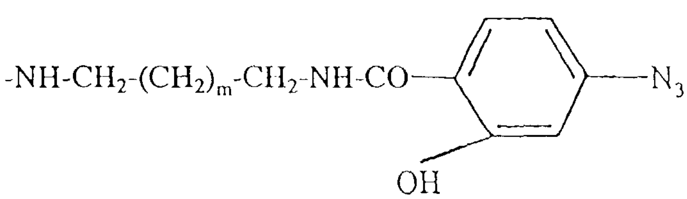

$-NH-(CH_2)_m-pC_6H_4OH$

$-NH-CH_2-(CH_2)_m-CH_2-NH-CO-(CH_2)_m-pC_6H_4OH$

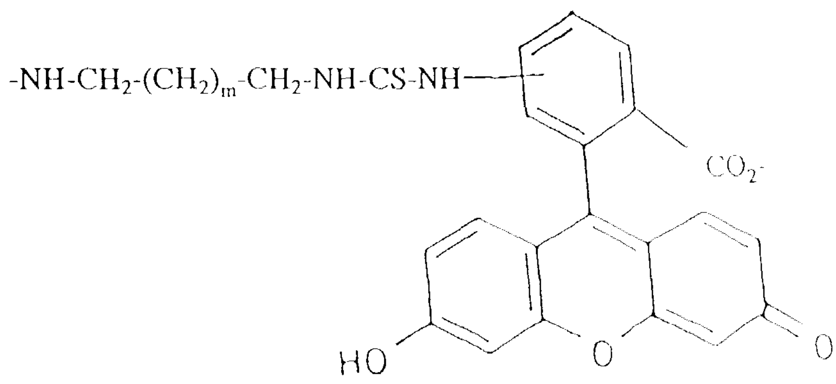

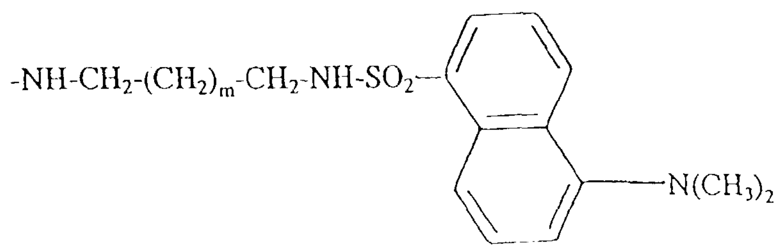

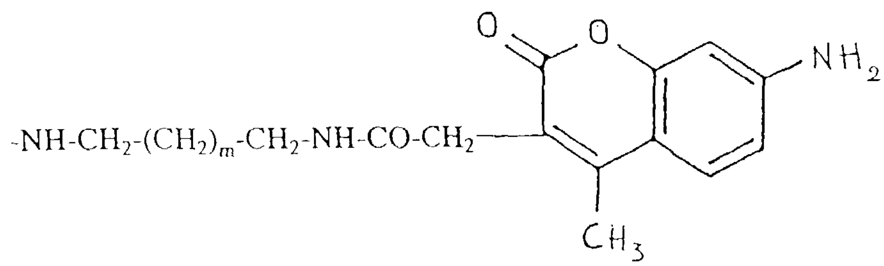

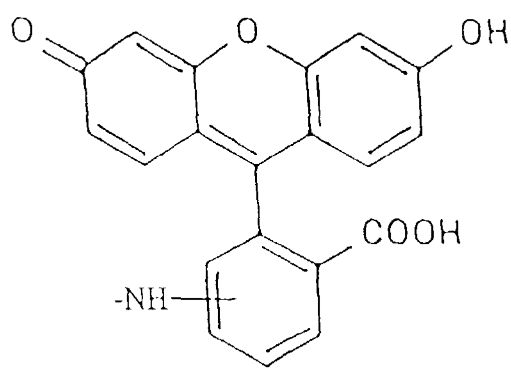

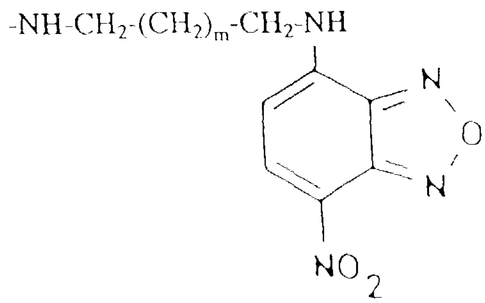

m représentant un nombre entier de 0 à 10, de préférence de 0 à 5, et avantageusement 1 ou 2, Pyr représentant le groupe 2-pyridine.

**16.** Composé selon l'une des revendications 1 ou 2, caractérisé en ce que P représente un oligopeptide, ou un polypeptide, notamment la polylysine gluconoylée, et P' ou P" représente un oligonucléotide, ou bien R représente la fluorescéine ou un de ses dérivés ou un autre dérivé fluorescent, et P' ou P" représente un oligonucléotide ou P représente un agent thérapeutique ou toute molécule d'intérêt.

**17.** Composés selon l'une quelconque des revendications 1 à 12, caractérisés en ce que le résidu oligosidique est un oligosaccharide comportant de 2 à 50 oses et notamment est choisi parmi:

| | |
|---|---|
| lacto-N-tétraose | Galβ 3 GlcNAcβ 3 Gal β 4 Glc |
| néolacto-N-tétraose | Galβ 4 GlcNAcβ 3 Gal β 4 Glc |
| groupe H | Fuc α 2 Galβ 3 Galβ 4 Glc |
| Lewis$^a$ | Galβ 3 GlcNAcβ 3 Gal β 4 Glc<br>Fuc α 4-↑ |
| Lewis$^x$ | Galβ 4 GlcNAcβ 3 Galβ 4 Glc<br>Fuc α 3-↑ |
| Lewis$^b$ | Fucα 2 Galβ 3 GlcNAc β 3 Galβ 4 Glc<br>Fuc α 4-↑ |
| Lewis$^y$ | Fuc α 2 Galβ 4 GlcNAcβ 3 Galβ 4 Glc<br>Fuc α 3-↑ |
| Disialolacto-N-tétraose | Neu 5Acα 3 Gal β 3 GlcNAc β 3 Gal β 4 Glc<br>Neu 5Acα 6-↑ |

Type complexe à 3 antennes

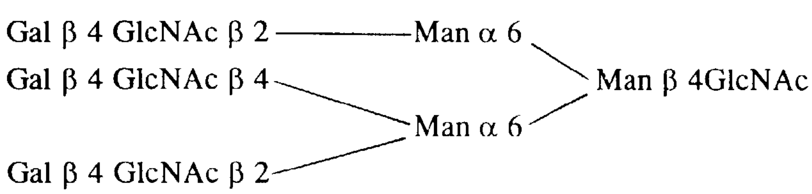

| | |
|---|---|
| Sialylactose 3 | Neu 5Ac α 3 Gal 4 Glc |
| Sialylactose 6 | Neu 5Ac α 6 Gal β 4 Glc |
| Disialylactose 3 | Neu 5Ac α 8 Neu 5Ac α 3 Gal β 4 Glc |

- des osides simples ou complexes reconnus par des lectines membranaires, et choisis parmi par exemple:

**a. Asialo-oligoside de type lactosamine triantenné : récepteur d'asialoglycoprotéine**

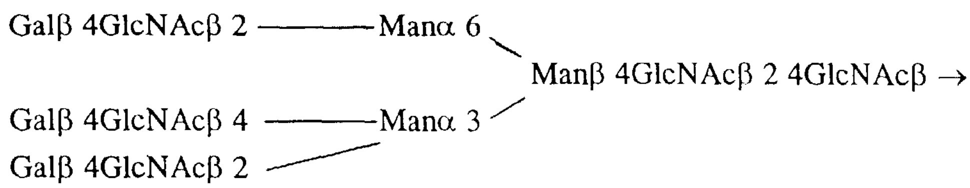

**b. Asialo oligoside de type lactosamine tétraantenné: récepteur d' asialoglycoprotéine**

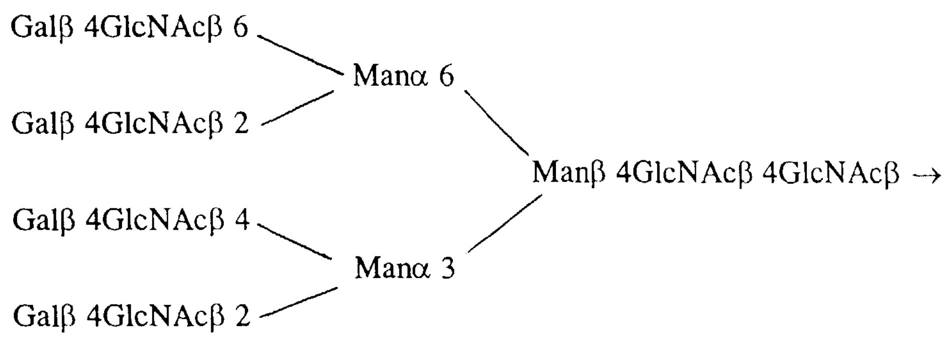

**c. Lewis x: LECAM 2/3**

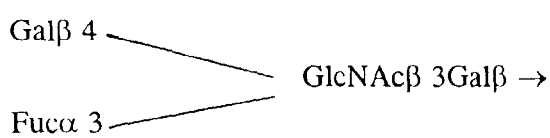

**d. Sialyl Lewis x : LECAM 3/2**

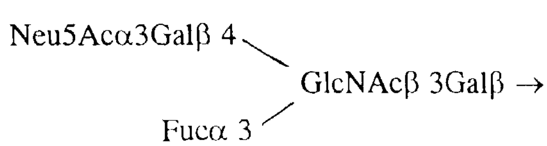

**e. Dérivé de Lewis x sulfaté (HNK1): LECAM 1**

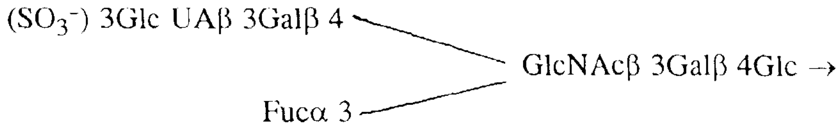

**f. Oligomannoside: récepteur du mannose**

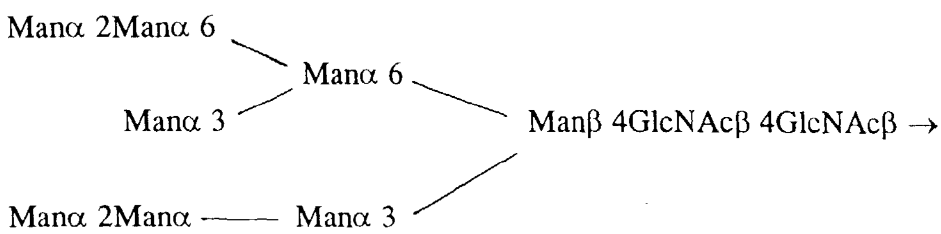

**g. Oligomannoside phosphorylé: récepteur de mannose 6 phosphate**

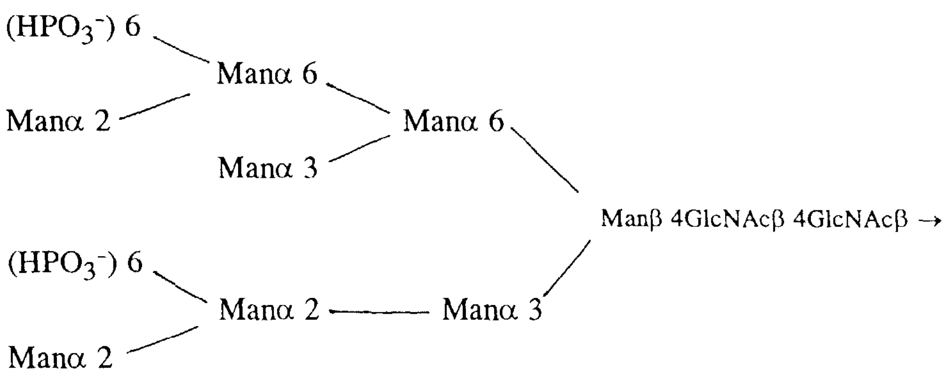

**h. Oligosaccharide de type lactosamine sulfaté: récepteur de GalNAc 4 sulfaté**

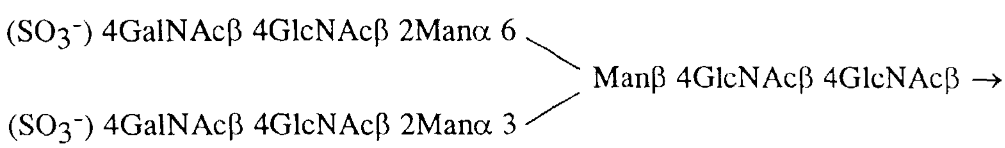

**18.** Procédé de préparation des composés selon l'une des revendications 7 à 12, caractérisé en ce que:

- l'on condense, dans un solvant approprié, un oligoside ayant un ose réducteur libre, sur l'atome d'azote d'une molécule intermédiaire, cet atome d'azote appartenant à un groupe amine, lié à un atome de carbone placé en a d'un groupe C = O, la molécule intermédiaire possédant éventuellement une chaîne latérale contenant un groupe fonctionnel tel que OH, SH, $NH_2$ ou COOH, libre ou protégé, cette molécule de départ étant choisie parmi les molécules intermédiaires suivantes: acide α aminé, naturel ou synthétique, dérivé d'acide α aminé, aminoacide en position N-terminale d'un peptide, ou d'un dérivé peptidique, éventuellement en présence d'un catalyseur tel que l'imidazole, dans un solvant approprié pour obtenir un dérivé de glycosylamine dans lequel l'ose terminal de l'oligoside conserve sa structure cyclique, et dans lequel l'hydroxyle semiacétalique est remplacé par l'amine α de l'une des susdites molécules de départ,

- lorsque la molécule intermédiaire ne possède pas de chaîne latérale contenant un groupe fonctionnel tel que défini ci-dessus, ou possède une chaîne latérale dont le groupe fonctionnel est éventuellement protégé, on acyle le dérivé de glycosylamine obtenu à l'issue de l'étape précédente par addition d'un acide organique activé par un activateur classique tel que le carbonyl diimidazole, le BOP (Benzotriazol-1-yloxytris(diméthylamino)-phosphonium hexafluorophosphate) ou HBTU (O-benzotriazol-1-yl-N,N,N'N'-tetramethyluronium hexafluorophosphate), pour obtenir un dérivé de glycosylamine N-acylé, suivi éventuellement d'une déprotection du groupe fonctionnel de la susdite chaîne latérale, en vue d'une éventuelle substitution,
- lorsque la molécule intermédiaire possède une chaîne latérale contenant un groupe carboxylique, on active le susdit groupe carboxylique afin qu'il réagisse intramoléculairement avec la susdite amine $\alpha$, entraînant une cyclisation à l'intérieur de la susdite molécule de départ, pour obtenir un dérivé de glycosylamine N-acylé,
- lorsque la molécule intermédiaire possède une chaîne latérale contenant un groupe carboxylique, on peut ajouter l'agent d'acylation sous forme d'un ester actif.

**19.** Procédé de préparation d'un composé selon l'une quelconque des revendications 1 à 6, caractérisé en ce que l'on fait réagir

- un ou plusieurs produits (dérivés de glycosylamine acylés) selon l'une des revendications 8 à 12, portant un groupe Q, activé ou activable, réagissant sur une molécule, matrice ou particule P, P' et P",
- un ou plusieurs produits (dérivés de glycosylamine acylés) selon l'une des revendications 8 à 12, portant soit un groupe R, activé ou activable, soit un groupe B' contenant un groupe fonctionnel activé ou activable, soit un groupe $A_i$ contenant un groupe fonctionnel sur une molécule, une matrice ou une particule, respectivement P, P' et P", contenant un groupe fonctionnel, pour obtenir un produit du type:
  (glycopeptidyl-R)$_n$-P, (glycopeptidyl-B')$_n$-P', ou (glycopeptidyl-$A_i$)$_n$-P", $n \geq 1$,
- un ou plusieurs produits (dérivés de glycosylamine acylés) de l'invention, portant d'une part un groupe R, activé ou activable, d'autre part un groupe B' contenant un groupe activé ou activable, ou un groupe $A_i$ contenant un groupe fonctionnel sur des molécules, matrices ou particules P et P', ou des molécules, matrices ou particules P et P",
- un ou plusieurs produits (dérivés de glycosylamine acylés) de l'invention, portant d'une part un groupe R, activé ou activable, d'autre part un groupe B' contenant un groupe activé ou activable, et un groupe $A_i$ contenant un groupe fonctionnel respectivement sur des molécules, matrices ou particules P, P' et P",
  R, B', $A_i$, P, P' et P" étant définis selon l'une quelconque des revendications 1 à 6.

**20.** Procédé selon la revendication 19 dans lequel le composé à substituer consistant en la molécule, matrice ou particule P, P' ou P", est une protéine, un lipide, un acide nucléique, un oligonucléotide, une polylysine, un polymère insoluble, une bille de latex ou une bille d'or.

## Patentansprüche

**1.** Verbindungen, umfassend ein oder mehrere Oligoside, wobei jedes der Oligoside über ein Zwischenmolekül, das aufweist ein an ein Kohlenstoffatom in $\alpha$-Stellung einer C=O-Gruppe gebundenes Stickstoffatom, wobei das Stickstoffatom N-acyliert ist, und eine oder mehrere funktionelle Gruppen, insbesondere eine, zwei oder drei, kovalent an ein oder mehrere Moleküle, Matrizen oder Partikel gebunden ist, vorzugsweise an eines, zwei oder drei, wobei jene eine Matrix als Träger für die Affinitätschromatographie; eine Gold-, Latexkugel für die Histologie und Zytologie; ein Protein, ein Lipid, Oligonukleotide oder auch Polymere für die Zielerkennung von Medikamenten, Oligonukleotiden oder Genen, darstellen,
wobei sich die kovalente Bindung zwischen dem Zwischenmolekül und dem Oligosid durch die Verbindung über das Stickstoffatom ergibt und sich die kovalente Bindung zwischen dem Zwischenmolekül und dem Molekül, der Matrix, dem Partikel oder den Molekülen, den Matrizen, den Partikeln durch die Verbindung über die funktionelle (n) Gruppe(n) des Zwischenmoleküls mit geeigneten funktionellen Gruppen auf dem (den) Molekül(en), der (den) Matrix (Matrizen) oder dem (den) Partikel(n) ergibt.

**2.** Verbindungen der allgemeinen Formel (I)

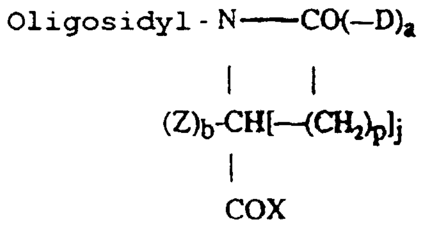

(I)

in der:

a = 0 oder 1,
j = 0 oder 1,
b = 0 oder 1,
p = 2 bis 4, insbesondere 2, ist,

mit der Maßgabe, daß a = b = 0 ist, falls j = 1 ist, was das Vorliegen eines cyclischen Moleküls bedeutet, bzw. daß a = b = 1 ist, falls j = 0 ist, was die Abwesenheit der $(CH_2)_p$-Gruppe impliziert,

D einen Rest einer organischen Säure der Formel $D(CO_2)H$, insbesondere H oder eine Alkylkette mit 1 bis 10 Kohlenstoffatomen, insbesondere $CH_3$, darstellt,
Z B, wobei B H, eine Alkylgruppe mit 1 bis 10 Kohlenstoffatomen oder eine Seitenkette einer natürlichen oder synthetischen α-Aminosäure, wie $CH(CH_3)_2$, $CH_2OH$, $CH_3$, und vorzugsweise H ist, oder
B'-P' darstellt, wobei B' eine Alkylidenkette mit 1 bis 10 Kohlenstoffatomen oder eine Seitenkette einer natürlichen oder synthetischen α-Aminosäure ist, wobei die Ketten eine freie oder geschützte, vorzugsweise geschützte Gruppe enthalten, die von einer funktionellen Gruppe abgeleitet ist, die aktiviert sein kann, wie Carboxyl, SH, OH oder Amin, und P' die nachstehend gezeigten Bedeutungen besitzt, X die Gruppe

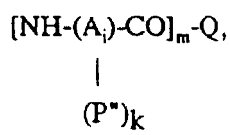

oder die Gruppe

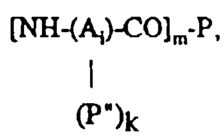

oder die Gruppe

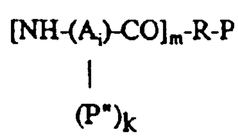

darstellt,
m eine Zahl von 0 bis 10, vorzugsweise von 0 bis 5 und vorteilhaft 1 oder 2 ist, k = 0 oder 1 ist,
Q OH, $OCH_3$, $OCH_2$-$C_6H_5$, O-$C_6H_5$, O-$C_6F_5$, O-p$C_6H_4$-$NO_2$,

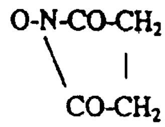

darstellt,
R eine Gruppe, umfassend eine Alkohol-, Phenol-, Thiol- oder Amin-Funktion, darstellt,
P wie nachstehend definiert ist,
$A_i$ ein organischer Rest, wie eine Alkylidenkette mit 1 bis 10 Kohlenstoffatomen, insbesondere $(CH_2)_n$-W-$(CH_2)_{n'}$, darstellt,
wobei n + n' eine ganze Zahl von 0 bis 10 darstellt, W CHY darstellt, Y H, eine lineare oder verzweigte Alkylgruppe mit 1 bis 6 Kohlenstoffatomen, ein Rest einer natürlichen oder synthetischen $\alpha$-Aminosäure ist oder W eine aromatische Verbindung, insbesondere Phenol, darstellt,
wobei P, P' und P" identisch oder verschieden sind und darstellen:

- eine Matrix als Träger für die Affinitätschromatographie;
- eine Gold-, Latexkugel für die Histologie und Zytologie;
- ein Protein für die Sichtbarmachung, die Reinigung, insbesondere 1) von spezifischen Osidrezeptoren, die Lectine, Adhesine, Agglutinine usw. genannt werden, oder auch 2) von Proteinen mit oder ohne enzymatischer Aktivität, die Affinität für die Oside aufweisen, insbesondere Glycosyltransferasen wie die Sialyltransferase, Sulfotransferasen, Phosphotransferasen, Exoglycosidasen oder Endoglycosidasen;
- ein Lipid für die Charakterisierung der vorstehenden Rezeptoren;
- Oligonukleotide für die selektive Erhöhung ihrer Bindung durch die Zielzellen;
- ein Protein oder Polymere für die Zielerkennung von Medikamenten, Oligonukleotiden oder Genen,

wobei P, P' und P" mindestens eine Funktion besitzen, die eine Kondensationsreaktion durch Umsetzung mit einem Oligopeptid erlaubt, beispielsweise

- eine Aminfunktion ($-NH_2$), die die Bildung eines Amids mit einem aktiven Ester, eines Amidins mit einem Imidat, eines Thioharnstoffs mit einem Isothiocyanat erlaubt,
- eine Thiolfunktion (-SH), die die Bildung einer Disulfidbrücke mit einem ein Thiol enthaltenden Oligopeptid, eines Thioethers mit einem eine Maleinimid- oder eine Halogenalkyl- oder eine Halogenalkanoylgruppe enthaltenden Oligopeptid erlaubt, oder
- ein Phenol ($-C_6H_4OH$), das die Bildung einer Azoverbindung mit einem eine Diazogruppe enthaltenden Oligopeptid erlaubt, mit der Maßgabe, daß Z B'-P' darstellt und/oder X P und/oder P" in seiner Formel enthält.

3. Verbindungen nach einem der Ansprüche 1 oder 2 mit der allgemeinen Formel (II)

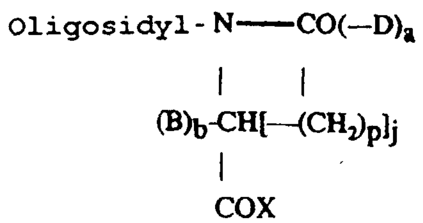

(II)

in der

a = 0 oder 1,
j = 0 oder 1,
b = 0 oder 1,
p = 2 bis 4, insbesondere 2, ist,

mit der Maßgabe, daß a = b = 0 ist, falls j = 1 ist, was das Vorliegen eines cyclischen Moleküls bedeutet, bzw. daß a = b = 1 ist, falls j = 0 ist, was die Abwesenheit der $(CH_2)_p$-Gruppe impliziert,

D einen Rest einer organischen Säure der Formel $D(CO_2)H$, insbesondere H oder eine Alkylkette mit 1 bis 10 Kohlenstoffatomen, insbesondere $CH_3$, darstellt,
B H, eine Alkylgruppe mit 1 bis 10 Kohlenstoffatomen oder eine Seitenkette einer α-Aminosäure wie $CH(CH_3)_2$, $CH_2OH$, $CH_3$, und vorzugsweise H, darstellt,
X entweder die Gruppe $[NH-(A_i)-CO]_m-P$ oder die Gruppe $[NH-(A_i)-CO]_m-R-P$ darstellt,
m eine Zahl von 0 bis 10, vorzugsweise von 0 bis 5 und vorteilhaft 1 oder 2 ist, R und P wie nachstehend definiert sind,
$A_i$ einen organischen Rest, wie eine Alkylidenkette mit 1 bis 10 Kohlenstoffatomen, insbesondere $(CH_2)_n-W-(CH_2)_{n'}$, darstellt,
wobei n + n' eine ganze Zahl von 0 bis 10 darstellt, W CHY darstellt, Y H, eine lineare oder verzweigte Alkylgruppe mit 1 bis 6 Kohlenstoffatomen, ein Rest einer natürlichen oder synthetischen α-Aminosäure ist oder W eine aromatische Verbindung, insbesondere Phenyl, darstellt,
wobei R eine Gruppe, umfassend eine Alkohol-, Phenol-, Thiol- oder Amin-Funktion, darstellt,
P darstellt:

- eine Matrix als Träger für die Affinitätschromatographie;
- eine Gold-, Latexkugel für die Histologie und Zytologie;
- ein Protein für die Sichtbarmachung, die Reinigung, besonders 1) von spezifischer Osidrezeptoren, die Lectine, Adhesine, Agglutinine usw. genannt werden oder auch 2) von Proteinen mit oder ohne enzymatischer Aktivität, die Affinität für die Oside aufweisen, insbesondere Glycosyltransferasen wie die Sialyltransferase, Sulfotransferasen, Phosphotransferasen, Exoglycosidasen oder Endoglycosidasen;
- ein Lipid für die Charakterisierung der vorstehenden Rezeptoren;
- Oligonukleotide für die selektive Erhöhung ihrer Bindung durch die Zielzellen;
- ein Protein oder Polymere für die Zielerkennung von Medikamenten, Nukleotiden oder Genen.

**4.** Verbindungen nach einem der Ansprüche 1 oder 2 mit der allgemeinen Formel (III)

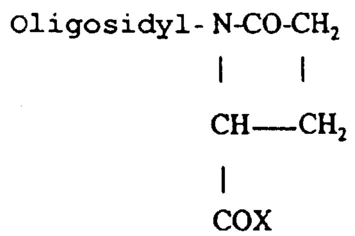

(III)

in der X $[NH-(A_i)-CO]_m-P$ oder $[NH-(A_i)-CO]_m-R-P$ darstellt, wobei $A_i$, m, P und R die in Anspruch 2 gezeigten Bedeutungen besitzen.

**5.** Verbindungen nach einem der Ansprüche 1 oder 2 mit der allgemeinen Formel (IV)

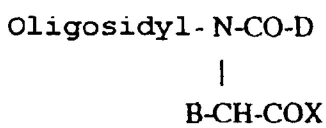

(IV)

in der D und B die in Anspruch 2 gezeigten Bedeutungen besitzen, und X $[NH-(A_i)-CO]_m-P$ oder $[NH-(A_i)-CO]_m-R-P$ darstellt, wobei $A_i$, m, R und P die in Anspruch 2 gezeigten Bedeutungen besitzen.

**6.** Verbindungen nach einem der Ansprüche 1 oder 2 mit der allgemeinen Formel (V)

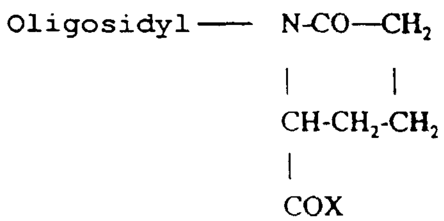

(V)

in der X $[NH-(A_i)-CO]_m$-P oder $[NH-(A_i)-CO]_m$-R-P darstellt, wobei P, $A_i$, m und R die in Anspruch 2 gezeigten Bedeutungen besitzen.

**7.** Produkte, die insbesondere als Zwischenprodukte für die Herstellung einer der Verbindungen nach einem der Ansprüche 1 bis 6 dienen können, wobei die Produkte ein Oligosid enthalten, das an ein Molekül gebunden ist, welches ein Stickstoffatom, gebunden an ein Kohlenstoffatom in α-Stellung einer C=O-Gruppe, und mindestens eine funktionelle Gruppe, insbesondere eine, zwei oder drei funktionelle Gruppen, umfaßt, wobei sich die kovalente Bindung zwischen dem Oligosid und dem Molekül durch die Verbindung über das Stickstoffatom ergibt.

**8.** Produkte der allgemeinen Formel (Ia)

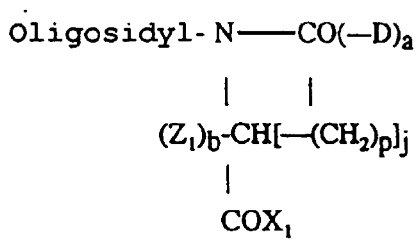

(Ia)

in der

a = 0 oder 1,
j = 0 oder 1,
b = 0 oder 1,
p = 2 bis 4, insbesondere 2, ist,

mit der Maßgabe, daß a = b = 0 ist, falls j = 1 ist, was das Vorliegen eines cyclischen Moleküls bedeutet, bzw. daß a = b = 1 ist, falls j = 0 ist, was die Abwesenheit der $(CH_2)_p$-Gruppe impliziert,

D einen Rest einer organischen Säure der Formel $D(CO_2)H$, insbesondere H oder eine Alkylkette mit 1 bis 10 Kohlenstoffatomen, insbesondere $CH_3$, darstellt,
$Z_1$ B, wobei B aus H, einer Alkylkette mit 1 bis 10 Kohlenstoffatomen oder einer Seitenkette einer α-Aminosäure wie $CH(CH_3)_2$, $CH_2OH$, $CH_3$, und vorzugsweise H ausgewählt ist, oder B' darstellt, wobei B' aus einer Alkylidenkette mit 1 bis 10 Kohlenstoffatomen oder einer Seitenkette einer α-Aminosäure ausgewählt ist, wobei die Ketten eine freie oder geschützte funktionelle Gruppe, wie Carboxyl, SH, OH oder Amin, enthalten,
X die Gruppe $[NH-(A_i)-CO]_m$-R,
die Gruppe$[NH-(A_i)-CO]_m$-Q darstellt,
R eine Gruppe, umfassend eine Alkohol-, Phenol-, Thiol- oder Amin-Funktion, darstellt,
Q OH, $OCH_3$, $OCH_2-C_6H_5$, $O-C_6H_5$, $O-C_6F_5$, $O-pC_6H_4-NO_2$,

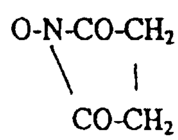

darstellt,

m eine ganze Zahl von 0 bis 10, vorzugsweise von 0 bis 5 und vorteilhaft 1 oder 2 ist,
$A_i$ einen organischen Rest wie eine Alkylidenkette mit 1 bis 10 Kohlenstoffatomen, insbesondere $(CH_2)_n$-W-$(CH_2)_n$, darstellt,
wobei n + n' eine ganze Zahl von 0 bis 10 darstellt, W CHY darstellt, Y H, eine lineare oder verzweigte Alkylgruppe mit 1 bis 6 Kohlenstoffatomen, ein Rest einer natürlichen oder synthetischen α-Aminosäure ist oder W eine aromatische Verbindung, insbesondere Phenyl, darstellt.

**9.** Produkte nach einem der Ansprüche 7 oder 8 der allgemeinen Formel (IIa)

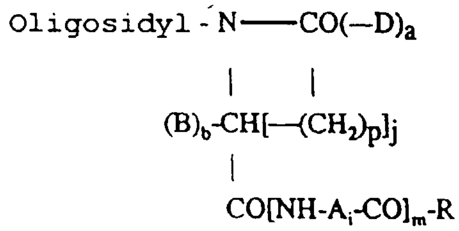

(IIa)

in der

a = 0 oder 1,
j = 0 oder 1,
b = 0 oder 1,
p = 2 bis 4, insbesondere 2, ist,

mit der Maßgabe, daß a = b = 0 ist, falls j = 1 ist, was das Vorliegen eines cyclischen Moleküls bedeutet, bzw. daß a = b = 1 ist, falls j = 0 ist, was die Abwesenheit der $(CH_2)_p$-Gruppe impliziert,

D einen Rest einer organischen Säure der Formel $D(CO_2)H$, insbesondere H oder eine Alkylkette mit 1 bis 10 Kohlenstoffatomen, insbesondere $CH_3$, darstellt,
B H, eine Alkylkette mit 1 bis 10 Kohlenstoffatomen oder eine Seitenkette einer α-Aminosäure wie $CH(CH_3)_2$, $CH_2OH$, $CH_3$, und vorzugsweise H darstellt,
m eine ganze Zahl von 0 bis 10, vorzugsweise von 0 bis 5 und vorteilhaft 1 oder 2 ist,
$A_i$ einen organischen Rest wie eine Alkylidenkette von 1 bis 10 Kohlenstoffatomen, insbesondere $(CH_2)_n$-W-$(CH_2)_n$, darstellt,
wobei n + n' eine ganze Zahl von 0 bis 10 darstellt, wobei W CHY darstellt, Y H, eine lineare oder verzweigte Alkylgruppe mit 1 bis 6 Kohlenstoffatomen, ein Rest einer natürlichen oder synthetischen α-Aminosäure ist oder W eine aromatische Verbindung, insbesondere Phenyl, darstellt,
R eine Gruppe, umfassend eine Alkohol-, Phenol-, Thiol- oder Amin-Funktion, darstellt.

**10.** Produkte nach einem der Ansprüche 7 oder 8 mit der allgemeinen Formel (IIIa)

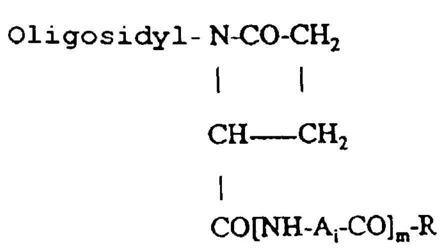

(IIIa)

in der Ai, m und R die in Anspruch 8 gezeigten Bedeutungen besitzen.

**11.** Produkte nach einem der Ansprüche 7 oder 8 mit der allgemeinen Formel (IVa)

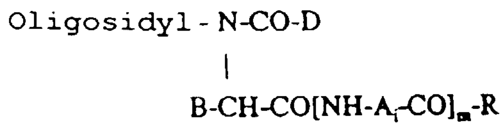

(IVa)

in der D, B, m, $A_i$ und R die in Anspruch 8 gezeigten Bedeutungen besitzen.

**12.** Produkte nach einem der Ansprüche 7 oder 8 mit der allgemeinen Formel (Va)

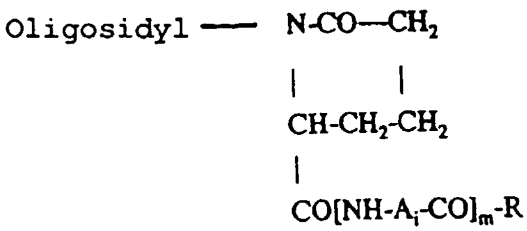

(Va)

in der $A_i$, m und R die in Anspruch 8 gezeigten Bedeutungen besitzen.

**13.** Produkte der Formel (VI)

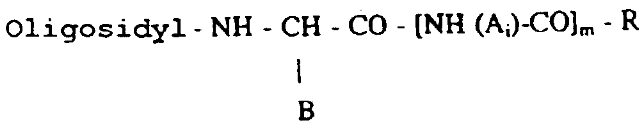

(VI)

in der B, $A_i$, m und R die in Anspruch 2 gezeigten Bedeutungen besitzen.

**14.** Produkte der Formel (VII)

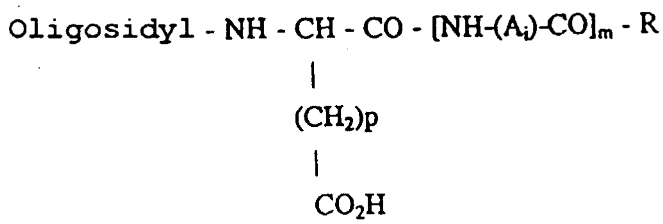

(VII)

in der p, $A_i$, R, m die in Anspruch 2 gezeigten Bedeutungen besitzen.

**15.** Verbindungen oder Produkte nach einem der Ansprüche 1 bis 14, dadurch gekennzeichnet, daß Q die folgenden Reste darstellt:

OH, $O-CH_2-C_6H_5$, $O-C_6H_5$, $O-C_6F_5$, $O-pC_6H_4-NO_2$,

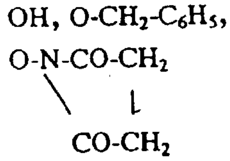

und dadurch gekennzeichnet, daß R einen der folgenden Reste darstellt:

- NH-p$C_6H_4$-N=C=S und seine Vorläufer:

$$-NH-pC_6H_4-NO_2$$

$$-NH-pC_6H_4-NH_2$$

$$-NH-CH_2-(CH_2)_m-C(=NH_2^+)OCH_3$$

$$-NH-CH_2-(CH_2)_m-CN$$

$$-NH-CH_2-(CH_2)_m-CH_2-NH-CO-CH_2-(CH_2)_m-C(=NH_2^+)OCH_3$$

$$-NH-CH_2-(CH_2)_m-CH_2-NH-CO-CH_2-(CH_2)_m-CN$$

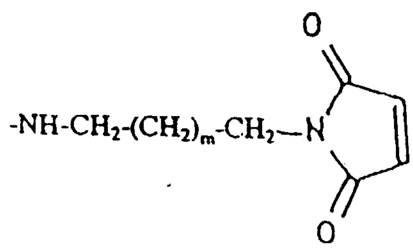

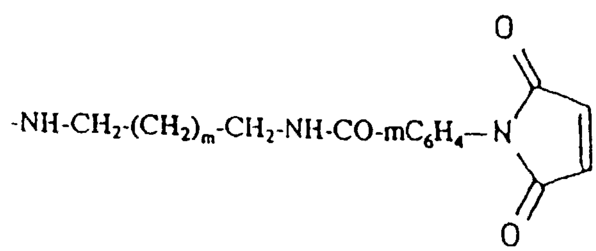

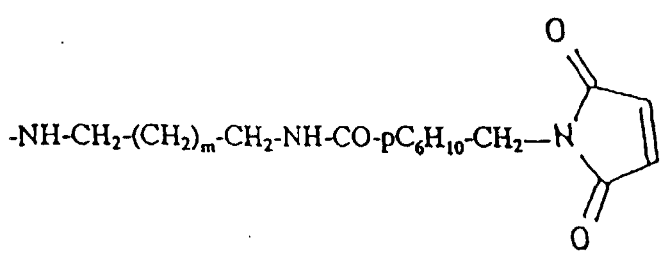

$$-NH-CH_2-(CH_2)_m-CH_2-NH-CO-CH_2-T \qquad T = Br, I, Cl$$

$$-NH-CH_2-CH_2-NH-CO-CH_2(CH_2)_m-S-S-Pyr$$

$-NH-CH_2-(CH_2)_m-CH_2-S-S-Pyr$

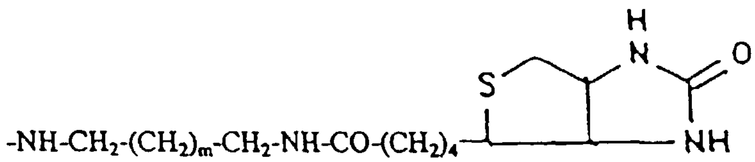

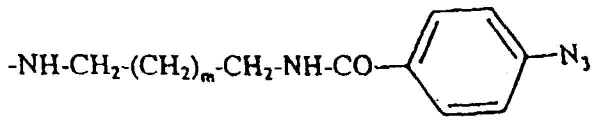

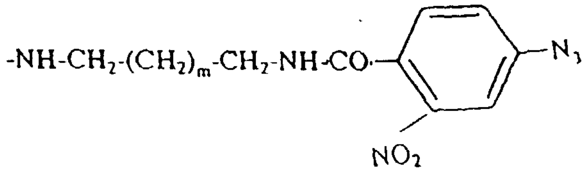

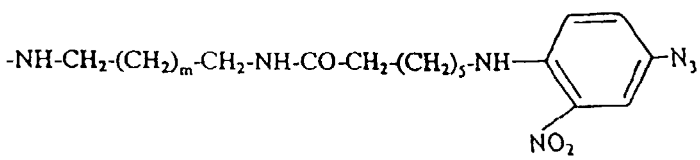

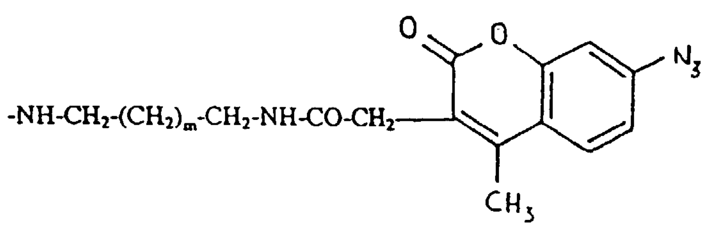

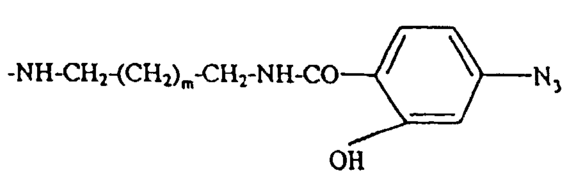

$-NH-(CH_2)_m-pC_6H_4OH$

$-NH-CH_2-(CH_2)_m-CH_2-NH-CO-(CH_2)_m-pC_6H_4OH$

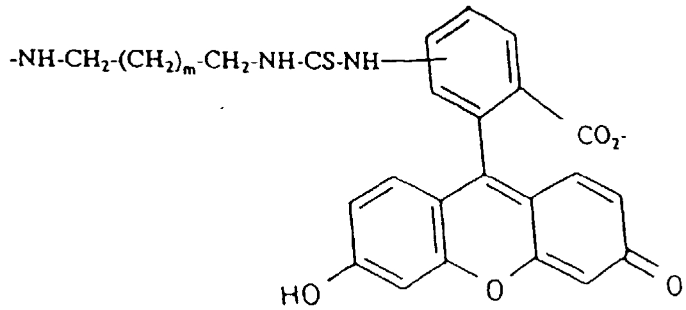

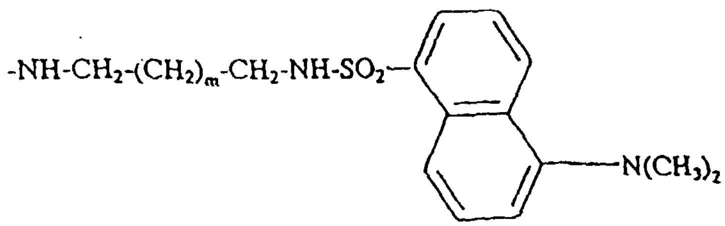

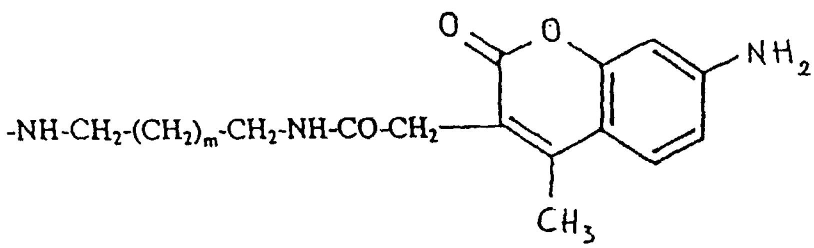

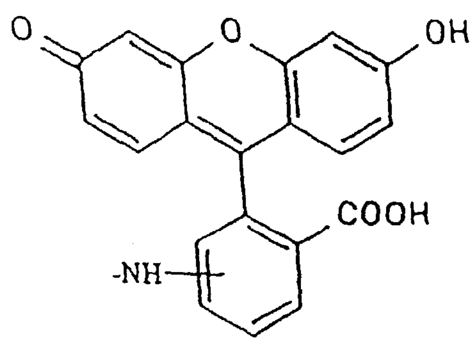

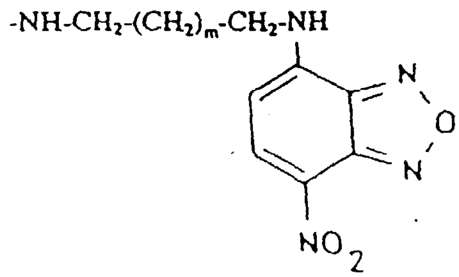

wobei m eine ganze Zahl von 0 bis 10, vorzugsweise 0 bis 5 und vorteilhaft 1 oder 2 darstellt, Pyr eine 2-Pyridin-Gruppe darstellt.

**16.** Verbindung nach einem der Ansprüche 1 oder 2, dadurch gekennzeichnet, daß P ein Oligopeptid oder ein Polypeptid, insbesondere gluconoyliertes Polylysin, darstellt und P' oder P" ein Oligonukleotid darstellen, oder R Fluorescein oder eines seiner Derivate oder ein anderes fluoreszierendes Derivat darstellt, und P' oder P" ein Oligonukleotid darstellen oder P ein therapeutisches Mittel oder irgendein Molekül von Interesse darstellt.

**17.** Verbindungen nach einem der Ansprüche 1 bis 12, dadurch gekennzeichnet, daß der oligosidische Rest ein 2 bis 50 Saccharide enthaltendes Oligosaccharid ist und insbesondere ausgewählt ist aus:

| | |
|---|---|
| Lacto-N-tetraose | Galβ 3 GlcNAcβ 3 Gal β 4 Glc |
| Neolacto-N-tetraose | Galβ 4 GlcNAcβ 3 Gal β 4 Glc |
| H-Gruppe | Fuc α 2 Galβ 3 Galβ 4 Glc |
| Lewis[a] | Galβ 3 GlcNAcβ 3 Gal β 4 Glc<br>Fuc α 4-↑ |
| Lewis[x] | Galβ 4 GlcNAcβ 3 Galβ 4 Glc<br>Fuc α 3-↑ |
| Lewis[b] | Fucα 2 Galβ 3 GlcNAc β 3 Galβ 4 Glc<br>Fuc α 4-↑ |
| Lewis[y] | Fuc α 2 Galβ 4 GlcNAcβ 3 Galβ 4 Glc<br>Fuc α 3-↑ |
| Disialolacto-N-tetraose | Neu 5Acα 3 Gal β 3 GlcNAc β 3 Gal β 4 Glc<br>Neu 5 Acα 6-↑ |

Komplexer Typ mit drei Antennen

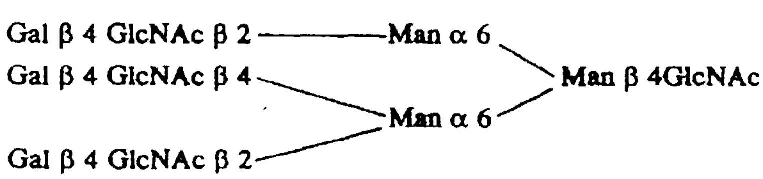


| | |
|---|---|
| Sialylactose 3 | Neu 5Ac α 3 Gal 4 Glc |

(fortgesetzt)

| | |
|---|---|
| Sialylactose 6 | Neu 5Ac α 6 Gal β 4 Glc |
| Disialylactose 3 | Neu 5Ac α 8 Neu 5Ac α 3 Gal β 4 Glc |

- einfachen oder komplexen Sacchariden, die von membranständigen Lectinen erkannt werden und beispielsweise ausgewählt sind aus:

a. Asialo-Oligosid des dreiantennigen Lactosamintyps: Asialoglycoproteinrezeptor

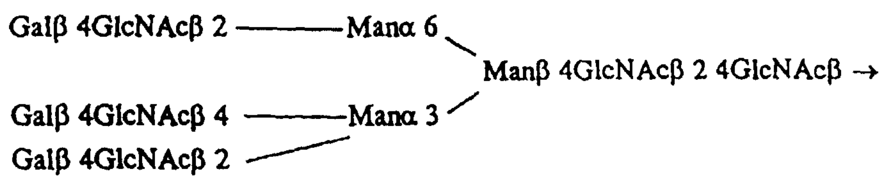

b. Asialo-Oligosid des vierantennigen Lactosamintyps: Asialoglycoproteinrezeptor

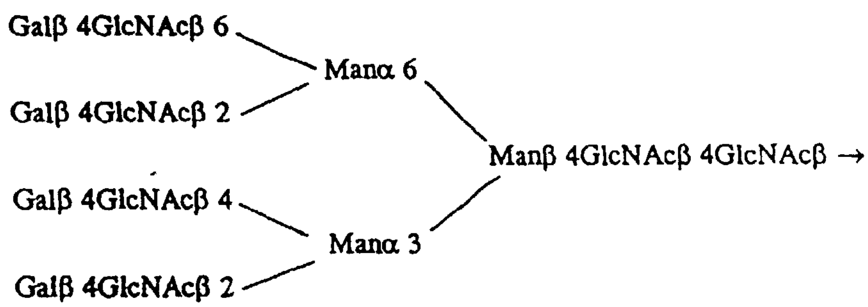

c. Lewis[x]: LECAM 2/3

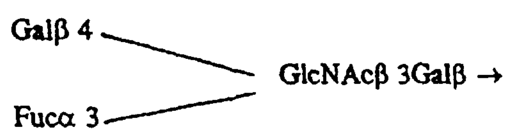

d. Sialyl-Lewis[x]: LECAM 3/2

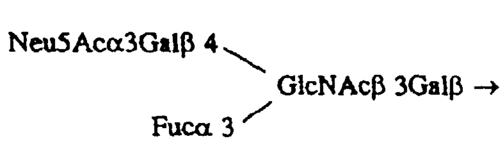

e. Derivat des Lewis[x]-Sulfats (HNK1): LECAM 1

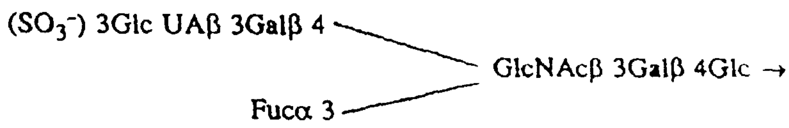

f. Oligomannosid: Mannoserezeptor

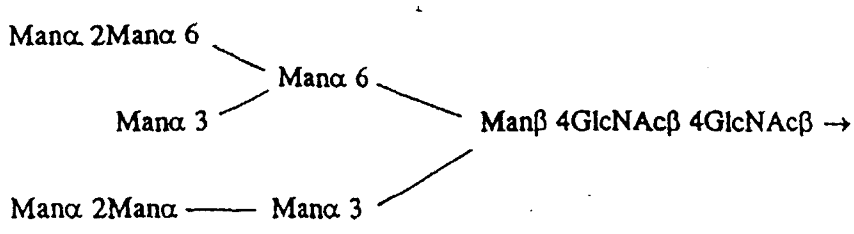

g. phosphoryliertes Oligomannosid: Mannose-6-phosphat-Rezeptor

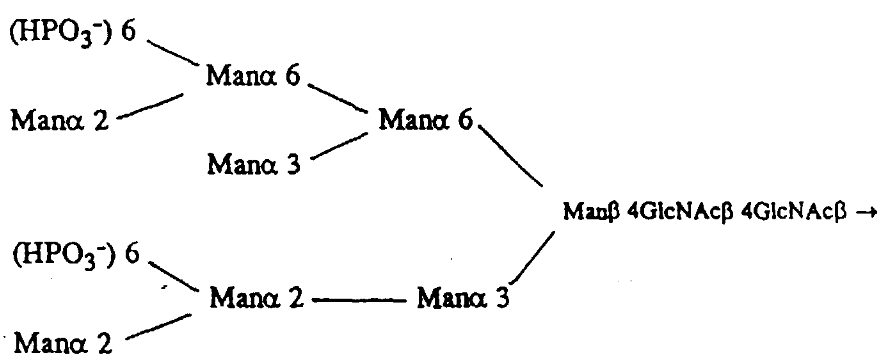

h. Oligosaccharid des Lactosaminsulfattyps: GalNAc4-sulfat

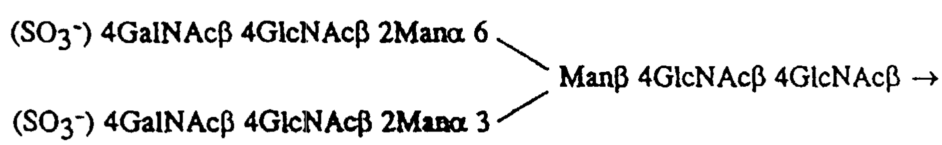

**18.** Verfahren der Herstellung der Verbindungen nach einem der Ansprüche 7 bis 12, dadurch gekennzeichnet, daß:

- ein Oligosid mit einem freien reduzierenden Saccharid in einem geeigneten Lösungsmittel mit einem Stickstoffatom eines Zwischenmoleküls kondensiert wird, wobei dieses Stickstoffatom zu einer Amino-Gruppe gehört, die an ein Kohlenstoffatom in α-Stellung einer C=O-Gruppe gebunden ist, wobei das Zwischenmolekül gegebenenfalls eine Seitenkette besitzt, die eine freie oder ungeschützte funktionelle Gruppe wie OH, SH, $NH_2$ oder COOH enthält, wobei dieses Ausgangsmolekül aus den folgenden Zwischenmolekülen ausgewählt ist: natürliche oder synthetische α-Aminosäure, Derivat einer α-Aminosäure, Aminosäure in N-terminaler Position eines Peptids oder eines peptidischen Derivats, gegebenenfalls in Gegenwart eines Katalysators wie Imidazol in einem geeigneten Lösungsmittel, um ein GlycosylaminDerivat zu erhalten, in dem das terminale Saccharid des Oligosids seine cyclische Struktur beibehält und in dem das semiacetalische Hydroxyl durch das α-Amin eines der Ausgangsmoleküle ersetzt ist,
- falls das Zwischenmolekül nicht eine Seitenkette besitzt, die eine wie vorstehend definierte funktionelle Gruppe enthält, oder eine Seitenkette besitzt, in der die funktionelle Gruppe gegebenenfalls geschützt ist, das am Ende des vorstehenden Schritts erhaltene Glycosylaminderivat durch Zugabe einer durch einen klassischen

Aktivator, wie Carbonyldiimidazol, BOP (Benzotriazol-1-yloxytris(dimethylamino)-phosphoniumhexafluorphosphat) oder HBTU (O-Benzotriazol-1-yl-,N,N,N',N'-tetramethyluroniumhexafluorphosphat), aktivierten organischen Säure acyliert wird, um eine N-acyliertes Glycosylaminderivat zu erhalten, gegebenenfalls gefolgt von einer Entschützung der funktionellen Gruppe der Seitenkette im Hinblick auf eine optionale Substitution,

- falls das Zwischenmolekül eine eine Carboxylgruppe enthaltende Seitenkette besitzt, die Carboxylgruppe aktiviert wird, um sie intramolekular mit dem α-Amin umzusetzen, was eine Cyclisierung innerhalb des Ausgangsmolekül zur Folge hat, um ein Derivat des N-acylierten Glycosylaminderivats zu erhalten.
- falls das Zwischenmolekül eine eine Carboxylgruppe enthaltende Seitenkette besitzt, ein Acylierungsmittel in Form eines aktiven Esters zugegeben werden kann.

**19.** Verfahren der Herstellung einer Verbindung nach einem der Ansprüche 1 bis 6, dadurch gekennzeichnet, daß umgesetzt werden:

- ein oder mehrere Produkte (acylierte Derivate des Glycosylamins) nach einem der Ansprüche 8 bis 12, die eine aktivierte oder aktivierbare Gruppe Q tragen, mit einem Molekül, einer Matrix oder einem Partikel P, P' und P",
- ein oder mehrere Produkte (acylierte Derivate des Glycosylamins) nach einem der Ansprüche 8 bis 12, die entweder eine aktivierte oder aktivierbare Gruppe R oder eine Gruppe B', enthaltend eine aktivierte oder aktivierbare funktionelle Gruppe, oder eine Gruppe $A_i$, enthaltend eine funktionelle Gruppe, tragen, mit einem Molekül, einer Matrix oder einem Partikel P, P' bzw. P", enthaltend eine funktionelle Gruppe, um ein Produkt des Typs $(\text{Glycopeptidyl-R})_n$-P, $(\text{Glycopeptidyl-B'})_n$-P' oder $(\text{Glycopetidyl-A}_i)_n$-P", $n \geq 1$, zu erhalten,
- ein oder mehrere erfindungsgemäße Produkte (acylierte Derivate des Glycosylamins), die einerseits eine aktivierte oder aktivierbare Gruppe R, andererseits eine Gruppe B', enthaltend eine aktivierte oder aktivierbare Gruppe, oder eine Gruppe $A_i$, enthaltend eine funktionelle Gruppe, tragen, mit Molekülen, Matrizen oder Partikeln P und P' oder Molekülen, Matrizen oder Partikeln P und P",
- ein oder mehrere erfindungsgemäße Produkte (acylierte Derivate des Glycosylamins), die einerseits eine aktivierte oder aktivierbare Gruppe R, andererseits eine Gruppe B', enthaltend eine aktivierte oder aktivierbare Gruppe, und eine Gruppe $A_i$, enthaltend eine funktionelle Gruppe, tragen, jeweils mit Molekülen, Matrizen oder Partikeln P, P' und P",

wobei R, B', $A_i$, P, P' und P" wie nach einem der Ansprüche 1 bis 6 definiert sind.

**20.** Verfahren nach Anspruch 19, wobei die aus dem Molekül, der Matrix oder dem Partikel P, P' oder P" bestehende zu substituierende Verbindung ein Protein, ein Lipid, eine Nukleinsäure, ein Oligonukleotid, ein Polylysin, ein unlösliches Polymer, eine Latexkugel oder eine Goldkugel ist.

## Claims

**1.** Compounds comprising one or several oligosides, each of said oligosides being fixed in a covalent manner on one or several molecules, matrices or particles, which represent a matrix as a support for affinity chromatography ; a bead of gold or latex, for histology and cytology ; a protein, a lipid, oligonucleotides or polymers for the targeting of drugs, of oligonucleotides or of genes, specifically one, two or three, via an intermediary molecule possessing a nitrogen atom carried by a carbon in α of a group C = O, the nitrogen atom being N-acetylated, and one or several functional groups, specifically one, two or three, the covalent link between said intermediary molecule and the oligoside taking place by the intermediary of said nitrogen atom, and the covalent link between said intermediary molecule and said molecule, said matrix, said particle, or said molecules, said matrices, said particles taking place by the intermediary of the said functional group(s) of said intermediary molecule and the functional groups appropriate to the molecule(s), the matrix(ces) or the particle(s).

**2.** Compounds of the general formula (I)

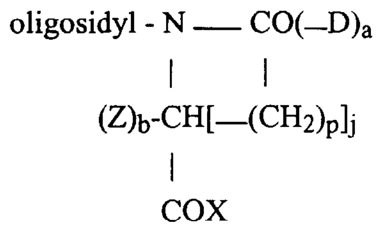

(I)

in which :

* $a = 0$ or 1,
* $j = 0$ or 1,
* $b = 0$ or 1,
* $p = 2$ to 4, in particular 2,
* provided that
  ** $a = b = 0$, when $j = 1$, which leads to the presence of a cyclic molecule,
  ** or $a = b = 1$, when $j = 0$ which implies the absence of the $(CH_2)_p$ group,
* D represents a residue of an organic acid of the formula $DCO_2H$, in particular H or an alkyl chain of 1 to 10 carbon atoms, in particular $CH_3$,
* Z represents
  ** B, B being H, an alkyl of 1 to 10 carbon atoms or a side chain of an α amino acid, natural or synthetic, such as $CH(CH_3)_2$, $CH_2OH$, $CH_3$, and preferably H, or
  ** B'-P', B' being an alkyl chain of 1 to 10 carbon atoms or a side chain on an α amino acid, natural or synthetic, the said chains containing a group derived from a functional group able to be activated, such as carboxylic, SH, OH, or amine, free or protected, preferably protected, P' having the significations indicated hereafter,
* X represents :
  • the group

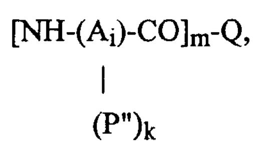

  • or the group

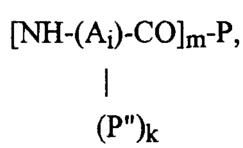

  • or the group

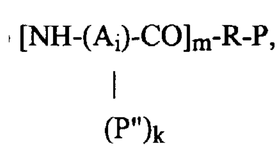

* m being an integer from 0 to 10, preferably from 0 to 5 and advantageously 1 or 2, k = 0 or 1
* Q representing OH, $OCH_3$, $OCH_2$-$C_6H_5$, O-$C_6H_5$, O-$C_6F_5$, O-p$C_6H_4$-$NO_2$,

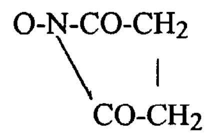

* R representing a group possessing an alcohol, phenol, thiol, or amine function,
* P being such as is defined hereafter,
* $A_i$ representing an organic radical such as an alkyl chain of 1 to 10 carbon atoms, in particular $(CH_2)_n$-W-$(CH_2)_{n'}$, n + n' representing an integer from 0 to 10, W representing CHY, Y being H, an alkyl from 1 to 6 linear or branched carbon atoms, an $\alpha$ amino acid residue, natural or synthetic, or W representing an aromatic compound, in particular phenyl group,
* P, P' and P" are identical or different and represent :

  - a matrix as a support for the affinity chromatography ;
  - a bead of gold, of latex, for histology and cytology ;
  - a protein for the visualization, purification, in particular 1) specific receptors of osides, receptors which are called lectins, adhesins, agglutinins, etc., or 2) proteins with or without enzymatic activity, which have an affinity for the osides, in particular the glycosyltransferases, such as sialyltransferase, sulfotransferases, phosphotransferases, exoglycosidases, or endoglycosidases ;
  - a lipid for the characterization of the preceding receptors ;
  - oligonucleotides to selectively increase their capture by target cells ;
  - a protein or polymers for the targeting of drugs, oligonucleotides or genes ;

* P, P' and P" possessing at least one chemical function allowing a condensation reaction by reaction with an oligopeptide, for example

  • an amine function (-$NH_2$) allowing the formation of an amide with an active ester, an amidine with an imidate, a thiourea with an isothiocyanate,
  • a thiol function (-SH) allowing the formation of a disulfide bridge with an oligopeptide containing a thiol, a thioether with an oligopeptide containing a maleimide group, or halogeno-alkyle, or a halogeno-alkanoyle,
  • a phenol (-$C_6H_4$OH) allowing the formation of an azoic with an oligopeptide containing a diazoïc,

  provided that if Z represents B'-P', and/or X comprises P and/or P" in the formula.

**3.** Compounds according to any one of claims 1 or 2, of the general formula (II)

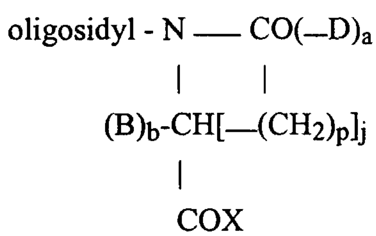

(II)

in which :

* a = 0 or 1,
* j = 0 or 1,
* b = 0 or 1,
* p = 2 to 4, in particular 2,
* provided that

** $a = b = 0$, when $j = 1$, which leads to the presence of a cyclic molecule,
** or $a = b = 1$, when $j = 0$ which implies the absence of the $(CH_2)_p$ group,

* D represents a residue of an organic acid of the formula $DCO_2H$, in particular H or an alkyl chain of 1 to 10 carbon atoms, in particular $CH_3$,
* B represents H, an alkyl of 1 to 10 carbon atoms, or a side chain of an α amino acid such as $CH(CH_3)_2$, $CH_2OH$, $CH_3$, and preferably H,
* X represents:

  • either the group $[NH-(A_i)-CO]_m$-P,
  • or the group $[NH-(A_i)-CO]_m$-R-P

  m being an integer from 0 to 10, preferably from 0 to 5 and advantageously 1 or 2, R and P being as defined hereinafter,

  * $A_i$ represents an organic radical such as an alkyl chain of 1 to 10 carbon atoms, in particular $(CH_2)_n$-W-$(CH_2)_{n'}$, n + n' representing an integer from 0 to 10, W representing CHY, Y being H, an alkyl from 1 to 6 linear or branched carbon atoms, an α amino acid residue, natural or synthetic, or W representing an aromatic compound, in particular phenyl,

* R represents a group possessing an alcohol, phenol, thiol, or amine function,
* P represents :

  - a matrix as a support for the affinity chromatography ;
  - a bead of gold, of latex, for histology and cytology ;
  - a protein for the visualization, purification, etc., particularly 1) specific receptors of osides, receptors which are named lectins, adhesins, agglutinins, etc... or 2) proteins having or not an enzymatic activity, which have an affinity for osides, particularly glycosyltransferases, such as sialyltransferase, sulfotransferases, phosphotransferases, exoglycosidases or endoglycosidases ;
  - a lipid for the characterization of the preceding receptors ;
  - oligonucleotides to selectively increase their capture by target cells ;
  - a protein or polymer for the targeting of drugs, oligonucleotides or genes.

**4.** Compounds according to any one of claims 1 or 2, of the general formula (III)

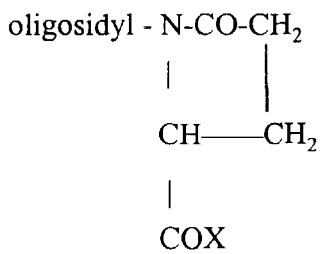

(III)

in which X represents $[NH-(A_i)-CO]_m$-P or $[NH-(A_i)-CO]_m$-R-P, $A_i$, m, P and R having the significations indicated in claim 2.

**5.** Compounds according to any one of claims 1 or 2, of the general formula (IV)

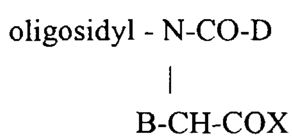

(IV)

in which D and B have the significations indicated in claim 2, and X represents $[NH-(A_i)-CO]_m$-P or $[NH-(A_i)-CO]_m$-R-P, $A_i$, m, R and P having the significations indicated in claim 2.

**6.** Compounds according to any one of claims 1 or 2, of the general formula (V)

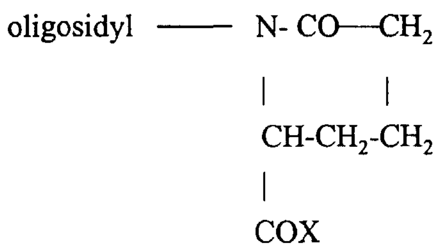

(V)

in which X represents $[NH-(A_i)-CO]_m$-P or $[NH-(A_i)-CO]_m$-R-P, P, $A_i$, m and R having the significations indicated in claim 2.

**7.** Products, able in particular to serve as intermediary products for the preparation of a compound according to any of claims 1 to 6, said products comprising an oligoside linked to a molecule possessing a nitrogen atom, carried by a carbon in α of a group C = O, and at least one functional group, in particular one, two or three functional groups, the covalent link between the oligoside and the molecule being made by the intermediary of said nitrogen atom.

**8.** Products of the general formula (Ia)

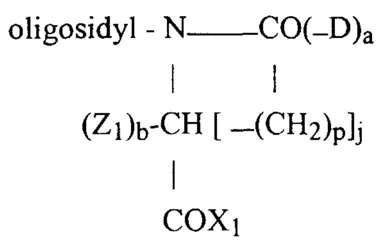

(Ia)

in which:

* a = 0 or 1,
* j = 0 or 1,
* b = 0 or 1,
* p = 2 to 4, in particular 2,
* provided that
    ** a = b = 0, when j = 1, which leads to the presence of a cyclic molecule,
    ** or a = b = 1, when j = 0 which implies the absence of the $(CH_2)_p$ group,
* D represents a residue of an organic acid of the formula $DCO_2H$, in particular H or an alkyl chain of 1 to 10 carbon atoms, in particular $CH_3$,
* $Z_1$ represents
    * B, B being chosen from among: H, an alkyl chain of 1 to 10 carbon atoms, or a side chain of an α amino acid such as $CH(CH_3)_2$, $CH_2OH$, $CH_3$, and preferably H, or
    * B', B' being chosen from among: an alkyl chain of 1 to 10 carbon atoms, or a side chain of an α amino acid, said chains containing a functional group such as carboxylic, SH, OH or amine, free or protected,
* $X_1$ represents

- the group $[NH-(A_i)-CO]_m$-R,
- or $[NH-(A_i)-CO]_m$-Q

* R represents OH, $OCH_3$, $OCH_2$-$C_6H_5$, O-$C_6H_5$, O-$C_6F_5$, O-p$C_6H_4$-$NO_2$,

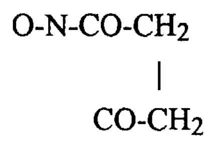

* m being an integer from 0 to 10, preferably from 0 to 5 and advantageously 1 or 2,
* $A_i$ represents an organic radical such as an alkyl chain from 1 to 10 carbon atoms, in particular $(CH_2)_n$-W-$(CH_2)_{n'}$, n + n' representing an integer from 0 to 10, W representing CHY, Y being H, an alkyl from 1 to 6 linear or branched carbon atoms, an α amino acid residue, natural or synthetic, or W representing an aromatic compound, in particular phenyl.

**9.** Products according to any one of claims 7 or 8, of the general formula (IIa)

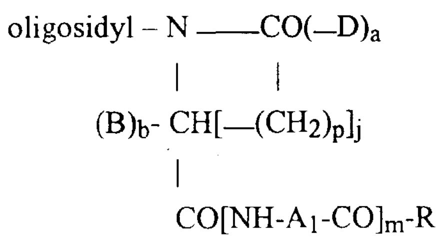

(IIa)

in which:

* a = 0 or 1,
* j = 0 or 1,
* b = 0 or 1,
* p = 2 to 4, in particular 2,
* provided that

  ** a = b = 0, when j = 1, which leads to the presence of a cyclic molecule,
  ** or a = b = 1, when j = 0 which implies the absence of the $(CH_2)_p$ group,

* D represents a residue of an organic acid of the formula $DCO_2H$, in particular H or an alkyl chain of 1 to 10 carbon atoms, in particular $CH_3$,
* B represents H, an alkyl chain of 1 to 10 carbon atoms, or a side chain of an a amino acid such as $CH(CH_3)_2$, $CH_2OH$, $CH_3$, and preferably H,
* m being an integer from 0 to 10, preferably from 0 to 5 and advantageously 1 or 2,
* $A_i$ represents an organic radical such as an alkyl chain from 1 to 10 carbon atoms, in particular $(CH_2)_n$-W-$(CH_2)_{n'}$, n + n' representing an integer from 0 to 10, W representing CHY, Y being H, an alkyl from 1 to 6 linear or ramified carbon atoms, an α amino acid residue, natural or synthetic, or W representing an aromatic compound, in particular phenyl,
* R represents a compound possessing an alcohol, phenol, thiol or amine function.

**10.** Products according to any one of claims 7 or 8, of the general formula (IIIa)

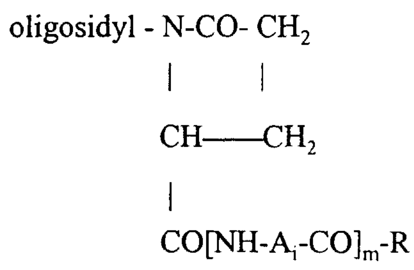

(IIIa)

in which $A_i$, m and R have the significations indicated in claim 8.

**11.** Products according to any one of claims 7 or 8, of the general formula (IVa)

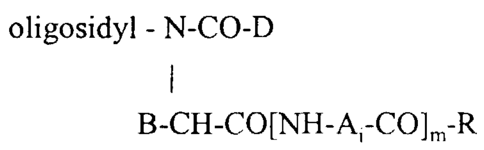

(IVa)

in which D, B, m, $A_i$ and R have the significations indicated in claim 8.

**12.** Products according to any one of claims 7 or 8, of the general formula (Va)

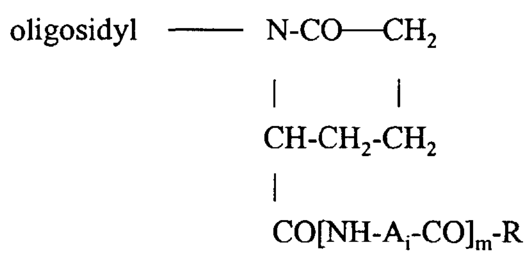

(Va)

in which $A_i$, m and R have the significations indicated in claim 8.

**13.** Products of formula (VI)

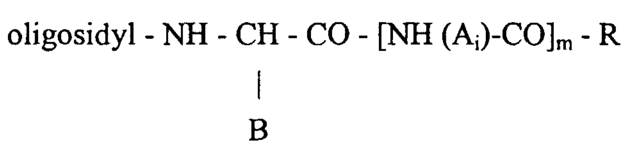

(VI)

in which B, $A_i$, m and R have the significations indicated in claim 2.

**14.** Products of formula (VII)

$$\text{oligosidyl - NH - }\underset{\underset{\underset{|}{CO_2H}}{\underset{|}{(CH_2)p}}}{\text{CH}}\text{ - CO - [NH-(A}_i\text{)-CO]}_m\text{ - R} \qquad (VII)$$

in which p, $A_i$, R, m have the significations indicated in claim 2.

**15.** Compounds or products according to any one of claims 1 to 14, characterized in that Q represents the following radicals :

OH, $O\text{-}CH_2\text{-}C_6H_5$, $O\text{-}C_6H_5$, $O\text{-}C_6F_5$, $O\text{-}pC_6H_4\text{-}NO_2$,

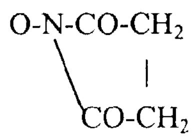

and characterized in that R represents one of the following radicals :

$-NH\text{-}pC_6H_4\text{-}N{=}C{=}S$ and its precursors :

$$-NH\text{-}pC_6H_4\text{-}NO_2$$

$$-NH\text{-}pC_6H_4\text{-}NH_2$$

$$-NH\text{-}CH_2\text{-}(CH_2)_m\text{-}C(=NH_2^+)OCH_3$$

$$-NH\text{-}CH_2\text{-}(CH_2)_m\text{-}CN$$

$$-NH\text{-}CH_2\text{-}(CH_2)_m\text{-}CH_2\text{-}NH\text{-}CO\text{-}CH_2\text{-}(CH_2)_m\text{-}C(=NH_2^+)OCH_3$$

$$-NH\text{-}CH_2\text{-}(CH_2)_m\text{-}CH_2\text{-}NH\text{-}CO\text{-}CH_2\text{-}(CH_2)_m\text{-}CN$$

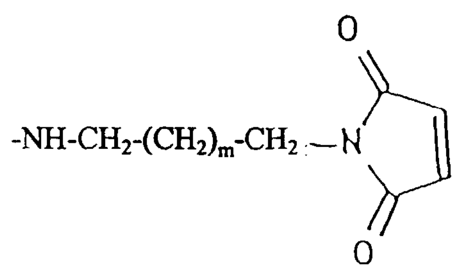

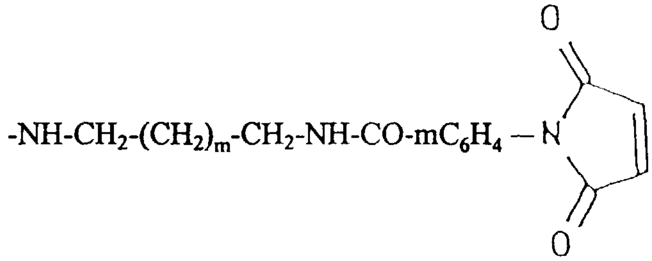

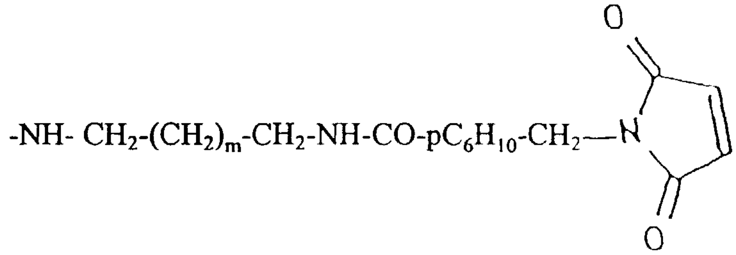

$-NH-CH_2-(CH_2)_m-CH_2-NH-CO-CH_2-T$ T = Br, I, Cl

$-NH-CH_2-CH_2-NH-CO-CH_2-(CH_2)_m-S-S-Pyr$

$-NH-CH_2-(CH_2)_m-CH_2-S-S-Pyr$

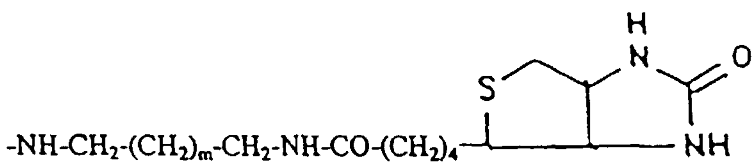

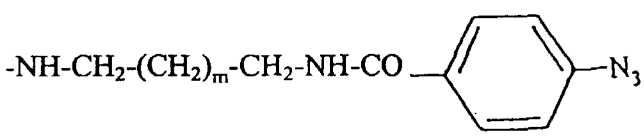

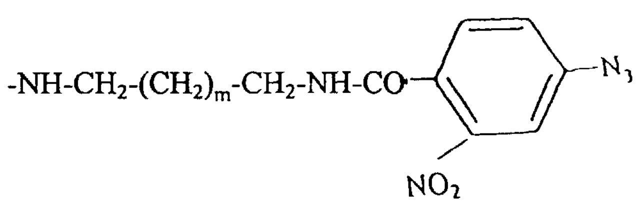

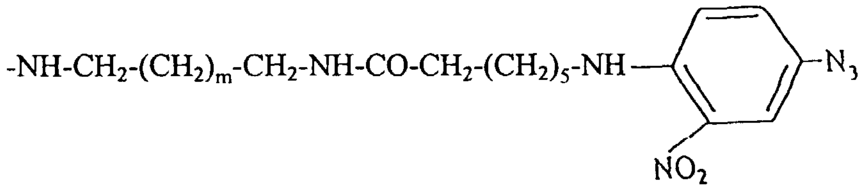

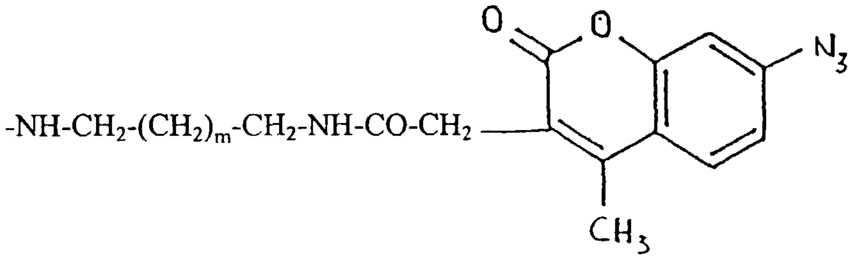

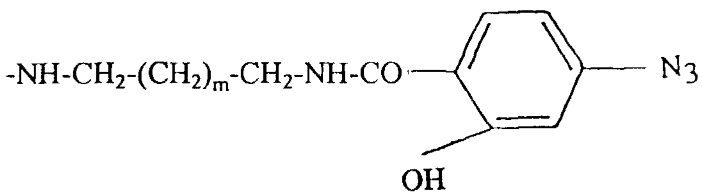

$-NH-(CH_2)_m-pC_6H_4OH$

$-NH-CH_2-(CH_2)_m-CH_2-NH-CO-(CH_2)_m-pC_6H_4OH$

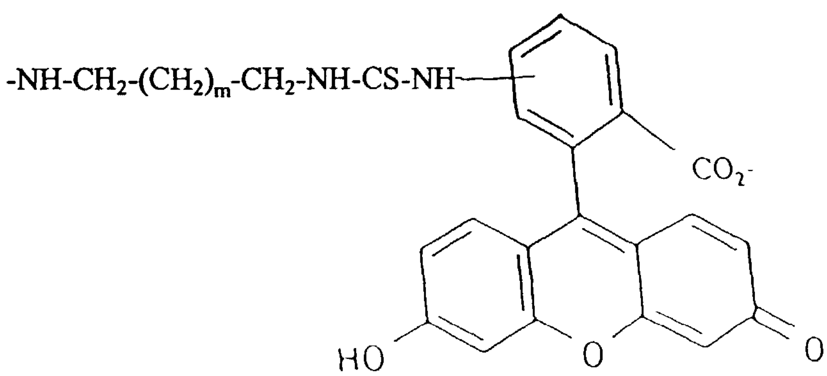

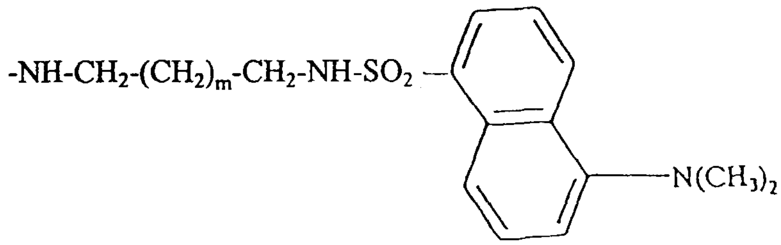

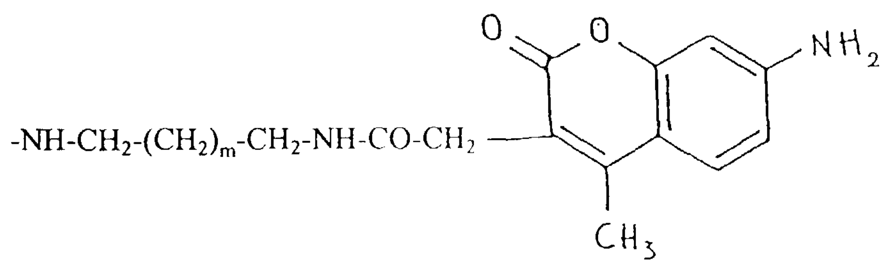

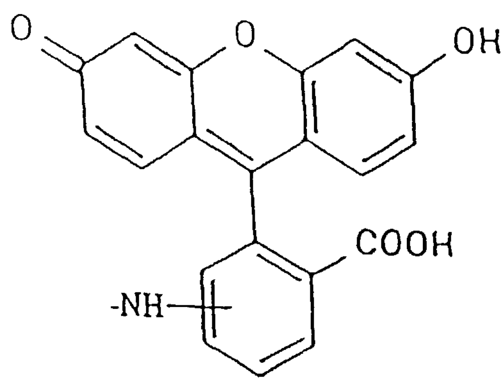

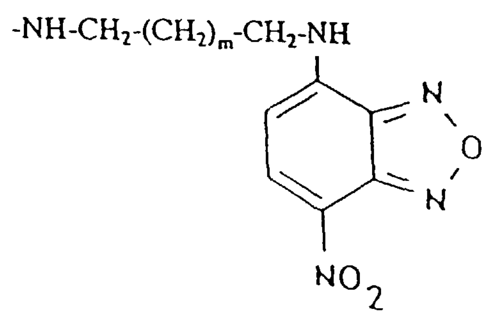

m representing an integer from 0 to 10, preferably from 0 to 5, and advantageously 1 or 2,
Pyr representing the 2-pyridine group.

**16.** Compound according to any one of claims 1 or 2, characterized in that P represents an oligopeptide, or a polypeptide, in particular the gluconoylated polylysine, and P' or P" represents an oligonucleotide, or R represents fluoresceine or one of its derivatives or another fluorescent derivative, and P' or P" represents an oligonucleotide or P represents a therapeutic agent or any molecule of interest.

**17.** Compounds according to any one of claims 1 to 12, characterized in that the oligoside residue is an oligosaccharide comprising from 2 to 50 oses and in particular is chosen from :

| | |
|---|---|
| lacto-N-tetraose | Galβ 3 GlcNAcβ 3 Galβ 4 Glc |
| neolacto-N-tetraose | Galβ 4 GlcNAcβ 3 Galβ 4 Glc |
| groupe H | Fuc α 2 Galβ 3 Galβ 4 Glc |
| Lewis[a] | Galβ 3 GlcNAcβ 3 Gal β 4 Glc<br>Fuc α 4-↑ |
| Lewis[x] | Galβ 4 GlcNAcβ 3 Galβ 4 Glc<br>Fuc α 3-↑ |
| Lewis[b] | Fuc 2 Galβ 3 GlcNAc β 3 Galβ 4 Glc<br>Fuc α 4-↑ |
| Lewis[y] | Fuc 2 Galβ 4 GlcNAcβ 3 Galβ 4 Glc<br>Fuc α 3-↑ |
| Disialolacto-N-tetraose | Neu 5Acα 3 Gal β 3 GlcNAc β 3 Galβ 4 Glc<br>Neu 5Acα 6-↑ |

Three antenna complex type

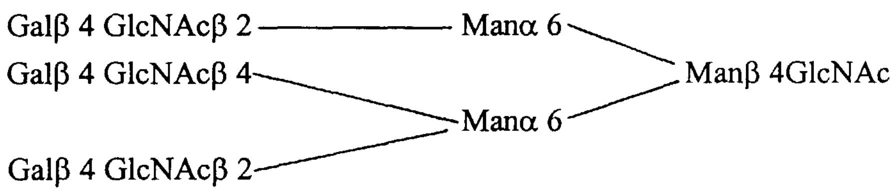

| | |
|---|---|
| Sialylactose 3 | Neu 5Ac α 3 Gal 4 Glc |
| Sialylactose 6 | Neu 5Ac α 6 Galβ 4 Glc |
| Disialylactose 3 | Neu 5Ac α 8 Neu 5Ac α 3 Galβ 4 Glc |

- simple or complex osides recognized by the lectin membranes, and chosen, for example, from :

**a. Asialo-oligoside of type lactosamine triantenna:** receptor of asialoglycoprotein

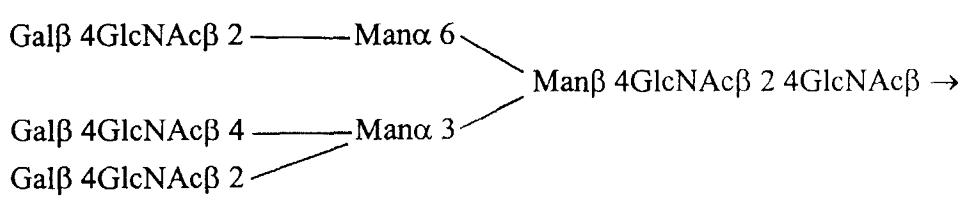

**b. Asialo-oligoside of type lactosamine tetraantenna: receptor of asialoglycoprotein**

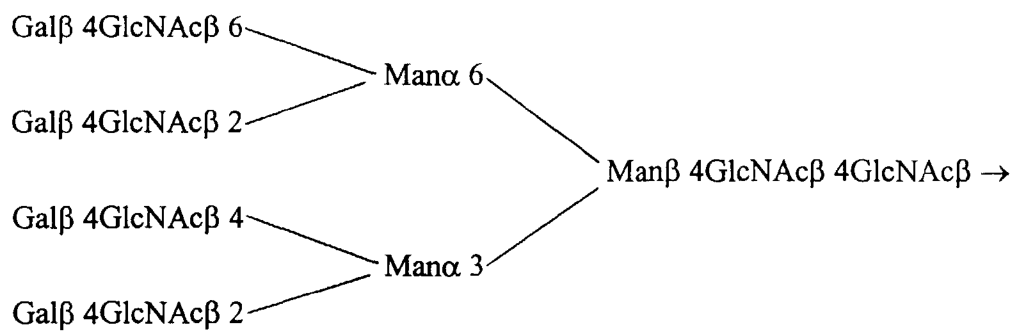

**c. Lewis x: LECAM 2/3**

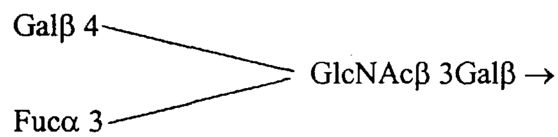

**d. Sialyl Lewis x : LECAM 3/2**

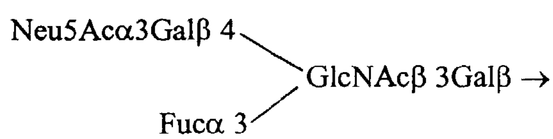

**e. Derivative of Lewis x sulfate (HNK1): LECAM 1**

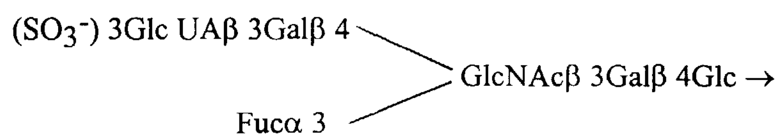

**f. Oligomannoside: receptor of mannose**

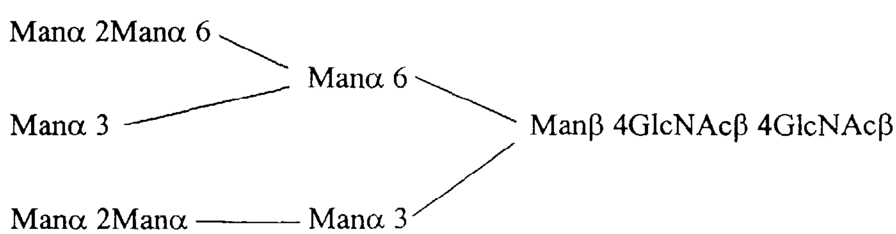

**g. Phosphorylated oligomannoside : receptor of mannose 6 phosphate**

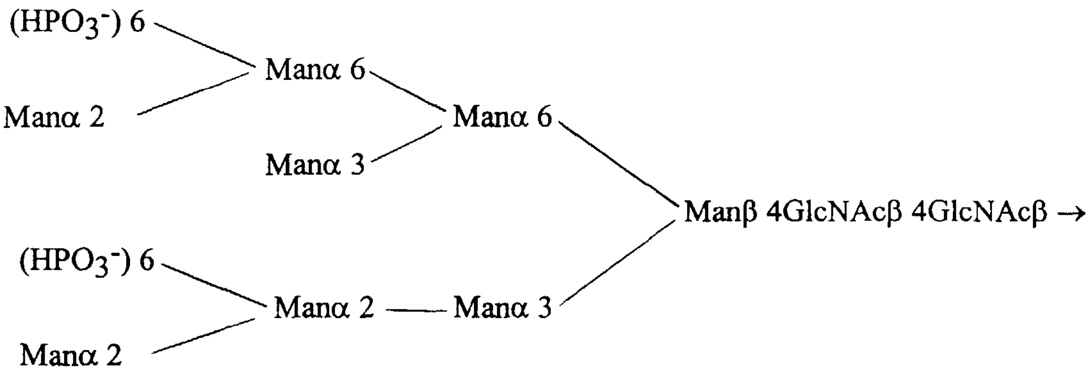

**h. Oligosaccharide of sulfated lactosamine type: receptor of sulfated GalNAc 4**

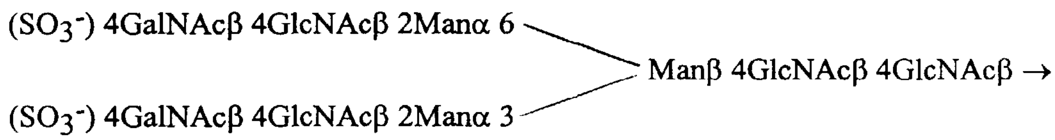

**18.** Process of preparation of compounds according to any one of claims 7 to 12, characterized in that:

- one condenses, in an appropriate solvent, an oligoside having a free reducing sugar, on the nitrogen atom of an intermediary molecule, this nitrogen atom belonging to an amine group, linked to a carbon atom placed in α of a C = 0 group, the intermediary molecule possibly possessing a side chain containing a functional group such as OH, SH, $NH_2$ or COOH, free or protected, this starting molecule being chosen from the following intermediary molecules: α amino acid, natural or synthetic, derivative of α amino acid, amino acid in N-terminal position of a peptide, or a peptidic derivative, possibly in presence of a catalyst such as imidazole, in a solvent appropriate for obtaining a derivative of glycosylamine in which the terminal ose of the oligoside conserves its cyclic structure, and in which the semiacetalic hydroxyl is replaced by the α amine of one of the said starting molecules,
- while the intermediary molecule does not possess a side chain containing a functional group such as described above, or a side chain in which the functional group is possibly protected, one acylates the derivative of glycosylamine obtained at the end of the preceding step by the addition of an organic acid activated by a classical activator such as carbonyl diimidazole, BOP (Benzotriazol-1-yloxytris (diméthylamino)-phosphonium hexafluorophosphate) or HBTU (O-benzotriazol-1-yl-N, N, N', N'-tetramethyluronium hexafluorophosphate), to obtain a derivative of N-acylated glycosylamine, followed possibly by a deprotection of the functional group of said side chain, in view of a possible substitution,
- while the intermediary molecule possesses a side chain containing a carboxylic group, one activates the said carboxylic group for it to intramolecularly react with the said α amine, leading to a cyclization inside the said intermediary molecule, to obtain a derivative of N-acylated glycosylamine.
- while the intermediary molecule possesses a side chain containing a carboxylic group, one can add the acylation agent in an active ester form.

**19.** Process of preparation of a compound according to any one of claims 1 to 6, characterized in that one makes to react

- one or several products (acylated derivatives of glycosylamine) according to one of claims 8 to 12, carrying a Q group, activated or able to be activated, reacting on a molecule, matrix or particle P, P' and P",
- one or several products (acylated derivatives of glycosylamine) according to one of claims 8 to 12, carrying either an R group, activated or able to be activated, or a B' group containing an activated or able to be activated functional group, or an $A_i$ group containing a functional group able to react with a molecule, matrix or particle, P, P' and P" respectively, containing a functional group, to obtain a product of type :
  (glycopeptidyl-R)$_n$-P, (glycopeptidyl -B')$_n$-P', or (glycopeptidyl-$A_i$)$_n$-P", n≥1,
- one or several products (acylated derivatives of glycosylamine) of the invention, carrying in one part an R

group, activated or able to be activated, in another part a B' group containing an activated or able to be activated group, or an $A_i$ group containing a functional group with molecules, matrixs or particles P and P', or of molecules, matrices or particles P and P",

- one or several products (acylated derivatives of glycosylamine) of the invention, carrying in one part an R group, activated or able to be activated, in another part a B' group containing an activated or able to be activated group, and an $A_i$ group containing a functional group respectively with molecules, matrices or particles P, P' and P",

R, B', $A_i$, P, P' and P" being defined according to any one of claims 1 to 6.

**20.** Process according to claim 19 in which the compound to be substituted consisting in the molecule, matrix or particle P, P' or P", is a protein, a lipid, a nucleic acid, an oligonucleotide, a polylysine, an insoluble polymer, a latex bead or a gold bead.

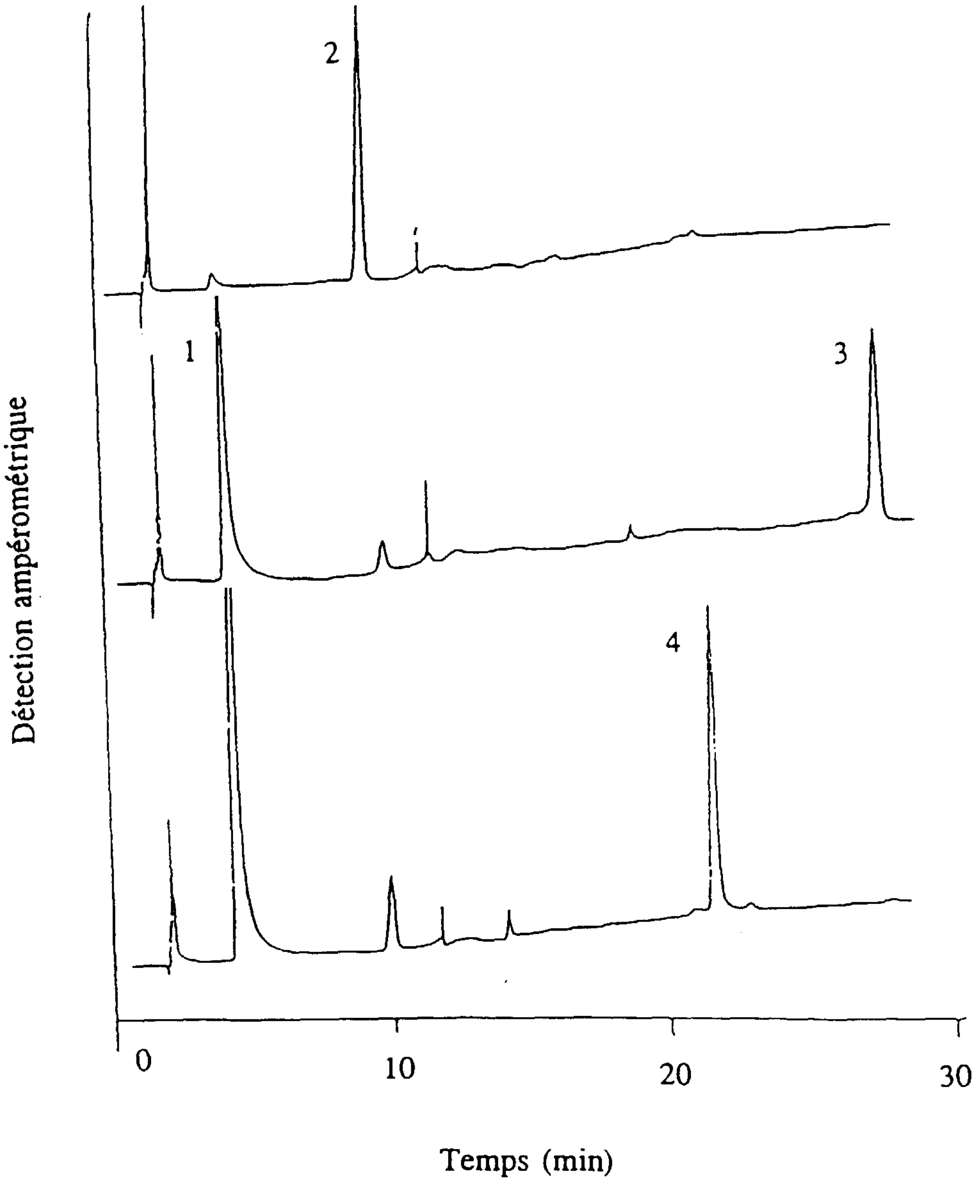


**Figure 1**

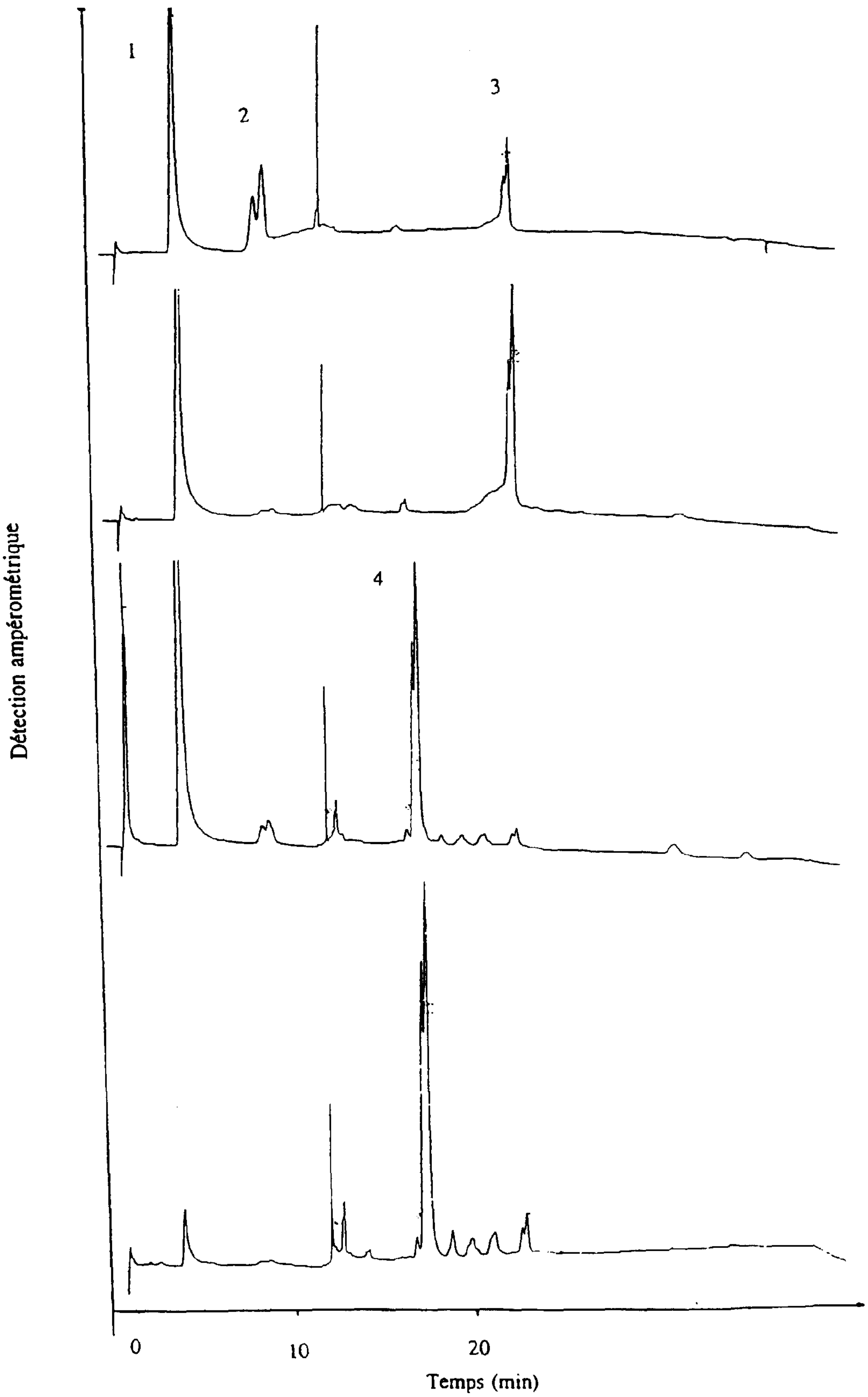


**Figure 2**